# EUROPEAN PATENT SPECIFICATION

(11) **EP 1 701 741 B1**
(45) Date of publication and mention of the grant of the patent: **28.05.2008**
(21) Application number: 05701809.5
(22) Date of filing: 07.01.2005
(51) Int. Cl.: A61K 45/00

(54) **COMPLEXES HAVING ADJUVANT ACTIVITY**
KOMPLEXE MIT ADJUVANTER WIRKUNG
COMPLEXES PRESENTANT UNE ACTIVITE D'ADJUVANT

(30) Priority: 07.01.2004 GB 0400264
(43) Date of publication of application: 20.09.2006
(73) Proprietor: Polytherics Limited, London EC4A 1JP (GB)
(72) Inventor: SHAUNAK, Sunil Department of Infectious Diseases, DuCane Road London W12 0NN (GB); BROCCHINI, Stephen Department of Pharmaceutics, University of London London WC1N 1AX (GB); GODWIN, Antony Department of Pharmaceutics, University of London London WC1N 1AX (GB); CHOI, Ji-Won Department of Infectious Diseases, DuCane Road London W12 0NN (GB)
(74) Representative: Scott, Susan Margaret
(86) International application number: PCT/GB2005/000039
(87) International publication number: WO 2005/065712

(56) References cited:
- EP-A- 1 031 353
- WO-A-01/18080
- WO-A-02/49666
- WO-A-03/039435
- GB-A- 2 290 707
- US-A- 5 160 737
- US-A- 5 753 256
- US-A- 5 889 078
- KREUTER ET AL.: "MODE OF ACTION OF IMMUNOLOGICAL ADJUVANTS SOME PHYSICOCHEMICAL FACTORS INFLUENCING THE EFFECTIVITY OF POLY ACRYLIC ADJUVANTS" INFECTION AND IMMUNITY, vol. 19, no. 2, 1978, pages 667-675, XP009045654 ISSN: 0019-9567
- GODWIN, HARTENSTEIN, MÜLLER, BROCCHINI: "Narrow molecular weight distribution precursors for polymer-drug conjugates" ANGEWANDTE CHEMIE - INTERNATIONAL EDITION, vol. 40, no. 3, 2001, pages 594-597, XP002347826

## Description

### Introduction

The present invention relates to complexes having adjuvant properties.

### Background to the invention

Protective immunity against exposure to pathogens is achieved by the integration of two distinct arms of the immune response. They are the innate response and the antigen-specific, also called adaptive, response. The innate immune response acts early after infection, within minutes, by detecting and responding to broad cues from invading pathogens. By contrast, the adaptive immune response takes time, up to two weeks, to become effective, but it provides the fine antigenic specificity that is required for complete elimination of the pathogen and for the generation of immunological memory. Antigen independent recognition of pathogens by the innate immune system leads to the immediate mobilisation of immune effector and regulatory mechanisms which provide the host with three important survival advantages:-
(i) Rapid initiation of immune responses, both innate and adaptive, and the creation of the inflammatory and co-stimulatory environment required for antigen recognition.
(ii) Establishment of a first line of defence to hold the pathogen in check during the maturation of the adaptive response.
(iii) Steering of the adaptive immune response towards the cellular or humoral elements of the immune response that are most effective for protection against a particular infectious agent.

So, although the overall objective of vaccination is the activation of antigen specific immunity, vaccines cannot achieve this goal optimally without first and effectively activating the pathogen detection mechanisms of the innate immune response.

Many vaccines comprise antigens and adjuvants. They are broadly defined by their functional ability to enhance the *in vivo* immunogenicity of the antigens with which they are co-administered. As such, adjuvants represent important components of most of the successful vaccines, particularly vaccines based on sub-units of pathogens including isolated fractions of the killed pathogens and/or recombinant antigens. However, as a consequence of the growing appreciation of innate immune mechanisms, and the details of antigen processing and presentation, new and more sophisticated adjuvants are required. Therefore, traditional and novel adjuvants are now being increasingly separated into those that act as a delivery system and those that act as immune potentiators. Whereas the main function of a delivery system is to localise the vaccine components to antigen presenting cells, immune potentiators directly activate antigen presenting cells through specific receptors that include Toll-like receptors.

It is important to keep in mind that infectious agents and most licensed vaccines, particularly live attenuated and killed whole cell products, contain all of the components necessary for activating an integrated immune response. That is because the pathogens used in such vaccines possess all of the relevant antigens in a particulate form, as a whole cell, which also contains many potent immune modulators; i.e., pathogen associated molecular patterns. However, the trend in vaccine development is to move away from such products towards safer and better defined sub-unit vaccines that are produced as highly purified recombinant proteins. Unfortunately, such recombinant antigens are often poorly immunogenic because they lack intrinsic immune potentiating activity. The challenge in sub-unit vaccine development is therefore to reintroduce selective signals for activation of the innate immune response that would be sufficient to mimic natural infection or whole pathogen cell vaccination approaches. The delivery systems and the immune potentiators used to improve the potency of subunit vaccines should be low cost, more effective and safer than whole cell vaccines.

Although the terms "delivery system" and "adjuvant" have generally been used interchangeably in relation to vaccines, a clear distinction can often be made and the respective role of each clearly defined. Included in lists of vaccine "adjuvants" are a number of particulate delivery systems, e.g., emulsions, liposomes, iscoms, virus like particles and microparticles, whose principle mode of action is to promote the delivery of antigens into the key antigen presenting cells responsible for the induction of immune responses. However, they are poor immune modulators and their potency therefore needs to be improved significantly by the addition of an immune potentiator. Unless specified otherwise, the term "adjuvant" is used herein to denote an immune potentiator. The main hurdle to the development of new and improved adjuvants as immune potentiators has been safety because new vaccines must have a minimal number of adverse effects if they are to be acceptable for clinical use. As a result, although many adjuvants have been extensively evaluated pre-clinically and clinically, only aluminium sources, generically called "alum", has been successfully licensed for use as a vaccine adjuvant in North America. Alum is poor at generating cytotoxic CD8 T cell immunity. It preferentially induces a Th2 biased immune response rather than a Th1 biased immune response.

Although proof of concept has been established in animals for the use of many natural pathogen-associated molecular pattern molecules as immune potentiator adjuvants, the trend for the future is to design synthetic analogues. This is mainly due to the lower manufacturing costs and the smaller regulatory hurdles associated with well defined and standardised synthetic immune potentiators.

It was shown as early as 1968 that several synthetic polyanionic polymers protect mice challenged with a lethal dose of Meningo virus and that murine peritoneal macrophages produced factors that inhibited vesicular stomatis virus when exposed to carboxylate co-polymer or a salt thereof *in vitro* (Merigan & Finkelstein. Interferon stimulating and in vivo antiviral effects of various synthetic anionic polymers. Virology 1968; 35: 363-374).

More recently, alginates (polymers isolated from marine algae) have been shown to elicit the release of TNF-α, IL-1 and IL-6 (Otterlei M et al. Induction of cytokine production from human monocytes stimulated with alginate. J. lmmunother. 1991; 10: 286-291). The 1-4-β-mannuronic acid moieties of alginates also induced the release of TNF-α through a CD14/Toll-like receptor 4 mediated mechanism from monocytes. Lignin derivatives and fucoidan also illicit TNF-α release from macrophages (Sorimachi K et al. Secretion of TNF-α from macrophages following induction with a lignin derivative. Cell Biol. Int. 1995; 19: 833-838; Heinzelmann et al. Modulation of LPS-induced monocyte activation by heparin-binding protein and fucoidan. Infect. Immun. 1998; 66: 5842-5847). Sulphated fucans and dextran sulphate have also been shown to dramatically increase circulating levels of IL-6, IL-8, MCP-1, M-CSF and G-CSF when injected into pig-tailed monkeys (Sweeney EA et al. Mobilization of stem/progenitor cells by sulphated polysaccharides does not require selectin presence. Proc. Natl. Acad. Sci. 2000; 6: 6544-6549).

Chemokines are a heterogenous group of inducible proinflammatory chemoattractive cytokines that include TNF-α, IL-1 and IL-6. β-Chemokines, which include MIP-1α and MIP-1β, act as chemoattractants for monocytes, eosinophils, basophils and lymphocytes (Luster AD. Chemokines - chemotactic cytokines that mediate inflammation. New Eng. J. Med. 1998; 338:436-446). Binding of chemokines to their respective receptors leads to a cascade of cellular activation including the generation of inositol triphosphate, the release of intracellular calcium, and the activation of protein kinase C. Hence, chemokines activate the cellular mechanisms that control chemotaxis. As such, they play a pivotal role in the recruitment of leucocytes to the areas in which they accumulate and in the generation of local immune responses.

However, excessive release of pro-inflammatory cytokines by the polymers mentioned above has meant that they are far too toxic to be safely administered to man. Therefore, although many polymers have been shown to have immuno-modulatory properties, they have never been effectively exploited for therapeutic use in man because of the problems associated with their isolation from natural sources, with the reproducibility of isolation, with the synthesis of synthetic polymers in the laboratory, and with the toxicity of polymers when they are administered to animals.

### Summary of the invention

The present invention provides a complex as defined in claim 1, that comprises a narrow molecular weight distribution polymer that includes units derived from an acrylic acid or a salt thereof, and
(i) a substance that has pharmacological activity against a pathogenic organism, or
(ii) a substance that has pharmacological activity against a cancer, or
(iii) one or more agents selected from antigens and immunogens.

The present invention also provides a pharmaceutical preparation which comprises a complex of the present invention in admixture or conjunction with a pharmaceutically suitable carrier.

The present invention further provides a complex of the invention for use as a medicament.

The invention also provides a complex for use in treatment of an infection by a pathogenic organism and/or for inducing an Immune response to the pathogenic organism, which complex comprises a narrow molecular weight distribution polymer that includes units derived from an acrylic acid or a salt thereof as defined in claim 1, and a substance that has pharmacological activity against the pathogenic organism.

The invention further provides a complex for use in treatment of a cancer, which complex comprises a narrow molecular weight distribution polymer that includes units derived from an acrylic acid or a salt thereof, as defined in claim 1, and a substance that has pharmacological activity against the cancer

The invention further provides a complex that comprises a narrow molecular weight distribution polymer that includes units derived from an acrylic acid or a salt thereof, as defined in claim 1, and one or more agents selected from antigens and immunogens, for inducing an immune response to the antigen or immunogen.

The invention further provides a narrow molecular weight distribution polymer that includes units derived from an acrylic acid or a salt thereof, for use with a substance that has pharmacological activity against a pathogenic organism for the treatment of an infection by the pathogenic organism and/or for inducing an immune response to the pathogenic organism.

The invention further provides a narrow molecular weight distribution polymer that includes units derived from an acrylic acid or a salt thereof, for use with one or more agents selected from antigens and Immunogens for Inducing an immune response to the antigen or immunogen.

The invention further provides a narrow molecular weight distribution polymer that Includes units derived from an acrylic acid or a salt thereof, as defined in claim 1, for use with a substance that has pharmacological activity against a cancer for the treatment of the cancer and/or for inducing an immune response to the cancer.

The invention further provides a narrow molecular weight distribution polymer that includes units derived from an acrylic acid or a salt thereof, as defined in claim 1, for use as an immune potentiating adjuvant in the manufacture of a vaccine

The invention further provides a narrow molecular weight distribution polymer that includes units derived from an acrylic acid or a salt thereof, as defined in claim 1, for use as an immune potentiating adjuvant in the manufacture of a vaccine that comprises an antigen or immunogen against which an immune response is to be induced.

### Definitions

The term "a narrow molecular weight distribution polymer that includes units derived from an acrylic acid or a salt thereof" as used herein denotes a polymer that includes units derived from an acrylic acid or a salt thereof, as described herein, for example, a polymer including units derived from methacrylic acid or a salt thereof, for example, a polymethacrylic acid or a salt thereof, for example a sodium salt thereof. The polymer may be either a homopolymer or copolymer of an acrylic acid, for example, of acrylic acid or methacrylic acid or a salt thereof.

The polymer has a narrow molecular weight distribution, that is, a polydispersity of 1.7 or less, for example 1.6 or less, for example 1.5 or less, for example 1.4 or less, for example 1.2 or less, for example less than 1.7, for example less than 1.6, for example less than 1.5, for example less than 1.4, for example, less than 1.2. In general, the lower the polydispersibility, the better. Accordingly, a polydispersibility of less than 1.2 is generally preferred.

The polymer generally has a molecular weight such that, when present in the blood, the polymer remains substantially in the circulating blood after passage through the kidney, with little or none of the polymer undergoing filtration through the glomerular apparatus. Renal filtration (also known as glomerular filtration) of large molecules is a function *inter alia* of the size and shape of the molecule, which is dependent in part on the molecular weight. In general, for any particular polymer, there is a threshold molecular weight or a narrow range of molecular weights below which renal filtration occurs and above which little or no renal filtration occurs. The threshold molecular weight for any particular polymer may be determined by standard tests, for example, using a radiolabelled polymer.

The polymer has a molecular weight of 100,000 or less, for example, 80,000 or less, for example, 75,000 or less, for example,65,000 or less, for example, 55,000 or less, for example, 45,000 or less, for example. The polymer generally has a molecular weight of 4,000 or more, for example, 5,000 or more, for example, 10,000 or more, for example, 20,000 or more, for example, 30,000 or more, for example, 40,000 or more. A polymer may have a molecular weight within a range that combines any of the upper molecular weight values given above with any of the lower molecular weight values given above. Examples of such ranges include but are not limited to ranges of from 80,000 to 4,000, 75,000 to 5,000, 65,000 to 10,000, 55,000 to 10,000, and 45,000 to 10,000. Further examples include the ranges from 50,000 to 4,000, for example, from 40,000 to 25,000. Molecular weights in the range of from 45,000 to 10,000 may be preferred.

The term "PMAA-Na" is used herein to denote polymethacrylic acid, sodium salt. Unless stated otherwise, it denotes a polymethacrylic acid, sodium salt prepared as described in Examples A1 to A4.

The term "complex" is used herein to denote an association between a narrow molecular weight distribution polymer that includes units derived from an acrylic acid or a salt thereof and another substance as defined herein, the association between the components being primarily non-covalent, for example, involving any one or more of ionic, electrostatic and van der Waals forces. Although a complex according to the present invention predominantly involves non-covalent association between the components, there may nevertheless be some covalent bonding.

### Brief description of the Figures

Figure 1:

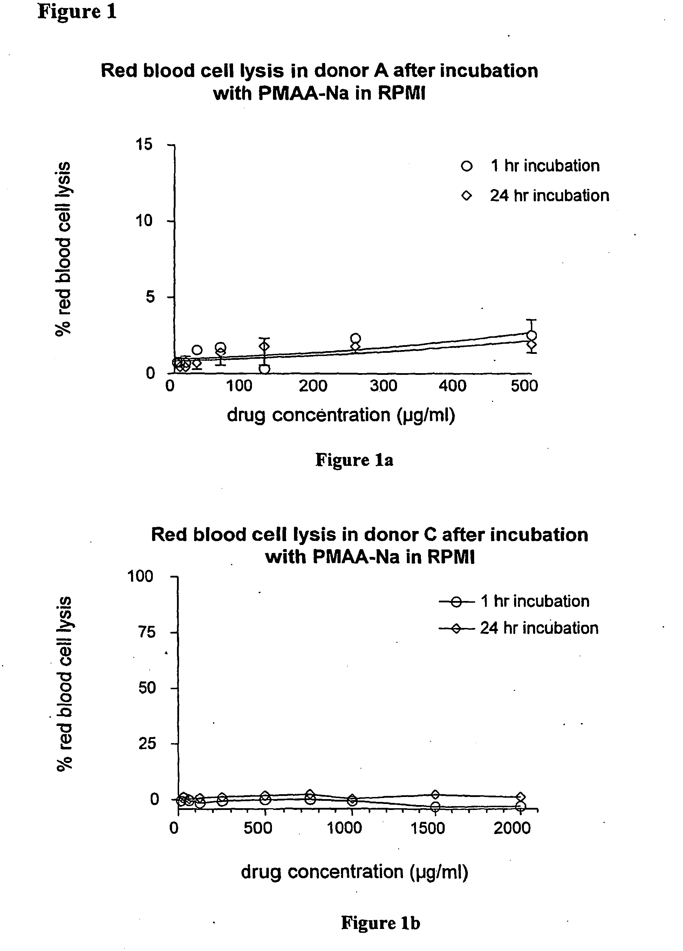

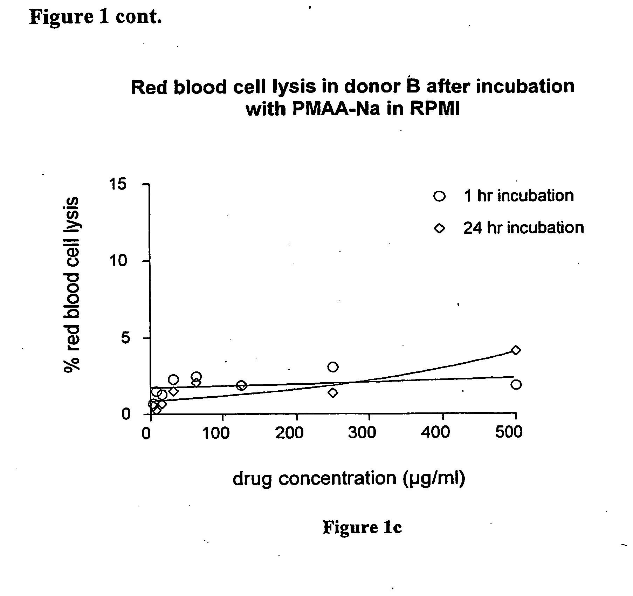

   Figure 1 shows red blood cell lysis after incubation of cells with PMAA-Na in RPMI.
   A 2% v/v solution of human red blood cells was incubated with a solution of PMAA-Na ("drug") in RPMI 1640 media for 1 h and 24 h at 37°C. No significant toxicity was seen with the PMAA-Na up to a concentration of 2,000 µglml after 1 h or after 24 hours. The figure shows the results for 3 human donors (A, B and C). Figure 1 a shows the results obtained for donor A, Figure 1b the results obtained for donor C and Figure 1c the results for donor B. The results obtained after incubation for 1 hour are shown as circles, the results after incubation for 24 hours are shown as diamonds.
Figure 2:

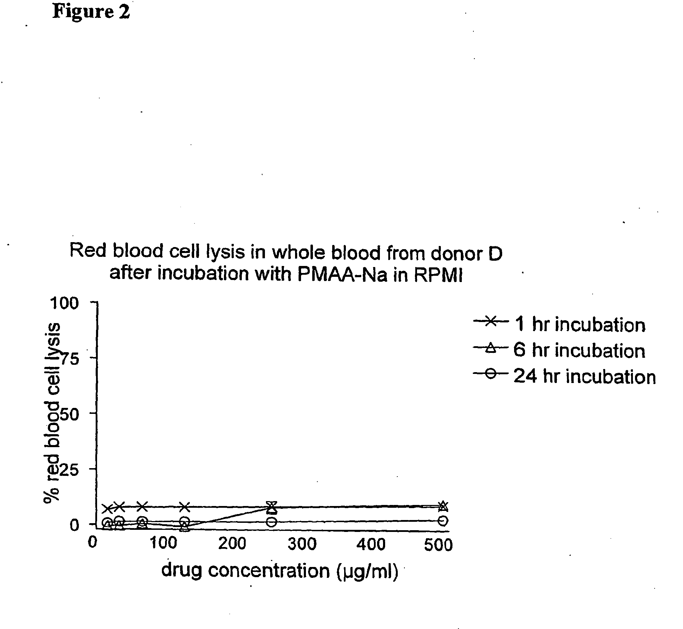

   Figure 2 shows red blood cell lysis in whole blood after incubation with PMAA-Na in RPMI.
   A 2% v/v solution of whole human blood (donor D) was incubated with a solution of PMAA-Na ("drug") in RPMI 1640. No significant toxicity was seen with the PMAA-Na using human whole blood up to a concentration of 500 µg/ml after 1 h (shown as crosses), after 6 h (shown as triangles) or after 24 hours (shown as circles).
Figure 3

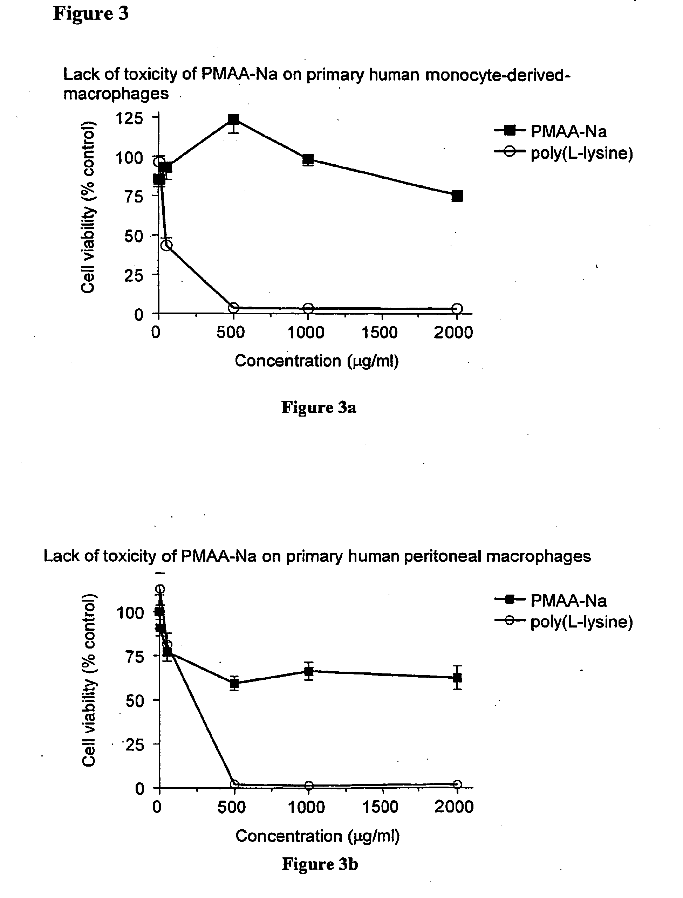

   Figure 3 shows the lack of toxicity of PMAA-Na on human monocyte derived macrophages (Figure 3a) and on primary human peritoneal macrophages (Figure 3b).
   Figure 3a shows the results obtained when monocyte derived macrophages were incubated with media containing PMAA-Na over the concentration range of 0 to 2,000 µg/ml. The cells were incubated for 71 h prior to the addition of thiazolyl blue (MTT, Sigma 5 mg/ml). The results obtained using PMAA-Na are shown as closed squares, the results with poly(L-lysine), used as control, are shown as circles. PMAA-Na was not toxic to MDMs at the highest concentration of 2,000 µg/ml tested using the MTT assay and the Trypan blue assay.
   Figure 3b shows the results obtained when human peritoneal cells were cultured with PMAA-Na over the concentration range of 0 to 2,000 µg/ml. The cells were incubated for 71 h prior to the addition of MTT. The results obtained using PMAA-Na are shown as closed squares, the results with poly(L-lysine), used as control, are shown as circles. PMAA-Na was not toxic to peritoneal macrophages up to 500 µg/ml. Between 500 µg/m and 2,000 µg/ml, a modest amount of toxicity was seen using the MTT assay and the Trypan blue assays.
Figure 4:

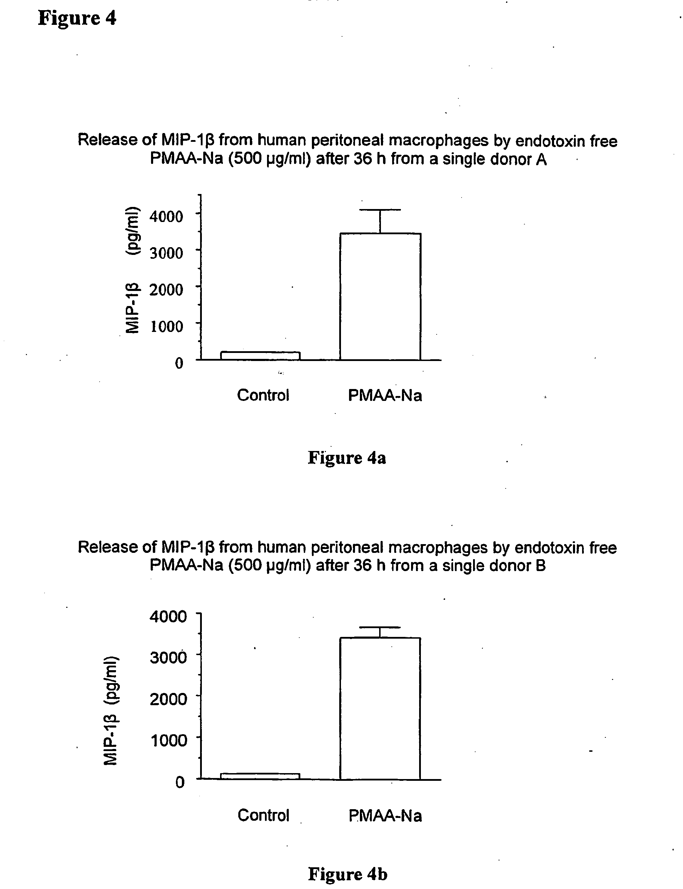

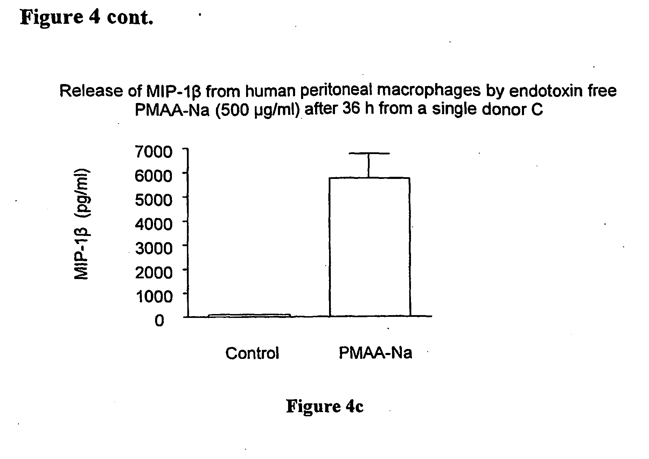

   Figure 4 shows release of MI P-1β from human peritoneal macrophages by endotoxin free PMAA-Na.
   Human peritoneal cells from 3 donors (A, B and C) were cultured with endotoxin-free PMAA-Na (500 µg/ml) and culture supernatants harvested after 36 h were analysed for MIP-1β. All reagents and the PMAA-Na contained <0.06 endotoxin units/ml (EU/ml). Figure 4a shows the results obtained for donor A, Figure 4b for donor B and Figure 4c for donor C. There was a significant release of MIP-1β in the presence of PMAA-Na.
Figure 5:

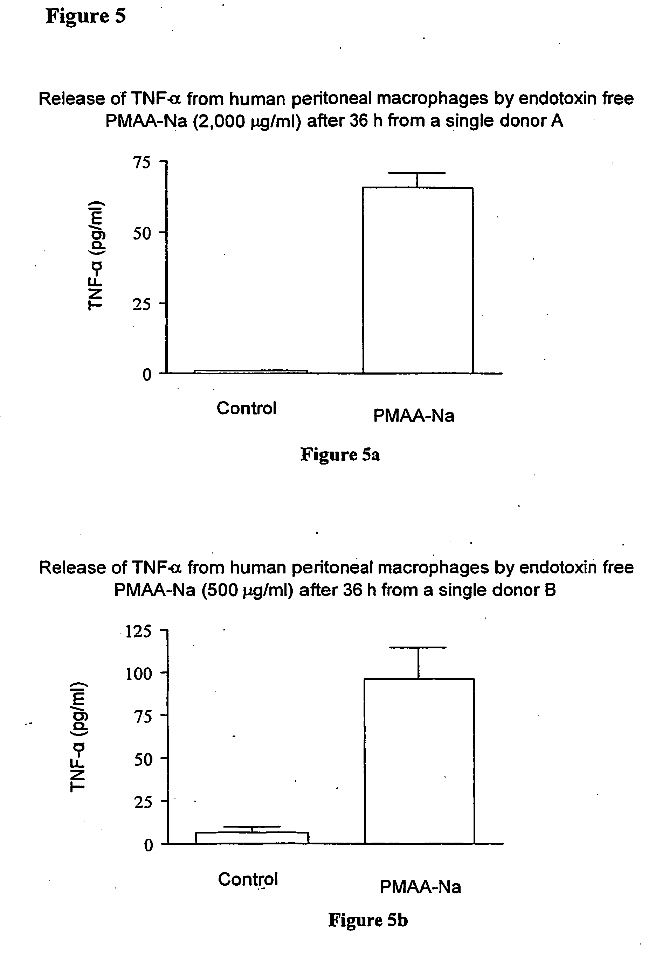

   Figure 5 shows release of TNF-α from human peritoneal macrophages by endotoxin free PMAA-Na.
   Human peritoneal cells from 2 donors (A and B, Figures 5a and 5b, respectively) were cultured with endotoxin-free PMAA-Na (500 µg/ml and 2,000 µg/ml) and culture supernatants harvested after 36 h were analysed for TNF-α. There was a significant release of TNF-α in the presence of PMAA-Na at both concentrations.
Figure 6:

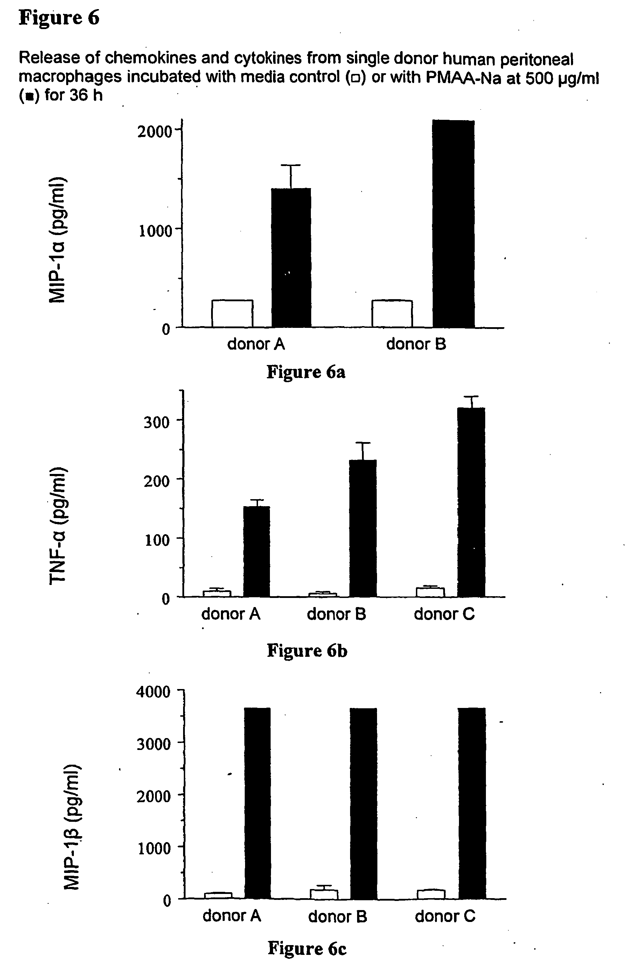

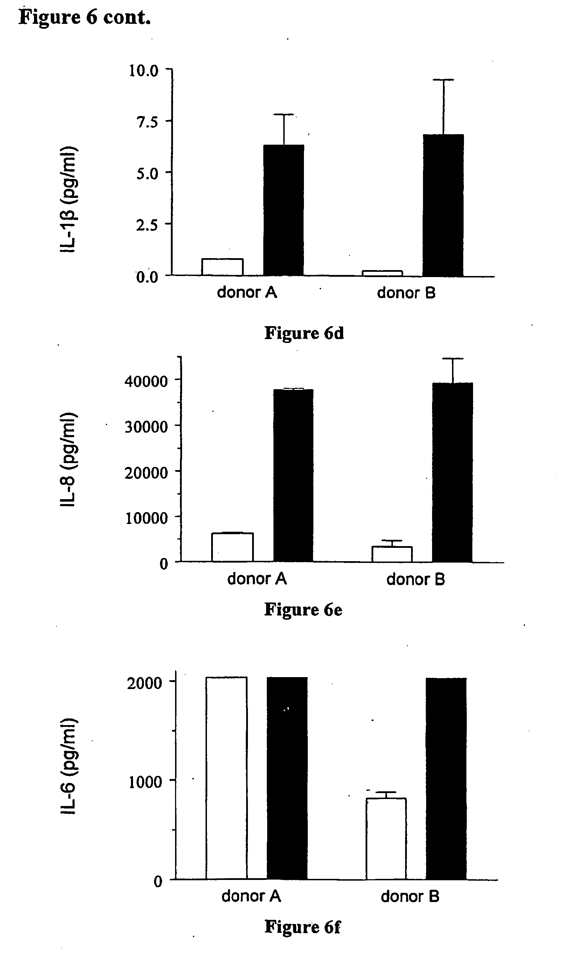

   Figure 6 shows the release of chemokines and cytokines from single donor human peritoneal macrophages incubated with media control or with PMAA-Na.
   Human peritoneal cells were cultured with PMAA-Na (500 µg/ml) and culture supernatants harvested after 36 h were analysed for the pro-inflammatory chemokines MIP-1α, MIP-1β and IL-8, and for the pro-inflammatory cytokines TNF-α, IL-1β and IL-6. The results with cells from up to 3 different human donors (A, B and C) are shown. The results obtained with the media control are shown as open blocks, those with PMAA-Na as black blocks. Release of MIP-1α is shown in Figure 6a, TNF-α in Figure 6b, , MIP-1β in Figure 6c, IL-8, and for the proinflammatory cytokines TNF-α, I L-1β in Figure 6d, IL-8 in Figure 6e, and IL-6 in Figure 6f. The release of these chemokines and cytokines from tissue antigen presenting cells was at a level that would promote a pharmacological Th1 response in man but not high enough to cause significant adverse side-effects in man.
Figure 7:

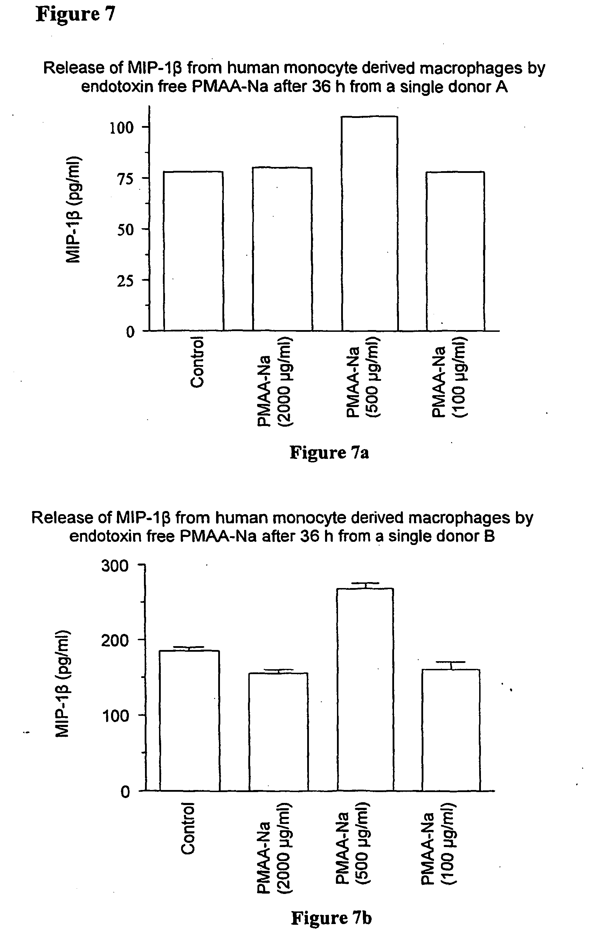

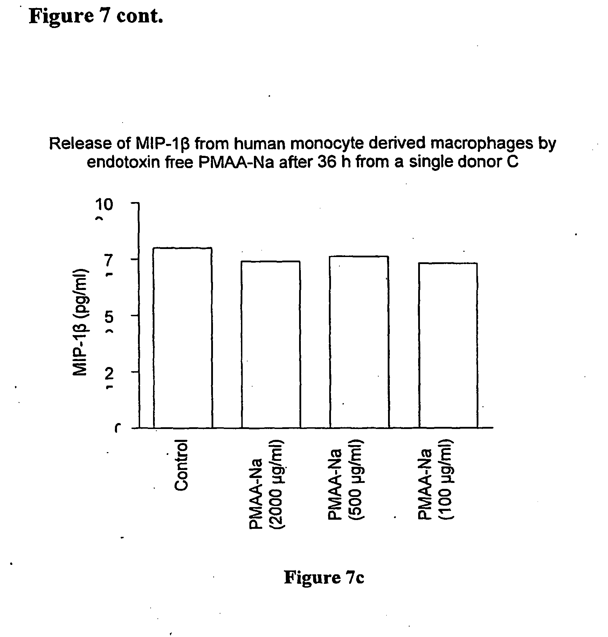

   Figure 7 shows the release of MIP-1β from human monocyte derived macrophages by endotoxin-free PMAA-Na
   Monocyte derived macrophages were cultured with PMAA-Na at concentrations of 100 µg/ml, 500 µg/ml and 2,000 µg/ml. The results with cells from 3 different human donors A, B and C are shown in Figures 7a, 7b and 7c, respectively. No release of MIP-1β was seen. There is therefore a differential immuno-modulatory effect of PMAA-Na on cells of blood monocyte origin compared to cells of macrophage origin from tissue body based compartments.
Figure 8**:**

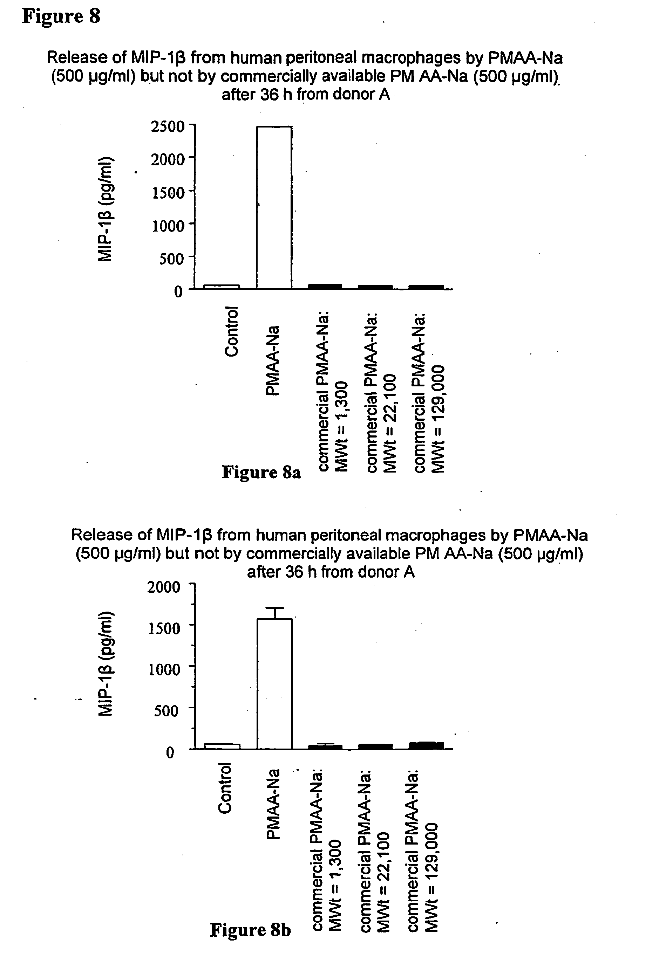

   Figure 8 shows the release of MIP-1β from human peritoneal macrophages by PMAA-Na but not by commercially available PMAA-Na
   Human peritoneal macrophages were cultured with commercially available PMAA-Na (Polymer Standards Service, Germany) of a range of MWfs (*M*ₙ = 1,300, 22,100 and 129,000 g/mol) and narrow molecular weight distribution and with PMAA-Na produced as described herein. One of the commercially available PMAA-Na possessed a MWt comparable to PMAA-Na prepared as described in Examples A1 to A4 (*M*ₙ = 22,000 g/mol) (PMAA-Na in the Figure). In each case 500 µg/ml of the PMAA-Na was used. The culture supernatants were harvested after 36 h. There was no release of MIP-1β. Figures 8a and 8b show the individual results from 2 human donors A and B, respectively.
Figure 9:

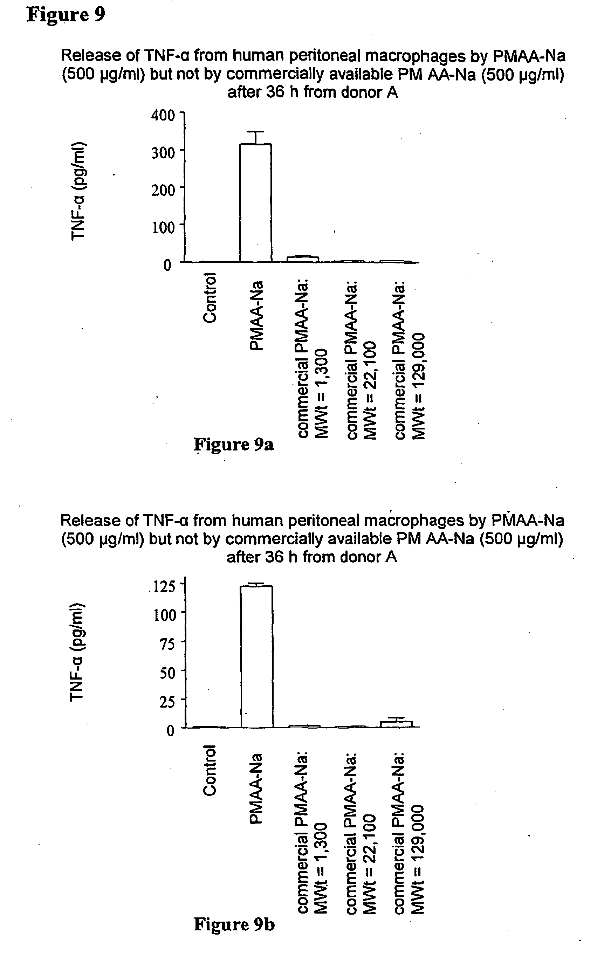

   Figure 9 shows the release of TNFα- from human peritoneal macrophages by PMAA-Na but not by commercially available PMAA-Na
   Human peritoneal macrophages were cultured with commercially available PMAA-Na (Polymer Standards Service, Germany) of a range of MWt's (*M*ₙ = 1,300, 22,100 and 129,000 g/mol) and narrow molecular weight distribution and with PMAA-Na produced as described herein. One of the commercially available PMAA-Na possessed a MWt comparable to our synthetic preparation of PMAA-Na (*Mₙ* = 22,000 g/mol) (PMAA-Na in the Figure). In each case 500 µg/ml of the PMAA-Na was used. The culture supernatants were harvested after 36 h. There was no release of TiVF-α. Figures 9a and 9b show the individual results from two human donors A and B, respectively.
Figures 10 and 11 and 12:
   Figures 10, 11 and 12 show red blood cell lysis after incubation with amphotericin B or with amphotericin B-PMAA-Na complex.
   The method was as described for Figure 1. The comparison was made between clinical grade amphotericin B (ampho B, results shown as open squares) and the amphotericin B - PMAA-Na complex prepared as described in Example C. (ampho B-PMAA-Na, results shown as open circles). Stock solutions of the compounds for testing were prepared in MGM. Human red cell lysis was determined after 1 hour (Figure 10), 6 hour (Figure 11) and 24 hour (Figure 12) incubation. In each case cells obtained from 3 different donors (A, B and C) were used. Each line represents the results from a single human donor.

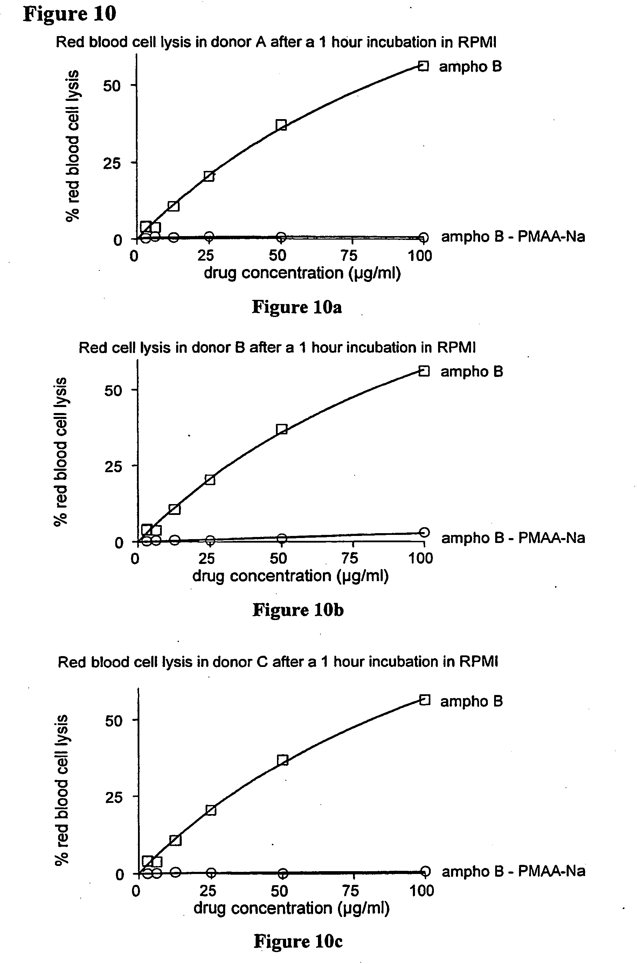

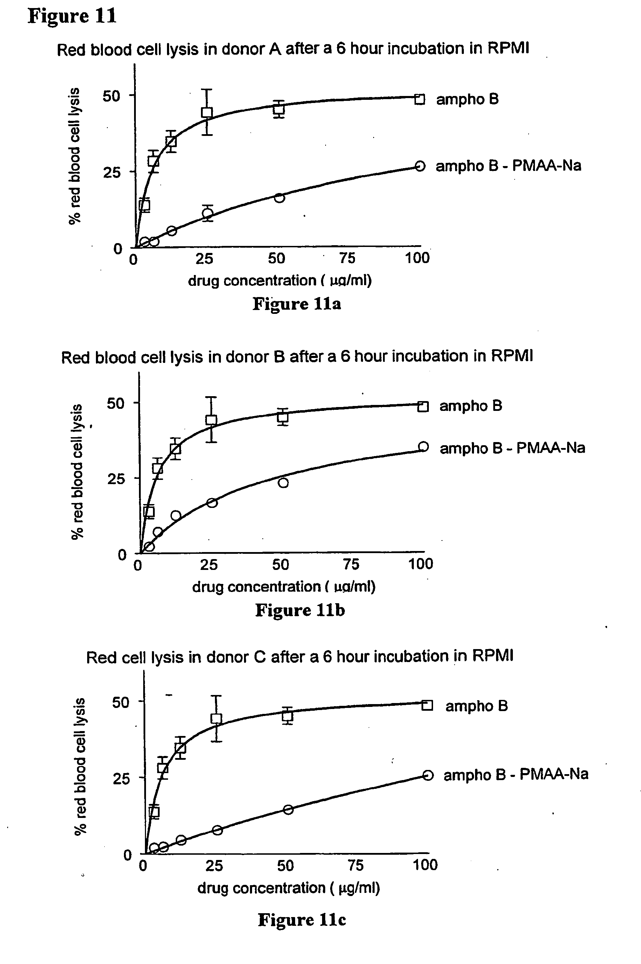

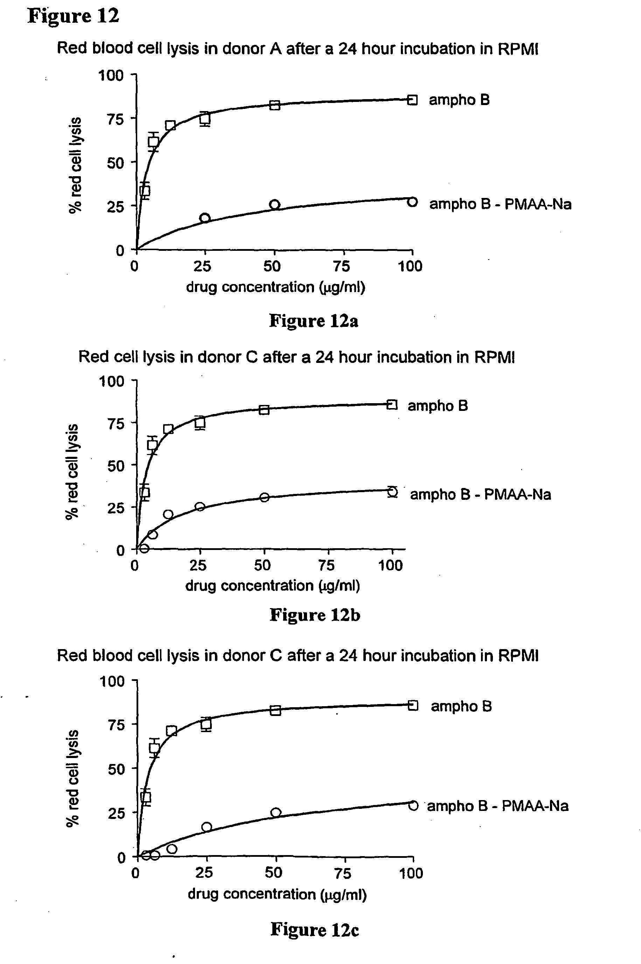

   Figures 10a, 10b and 10c show the results of a 1 hour incubation with cells of donors A, B and C, respectively. Figures 11a, 11b and 11c show the results of a 6 hour incubation with cells of donors A, B and C, respectively. Figures 12a, 12b and 12c show the results of a 24 hour incubation with cells of donors A, B and C, respectively,
   No toxicity was seen with the amphotericin B - PMAA-Na preparation after a 1 hour incubation. After a 6 hour incubation, the toxicity of the amphotericin B - PMAA-Na preparation was considerably less than that of clinical grade amphotericin B. When the incubation time was increased to 24 h, the toxicity of the clinical grade amphotericin B increased to 100% but there was no further increase in the toxicity of the amphotericin B - PMAA-Na preparation.
Figure 13:

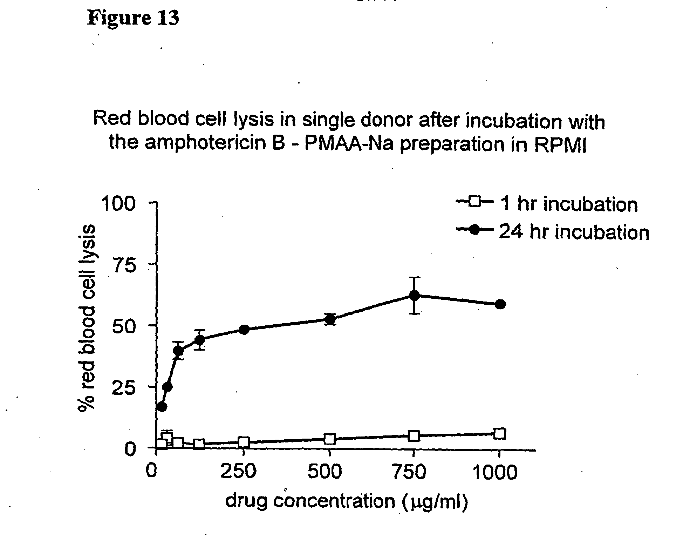

   Figure 13 shows red blood cell lysis in a single donor after incubation with amphotericin B-PMAA-Na complex.
   The method was as described for Figure 11, the amphotericin B-PMAA-Na complex being prepared as described in Example C. The degree of red cell lysis by the amphotericin B - PMAA-Na preparation ("drug") was determined after a 1 hour and 24 hour incubation for concentrations up to 1,000 µg/ml. The results obtained after a 1 hour incubation are shown as open squares, the results after a 24 hour incubation as closed circles.
Figure 14:

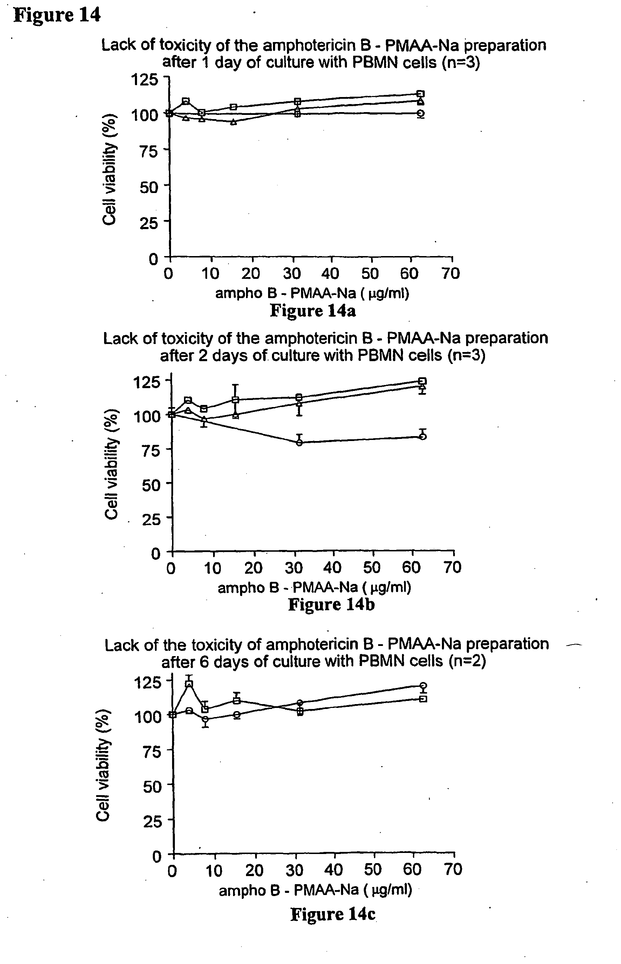

   Figure 14 shows the lack of toxicity of amphotericin B-PMAA-Na complex after 1, 2 and 6 days culture with PBMN cells.
   PBMCs in RPMI medium were cultured with media containing the amphotericin B - PMAA-Na complex prepared as described in Example C ("drug") over the concentration range of 0 to 70 µg/ml. The cells were incubated for 1 day or 2 days or 6 days prior to the addition of MTT (5 mg/ml). The compound was not toxic to PBMCs at the highest concentration of 70 µg/ml tested using the MTT assay and the Trypan blue assay after 1 day of culture, 2 days of culture or after 6 days of culture. Figure 14 shows the individual results from up to 3 representative human donors. Each line represents a single donor. Figures 14a, 14b and 14c show the results after incubation for 1 day, 2 days and 6 days, respectively.
Figure 15:

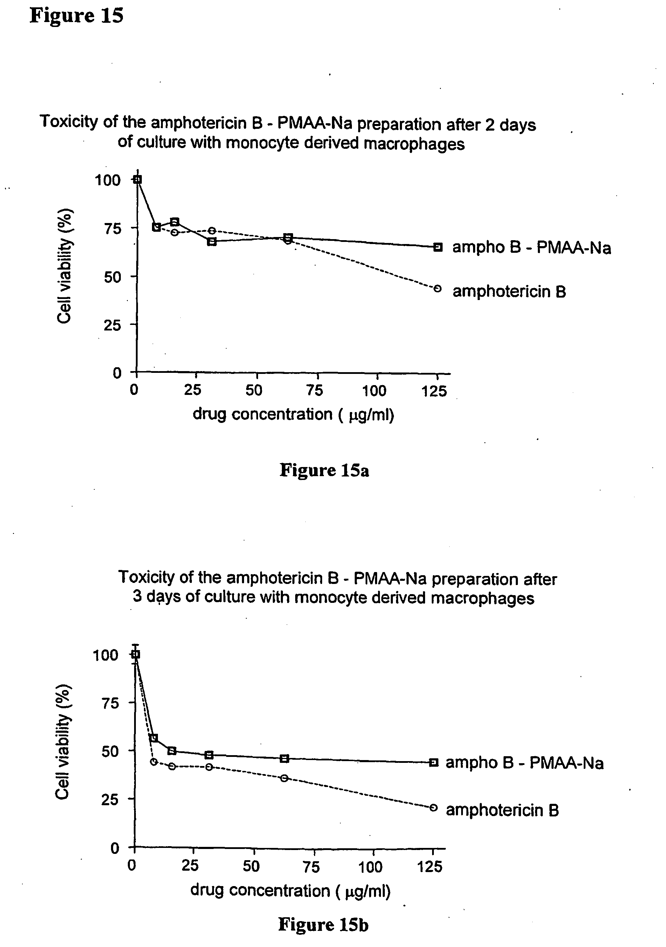

   Figure 15 shows toxicity of the amphotericin B-PMAA-Na complex after 2 and 3 days culture with monocyte derived macrophages.
   Monocyte derived macrophages (MDM) were cultured with media containing the compounds ("drugs") up to a concentration of 125 µg/ml. The cells were incubated for 2 days or 3 days prior to the addition of MTT. The results obtaining using amphoteracin B are shown as open circles, the results with the amphoteracin B-PMAA-Na complex prepared as described in Example C are shown as open squares. Figure 15a shows the results obtained after 2 days culture, Figure 15b the results after 2 days culture. The amphotericin B - PMAA-Na preparation was less toxic than clinical grade amphotericin B at all of the concentrations up to 125 µg/ml tested using the MTT assay and the Trypan blue assay after both 2 days and after 3 days of culture.
Figure 16:

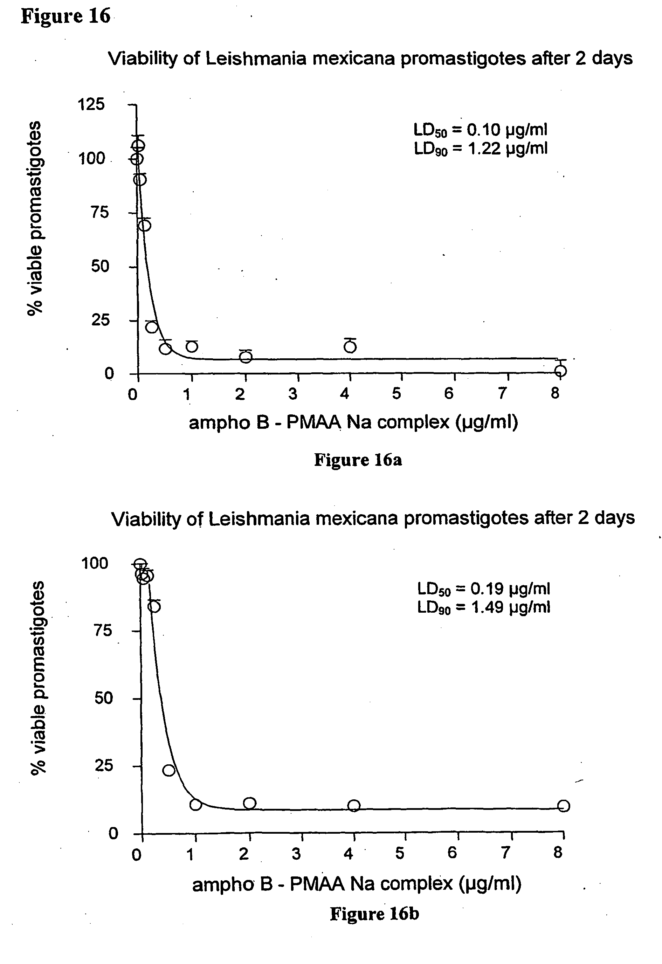

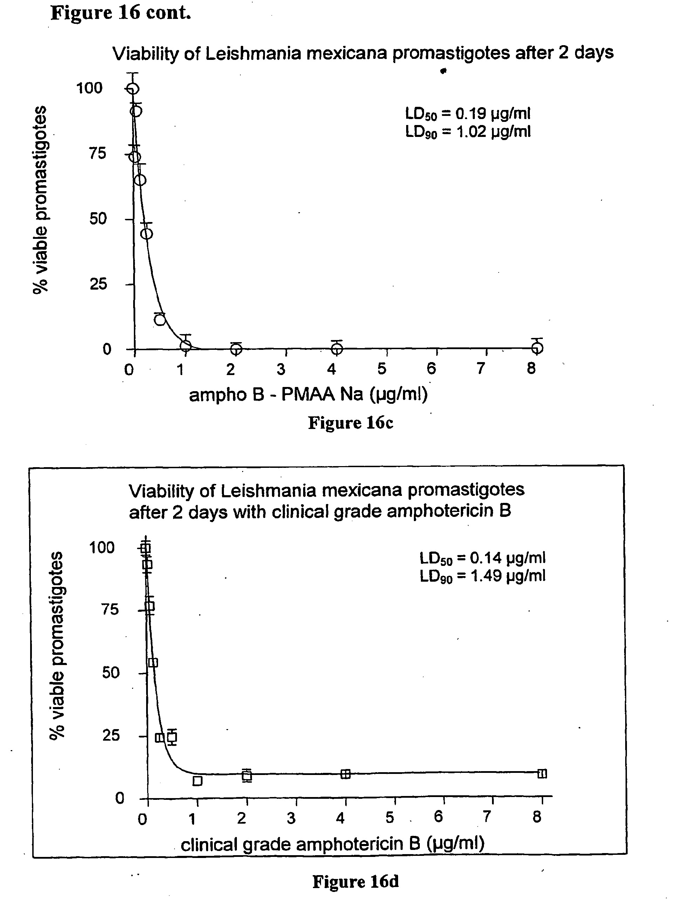

   Figure 16 shows viability of *Leishmania mexicana* promastigotes treated with amphotericin or with amphotericin B - PMAA-Na complex.
   The *Leishmania mexicana* promastigotes used for these experiments were maintained in Schneider's Drosophila growth medium (Invitrogen) supplemented with 15% fetal calf serum (heat inactivated at 56°C for 1 hour) and gentamicin (1 mg/100 ml). The 50% lethal dose (LD₅₀) of clinical grade amphotericin B for *Leishmania mexicana* promastigotes was 0.14 µg/mi (Figure 16d). The 90% lethal dose (LD₉₀) of clinical grade amphotericin B for *Leishmania mexicana* promastigotes was 1.49 µg/ml (Figure 16d). The results from 3 experiments with the amphotericin B - PMAA-Na complex prepared as described in Example C are shown in Figures16a, 16b and 16c. The 50% lethal dose (LD₅₀) of the amphotericin B - PMAA-Na preparation for *Leishmania mexicana* promastigotes was 0.10 to 0.19 µg/ml (Figures 16a, 16b and 16c ). The 90% lethal dose (LD₉₀) of amphotericin B - PMAA-Na preparation for *Leishmania mexicana promastigotes* was 1.02 to 1.49 µg/ml (Figures 16a, 16b and 16c). The activity of the amphotericin B - PMAA-Na preparation against Leishmania mexicana promastigotes was similar to that of clinical grade amphotericin B on a weight per weight basis.
Figure 17:

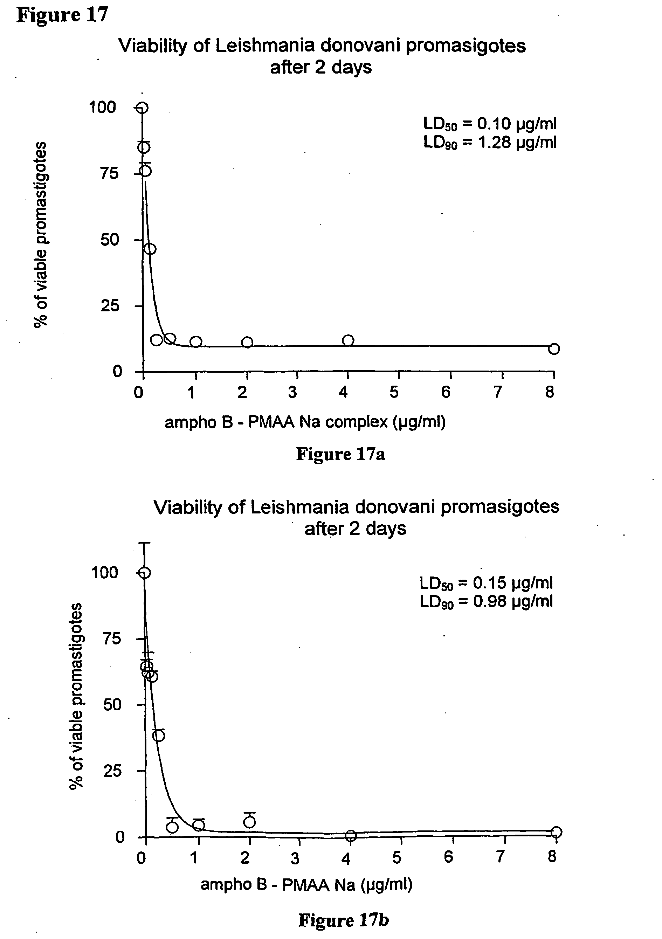

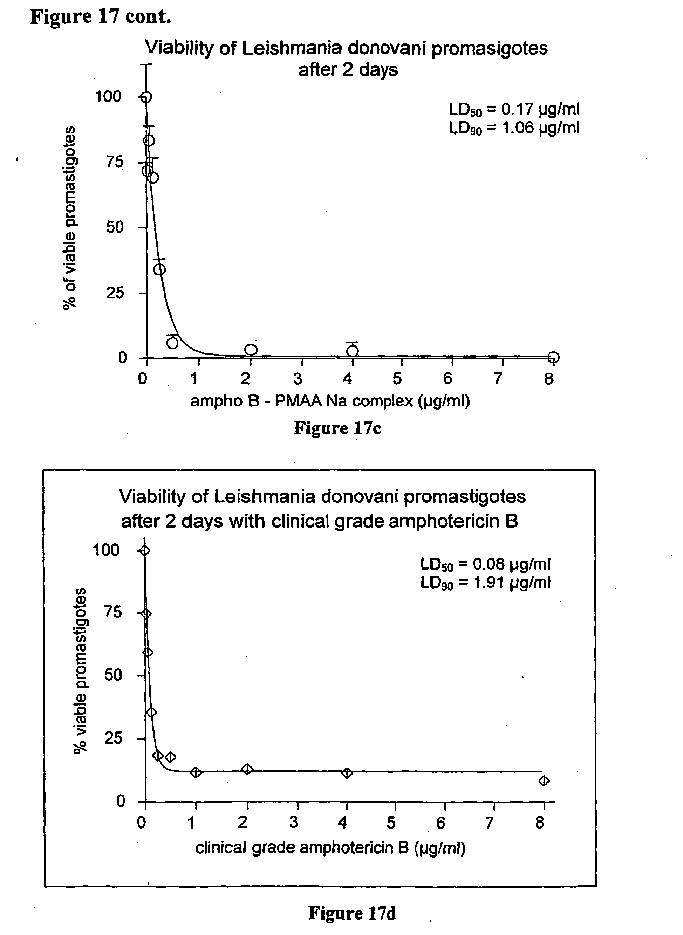

   Figure 17 shows viability of *Leishmania donovani* promastigotes treated with amphotericin or with amphotericin B - PMAA-Na complex.
   The *Leishmania donovania* promastigotes used for these experiments were maintained in Schneider's Growth Media 199 supplemented with 15% fetal calf serum (heat inactivated at 56°C for 1 hour) and gentamicin (1 mg/100 ml). The 50% lethal dose (LD₅₀) of clinical grade amphotericin B for *Leishmania donovani* promastigotes was 0.08 µg/ml (Figure 17d). The 90% lethal dose (LD₉₀) of clinical grade amphotericin B for Leishmania donovania promastigotes was 1.91 µg/ml (Figure 17d). The results from 3 experiments with the amphotericin B - PMAA-Na complex prepared as described in Example C are shown in Figures 17a, 17b and 17c. The 50% lethal dose (LD₅₀) of amphotericin B - PMAA-Na preparation for Leishmania donovani promastigotes was 0.10 to 0.17 µg/ml (Figures 17a, 17b and 17c). 90% lethal dose (LD₉₀) of amphotericin B - PMAA-Na preparation for Leishmania donovani promastigotes was 0.98 to 1.28 µg/ml (Figures 17a, 17b and 17c). The activity of the amphotericin B - PMAA-Na preparation against Leishmania donovani promastigotes was therefore similar to that of clinical grade amphotericin B on a weight-per-weight basis.
Figures 18 & 19:

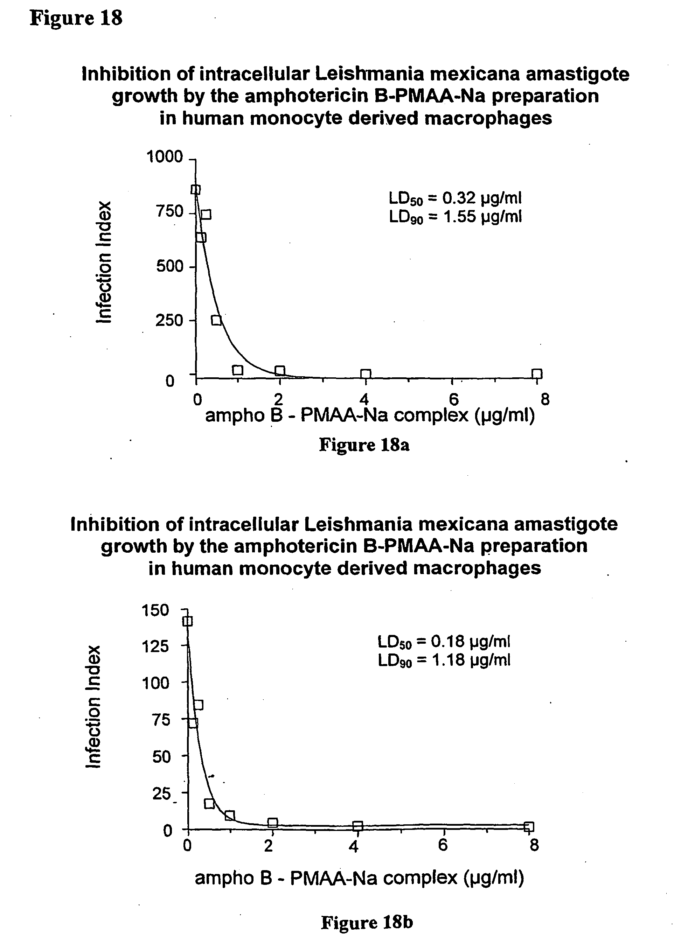

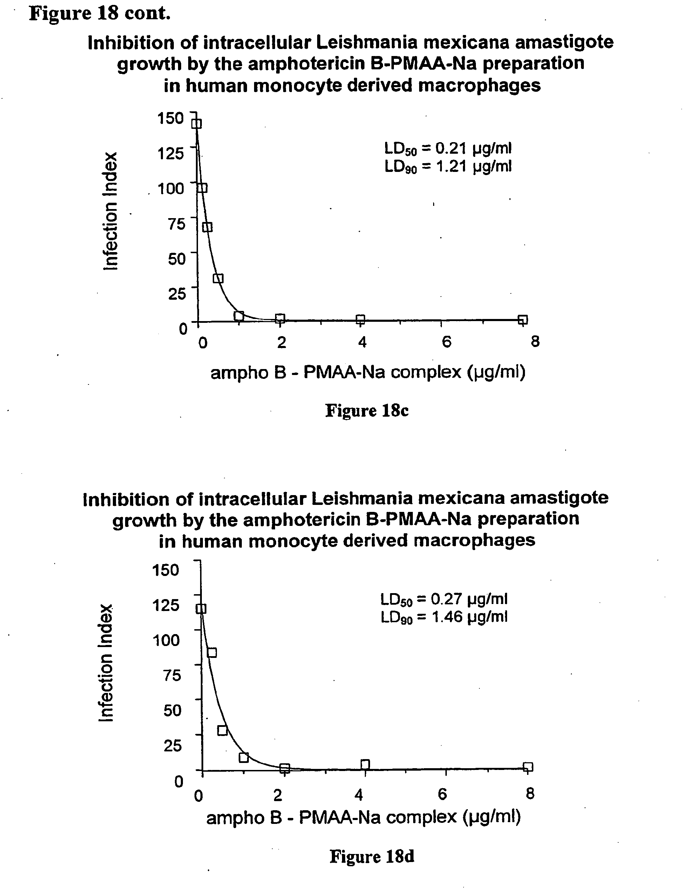

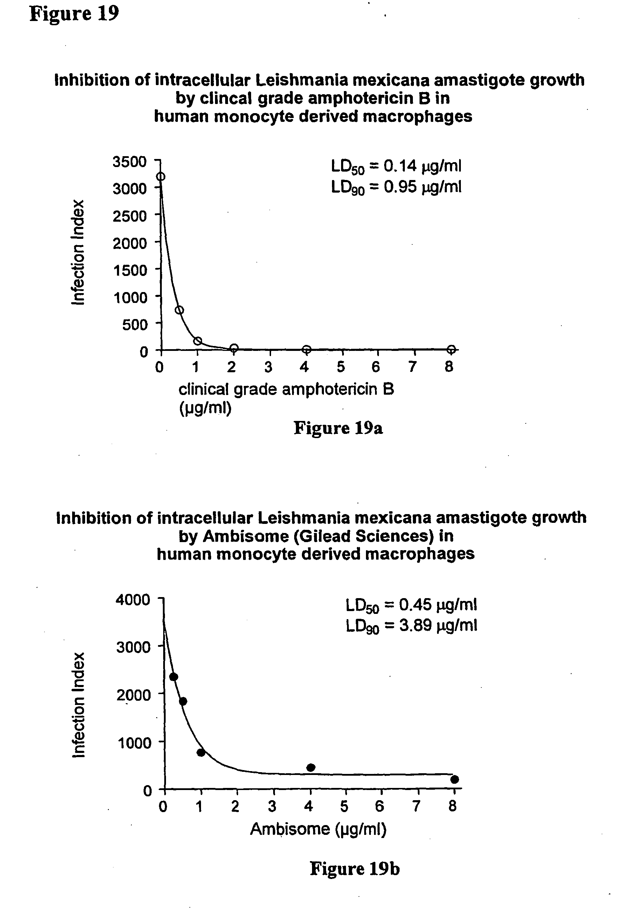

   Figure 18 shows inhibition of intracellular *Leishmania mexicana* amastigote growth by amphotericin B-PMAA-Na complex in human monocyte derived macrophages. Figure 19 shows the corresponding inhibition using clinical grade amphotericin B or AmBiosome (liposomal amphotericin B, Gilead Sciences, Great Abingdon, Cambridge, UK).
   *Leishmania mexicana* amastigotes were used to infect human monocyte derived macrophages that were maintained in RPMI 1640 (Invitrogen) supplemented with 10% human serum (heat inactivated at 56°C for 1 hour) and 200 IU/ml penicillin and 200 µg/ml streptomycin. The infection index was calculated as the average - number of parasites/cell multiplied by the percentage of infected cells. The results of four experiments using the amphotericin B-PMAA-Na complex prepared as described in Example C are shown in Figures 18a to 18d. Inhibition using clinical grade amphotericin B is shown in Figure 19a and using AmBiosome (Gliead Sciences) in Figure 19b. A 50% inhibition of intracellular *Leishmania mexicana* amastigote growth was achieved with 0.18 to 0.32 µg/ml of the amphotericin B = PMAA-Na preparation ) (Figures 18a to 18d) compared to 0.14 µg/ml of clinical grade amphotericin B (Figure 19a) or 0.45 µg/ml of Ambisome (Figure 19b). A 90% inhibition of intracellular *Leishmania mexicana* amastigote growth was achieved using 1.18 to 1.55 µg/ml of the amphotericin B - PMAA-Na preparation (Figures 18a to 18d) compared to 0.95 µg/ml of clinical grade amphotericin B (Figure 19a) or 3.89 µg/ml of AmBisome (Figure 19b).
Figure 20:

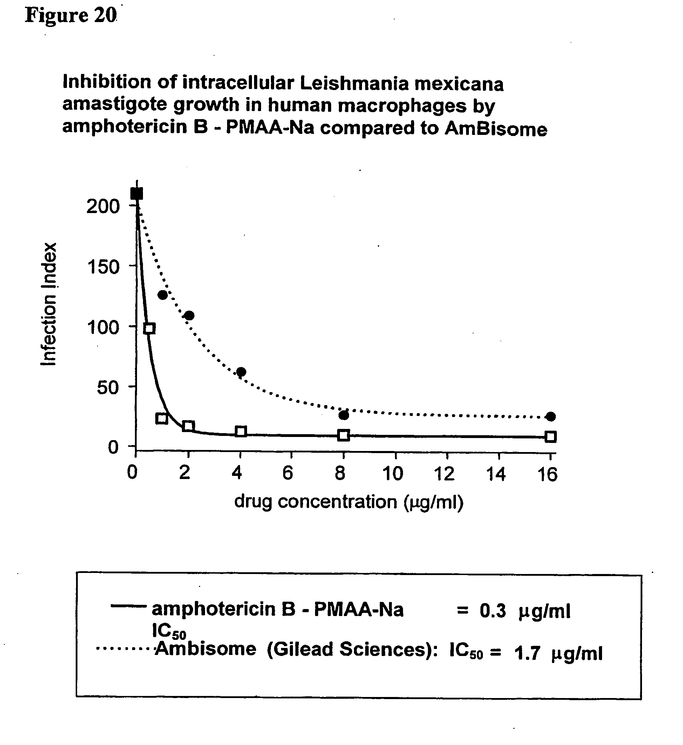

   Figure 20 shows inhibition of intracellular *Leishmania mexicana* amastigote growth in human macrophages by amphotericin B -PMAA-Na complex compared to AmBiosome.
   The graph shown in Figure 20 compares the slopes of relative activity of the amphotericin B - PMAA-Na complex prepared as described in Example C (shown as open squares; IC₅₀= 0.3 µg/ml) and AmBisome (liposomal amphotericin B, Gilead Sciences, Great Abingdon, Cambridge, UK) (shown as closed circles; IC₅₀= 1.7 µg/ml) against intracellular *Leishmania mexicana* amastigotes. It demonstrates that the killing curve for the amphotericin B - PMAA-Na preparation is steeper than the killing curve for AmBisome.
Figures 21 & 22:

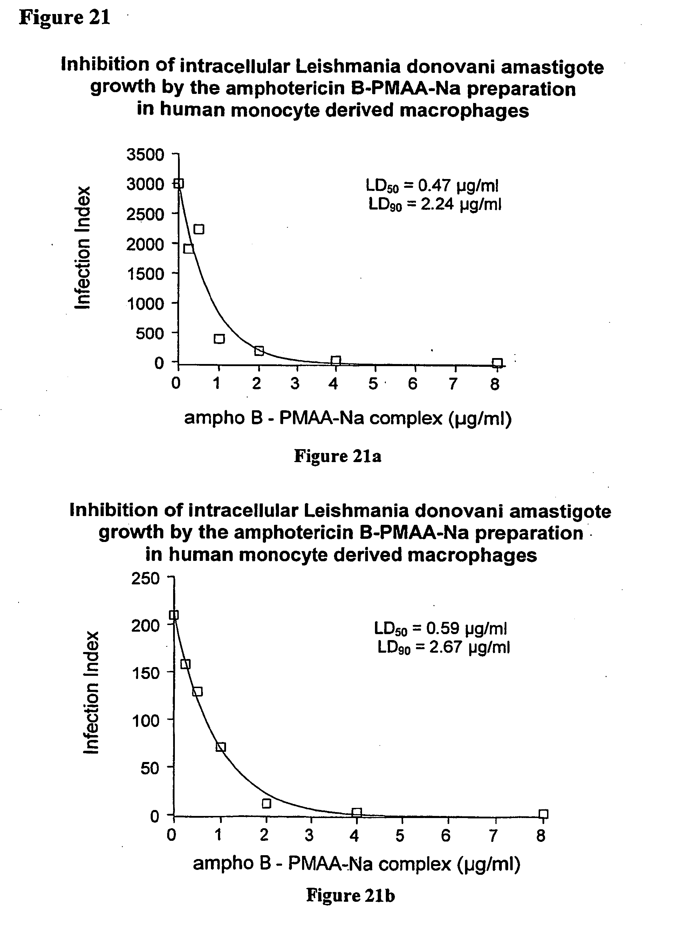

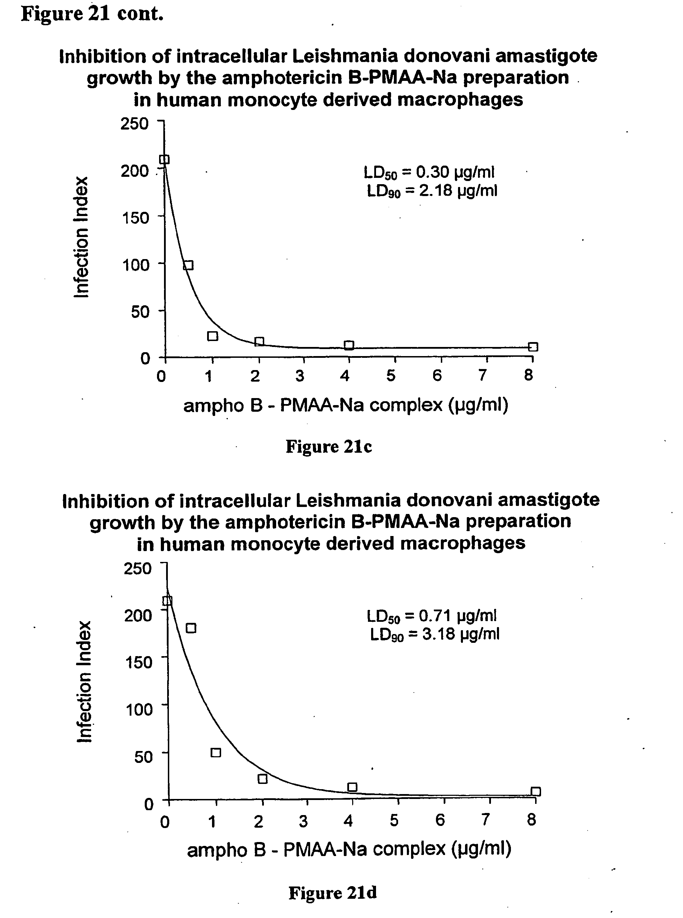

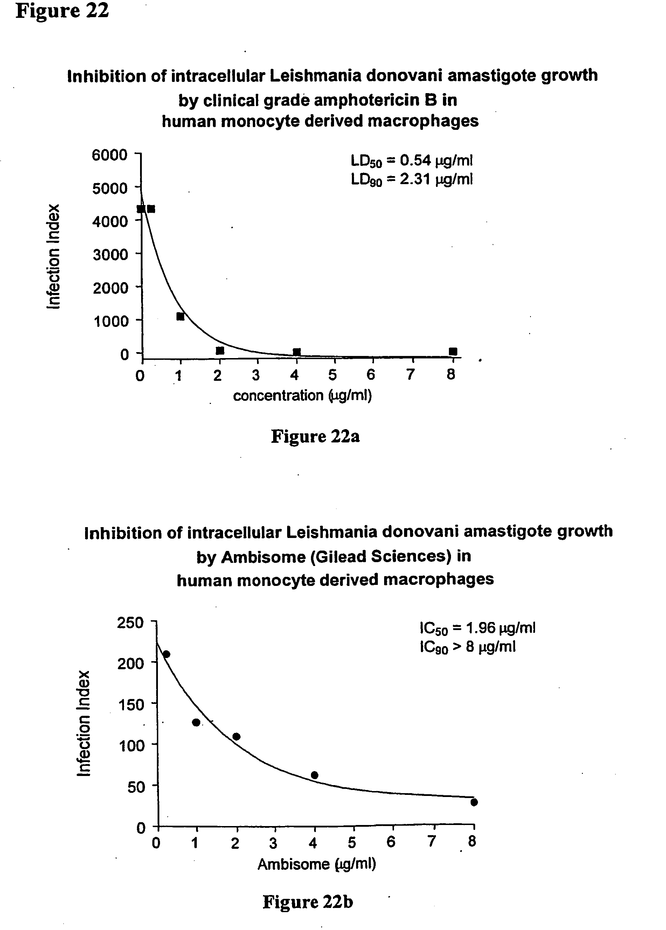

   Figures 21 and 22 show inhibition of intracellular *Leishmania donovani* amastigote growth by amphotericin B-PMAA-Na complex in human monocyte derived macrophages (four experiments, shown in Figures 21 a to 21d), by clinical grade amphotericin B (Figure 22a) and by AmBiosome (Gilead Sciences, as above) (Figure 22b).
   *Leishmania donovani* amastigotes were used to infect human monocyte derived macrophages that were maintained in RPMI 1640 (Invitrogen) supplemented with 10% human serum (heat inactivated at 56°C for 1 hour) and 200 IU/ml penicillin and 200 µg/ml streptomycin. The infection index was calculated as the average number of parasites/cell multiplied by the percentage of infected cells. A 50% inhibition of intracellular *Leishmania donovani* amastigote growth was achieved with 0.30 to 0.71 µg/ml of the amphotericin B - PMAA-Na preparation prepared as described in Example C (Figures 21a to 21d) compared to 0.54 µg/ml of clinical grade amphotericin B (Figure 22a) or 1.96 µg/ml of AmBisome (Figure 22b). A 90% inhibition of intracellular *Leishmania donovani* amastigote growth was achieved using 2.18 to 3.18 µg/ml of the amphotericin B - PMAA-Na complex prepared as described in Example C (Figures 21a to 21d) compared to 2.31 µg/ml of clinical grade amphotericin B (Figure 22a) or >8 µg/ml of Ambisome (Figure 22b).
Figure 23:

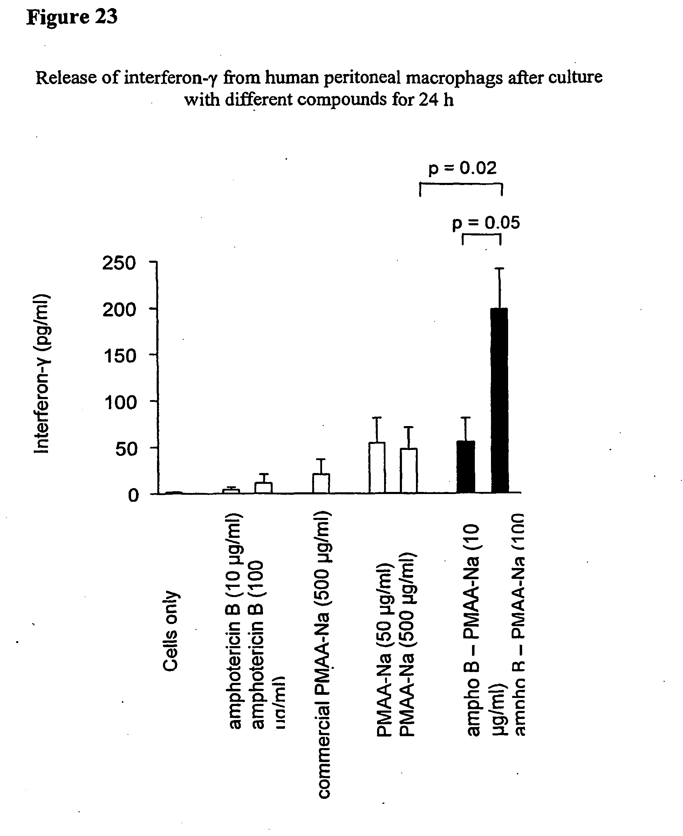

   Figure 23 shows the release of interferon-γ from human peritoneal macrophages after culture with different compounds for 24 hours.
   Human peritoneal cells were cultured in macrophage growth medium with each compound at the concentrations shown. The compounds used were the amphotericin B - PMAA-Na complex prepared as described in Example C, clinical grade amphotericin B, commercial PMAA-Na, and PMAA-Na prepared as described in Examples A1 to A4. All reagents and compounds contained <0.06 endotoxin unit/ml. The culture supernatants were harvested 24 h later. Interferon-γ was measured by EIA (R & D systems). The release of the Th1 promoting cytokine, interferon-y, from peritoneal macrophages was significantly higher in the presence of the amphotericin B - PMAA-Na preparation (100 µg/ml) compared to cells only control, clinical grade amphotericin B, commercial PMAA-Na, or the PMAA-Na.
Figures 24:

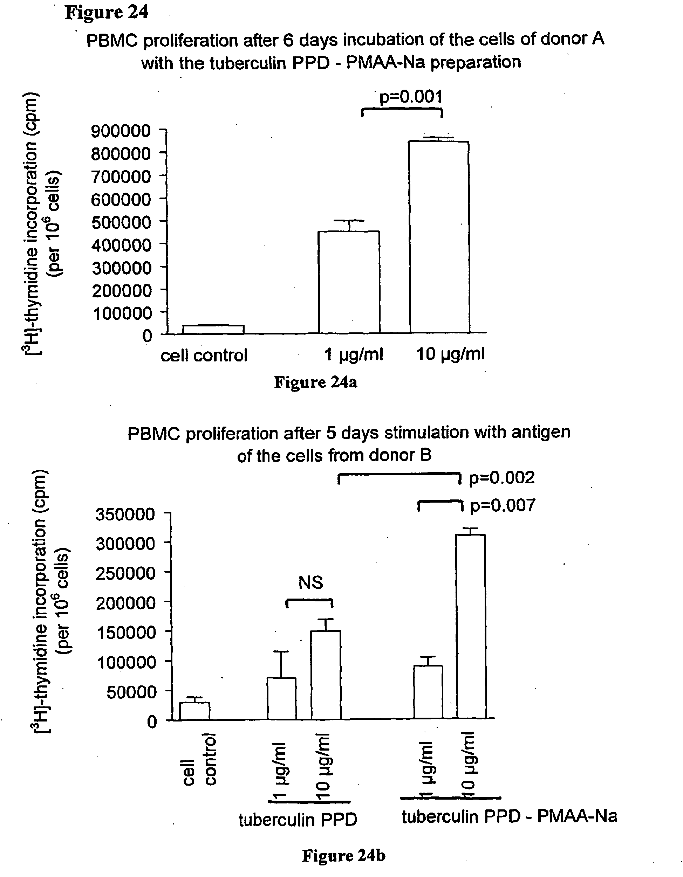

   Figure 24 shows PMBC proliferation of cells after incubation with tuberculin PMAA-Na complex.
   PBMCs were resuspended in RPMI 1640 supplemented with 10% human serum, 200 µg/ml penicillin and 200 IU/ml streptomycin. Tuberculin PMAA-Na complex (also called tuberculin PPD-PMAA-Na) was prepared as described in Example L2. All reagents and compounds contained <0.06 endotoxin unit/ml as determined using the *Limulus* amebocyte lysate assay (Pyrotell, Associates of Cape Cod, US).
   The cells (10⁵ cells/well) and each compound (tuberculin PPD and tuberculin-PMAA-Na at the concentrations shown) were mixed in duplicate and incubated at 37°C / 5% CO₂ for 4 days. [³H]-Thymidine (specific activity 20-30 Ci/mmol; Amersham Biosciences, UK) was added at 1 µCi/well for a further 18 hours. The cells were then harvested and proliferation determined using a liquid scintillation counter. The results were expressed as the mean counts/minute (cpm) ± sem.
   Figure 24a shows the results obtained for the PBMC proliferation after 6 days incubation of the cells of donor A with the tuberculin-PMAA-Na preparation. Figure 24a shows that there was significantly increased proliferation of T lymphocytes when they were cultured with 1 µg/ml of the tuberculin-PMAA-Na preparation. There was even more proliferation of T lymphocytes when they were cultured with 10 µg/ml of tuberculin-PMAA-Na preparation (P = 0.001).
   Figure 24b shows the results for the PBMC proliferation after 5 days incubation of the cells of donor B with tuberculin PPD and with the tuberculin -PMAA-Na preparation. There was significantly more T lymphocyte proliferation with 10 µg/ml of the tuberculin-PMAA-Na preparation than with 10 µg/ml of tuberculin antigen (P = 0.002). This shows that the tuberculin-PMAA-Na preparation causes significantly more T lymphocyte proliferation than the tuberculin antigen on its own.
Figure 25:

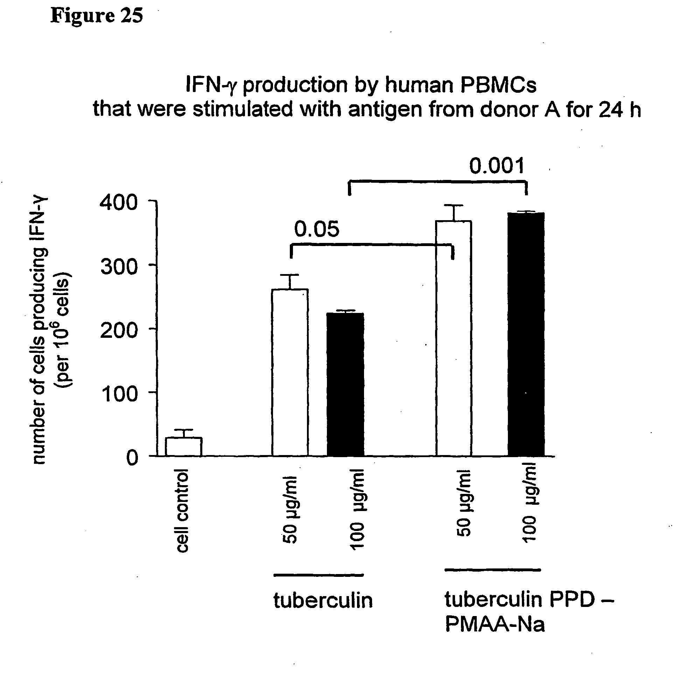

   Figure 25 shows IFN-γ production by human PMBCs that were stimulated with antigen from donor A for 24 hours.
   Human PBMCs were isolated and adjusted to 2 x 10⁵ cells/well in RPMI 1640 supplemented with 10% human serum, 200 µg/ml penicillin and 200 IU/ml streptomycin. All reagents and the compounds contained <0.06 endotoxin unit/ml. The PBMCs and the compounds (tuberculin and tuberculin-PMAA-Na complex, also called tuberculin PPD-PMAA-Na, prepared as described in Example L2, at the concentrations shown) were mixed in the wells of an ELlSpot PVDF-backed microplate coated with the human interferon-γ monoclonal antibody (R&D Systems, UK). Unstimulated cells were used as the negative control and recombinant human interferon-γ was used in the positive control. The plate was incubated for 24 hours at 37°C / 5% CO₂. The manufacturer's instructions were then followed to develop the microplate and the number of positive spots for interferon-y counted. Each spot represented a single interferon-γ secreting cell.
   Figure 25 shows the number of cells producing IFN-γ. Figure 25 shows that the tuberculin PPD - PMAA-Na preparation was significantly more effective than tuberculin antigen alone in stimulating interferon-γ release from primary human T lymphocytes. This increase in interferon-γ secretion from T lymphocytes was seen with 50 µg/ml of the tuberculin PPD - PMAA-Na preparation and with 100 µg/ml of the tuberculin PPD - PMAA-Na preparation.
Figure 26:

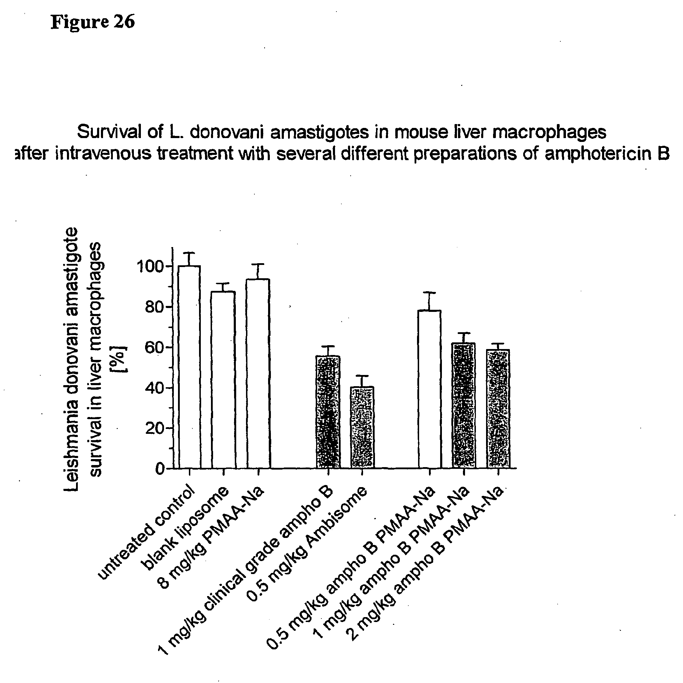

   Figure 26 shows the survival of *Leishmania donovani* amastigotes in mouse liver macrophages after intravenous treatment with clinical grade amphotericin B, AmBisome or amphotericin B-PMAA-Na, with untreated controls and blank liposomes as controls.
   The various compounds were used at the concentrations shown in Figure 26. The number of amastigotes/500 liver cells were counted microscopically and the results expressed as a percentage of the untreated control. There was significant killing activity (P<0.0001) of amphotericin B - PMAA-Na complex prepared as described in Example C at 1 mg/kg of *Leishmania donovani* amastigotes compared to the controls. These results show that the amphotericin B - PMAA-Na preparation was effective in this animal model of visceral leishmaniasis.
Figure 27:
   The toxicity of PMAA-Na constructs of different molecular weights for red blood cells was established using an MTT assay.

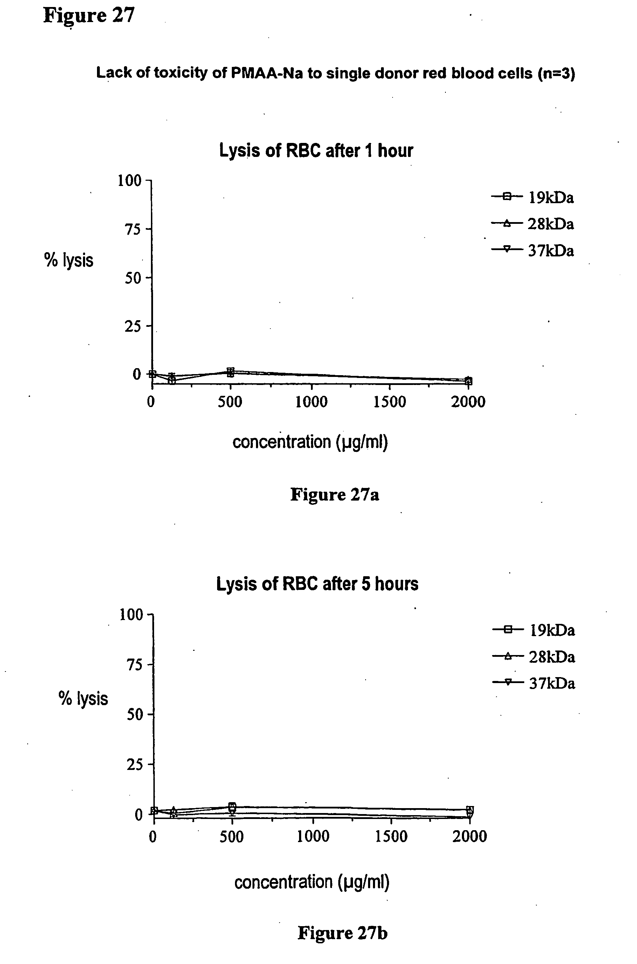

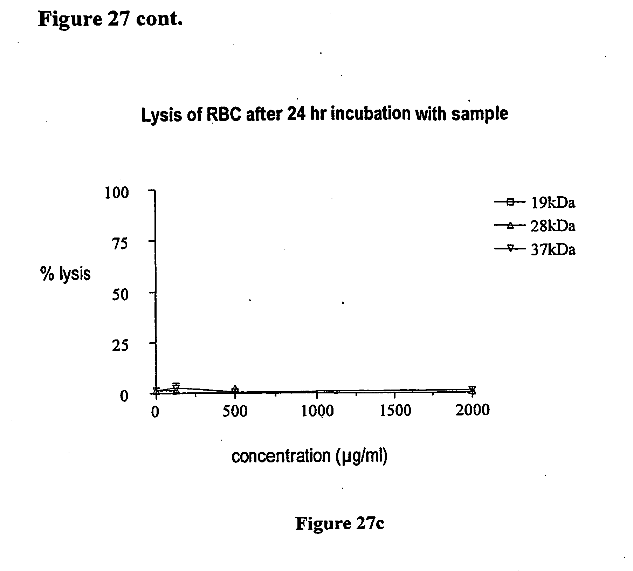

   Figures 27a to 27c show the lack of toxicity of PMAA-Na constructs having varying molecular weights and produced as described in Example N to single donor red blood cells. In each case the concentration of the PMAA-Na construct is given in µg/ml and the lysis of the red blood cells is given as a percentage. The squares represent the results obtained using a PMAA-Na construct of molecular weight 19kDa, the triangles the results using a 28 kDa PMAA-Na construct, and the inverted triangles the results using a 37 kDa PMAA-Na construct (n=3). Figure 27a shows the lysis-of the red blood cells (RBCs) after incubation for 1 hour with the PMAA-Na constructs, Figure 27b shows the lysis of the RBCs after 5 hours and Figure 27c shows the lysis of the RBCs after 24 hours The PMAA-Na constructs did not cause red blood cell lysis at a concentration of 2 mg/ml after a 1 h, 5 h and a 24 h incubation. These results were not affected by the molecular weight of the PMAA-Na constructs made.
Figure 28:
   The toxicity of PMAA-Na constructs of different molecular weights for peripheral blood mononuclear cells (PMBCs) was established using an MTT assay.

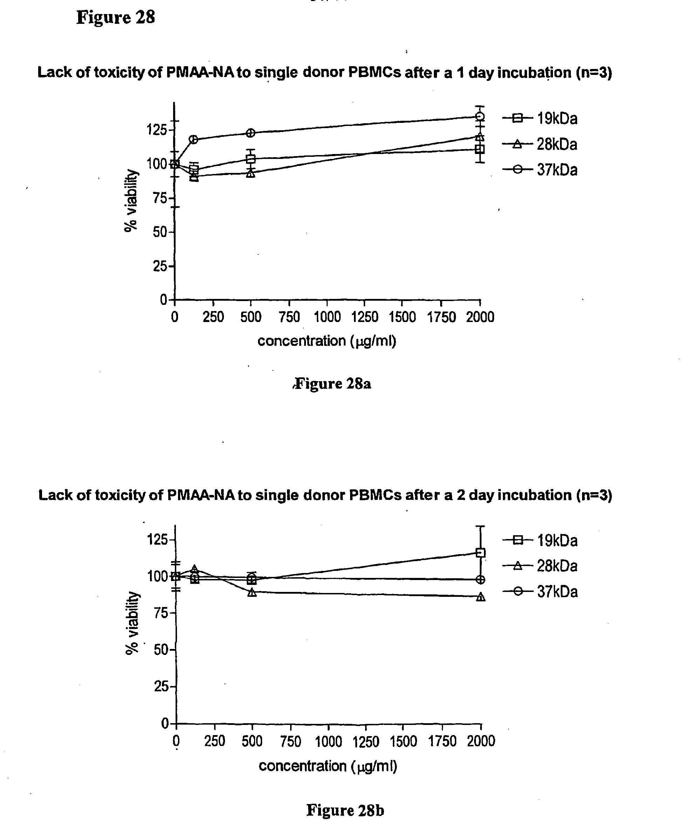

   In Figures 28a and 28b, the concentration of the PMAA-Na constructs, produced as described in Example, N, is plotted against the percentage of the PMBCs. The squares represent the results obtained using a PMAA-Na construct of molecular weight 19kDa, the triangles the results using a 28 kDa PMAA-Na construct, and the circles the results using a 37 kDa PMAA-Na construct (n=3). Figure 28a shows the viability of the PMBCs after incubation for 1 day with the PMAA-Na constructs, Figure 28b shows the viability of the PMBCs after incubation for 2 days with the constructs. The constructs used were not toxic to peripheral blood mononuclear cells at 2 mg/ml after incubation for 1 day and for 2 days. The results were not affected by the molecular weight of the PMAA-Na constructs made.
Figure 29:

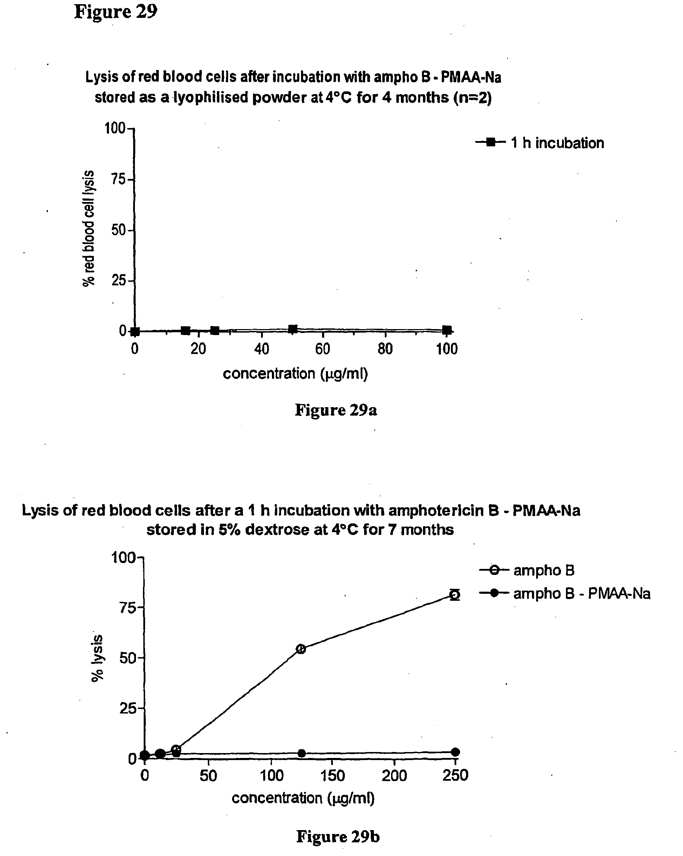

   Figure 29 shows the effect of storage of an amphotericin B - PMAA-Na complex under different conditions on its toxicity to red blood cells.
   Figure 29a shows the lack of toxicity to red blood cells (RBCs) of a PMAA-Na complex that had been prepared as desceibed in Example C and stored as a lyophilized powder at 4°C for 4 months. The complex was incubated with the RBCs for 1 hour at various concentrations and the percentage lysis of the RBCs was measured. Figure 29a shows that the amphotericin B - PMAA-Na complex was not toxic to red blood cells after storage as a lyophilized powder at 4°C for 4 months.
   Figure 29b shows the results obtained when amphotericin B - PMAA-Na complex that had been prepared according to Example C and stored in 5% dextrose at 4°C for 7 months was incubated with RBCs for one hour. Lysis is shown as a percentage. Freshly prepared, clinical grade amphotericin B was used as a reference for the comparison. Incubations were undertaken for 1 h and 6 h; the results for the 1 hour incubation are shown. Figure 29b shows that the amphotericin B - PMAA-Na complex was not toxic to red blood cells after storage in 5% dextrose at 4°C for 7 months.
Figure 30:

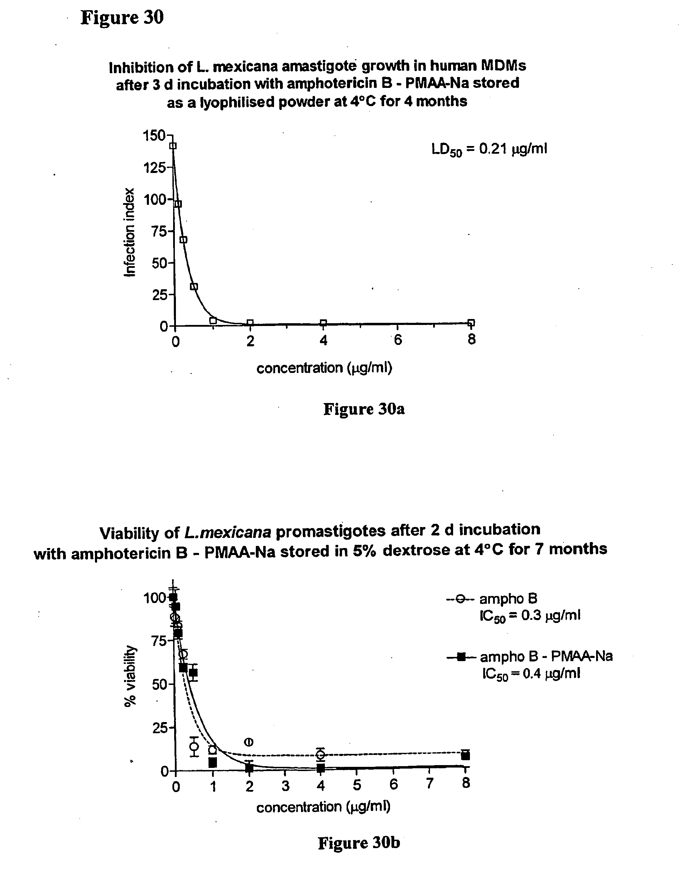

   Figure 30 shows the inhibition of *Leishmania* amastigotes and promastigotes by an amphotericin B - PMAA-Na complex that had been stored under different conditions.
   Figure 30a shows the inhibition of the growth of *Leishmania mexicana* intracellular amastigotes in MDMs by the amphotericin B - PMAA-Na complex that had been prepared according to Example C and stored as a lyophilized powder for 4 months at 4°C. The infection index is plotted against the concentration of the complex. The LD₅₀ was 0.21 µg/ml.
   Figure 30b shows the viability of *Leishmania mexicana* promastigotes after 2 days incubation with intracellular amastigotes in MDMs by the amphotericin B - PMAA-Na complex that had been prepared according to Example C and stored for 7 months at 4°C in 5% dextrose.
   Percent viability is plotted against the concentration of the complex. Freshly prepared, clinical grade amphotericin B was used as a reference for the comparison. The LD₅₀ of the complex was 0.4 µg/ml,. the LD₅₀ of the amphoteracin B was 0.3 µg/ml. The results obtained with the complex are shown by solid squares, the results with ampoteracin B as open circles.
   The activity of amphotericin B - PMAA-Na was not affected by storage as a lyophilized powder for 4 months at 4°C for 7 months at 4°C in 5% dextrose.
Figure 31:

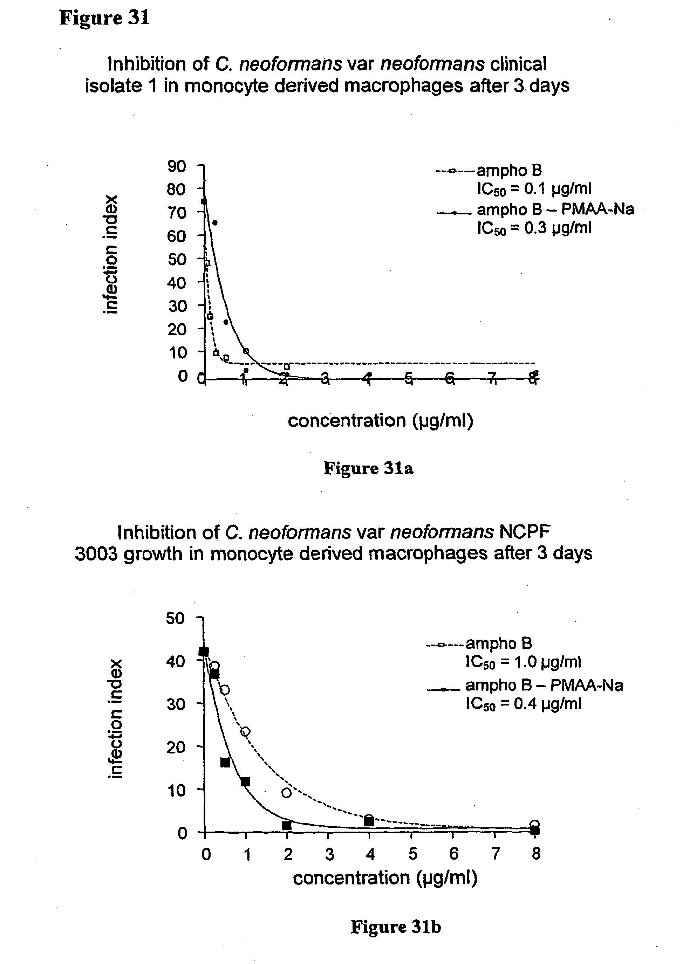

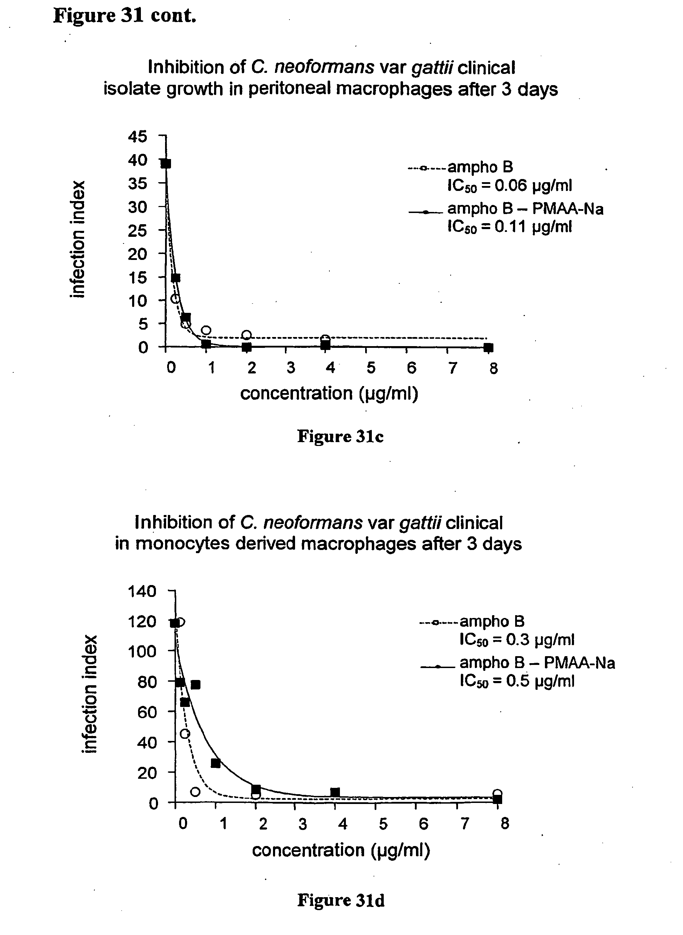

   Figure 31 shows the inhibition of various strains of *Cryptococcus neoformans* that have infected human monocyte derived macrophages.
   The inhibition was measured after using various concentrations of amphotericin B - PMAA-Na complex prepared as described in Example C (solid squares) for three days, with amphoteracin B (open circles) as a reference for comparison. The number of infected and uninfected macrophages, and the number of gram positive yeast CFU were counted. The results were expressed as the infection index, which was calculated as the average number of CFU/percentage of infected cells. The LD₅₀ values were also determined.
   Figure 31a shows the inhibition of *C. neoformans* var *neoformans* clinical isolate 1,
   Figure 31b shows the inhibition of *C. neoformans* var *neoformans NCPF 3003,*
   Figure 31c shows the inhibition of *C. neoformans* var *gattii* clinical isolate, and
   Figure 31d shows the inhibition of *C. neoformans* var *gattii* clinical isolate. The LD₅₀ for amphotericin B (0.9 - 1.4 µg/ml) and for amphotericin B - PMAA-Na (1.6 - 2.7 µg/ml are similar. Therefore, the complex is as active against cryptococci as amphotericin B alone.
Figure 32

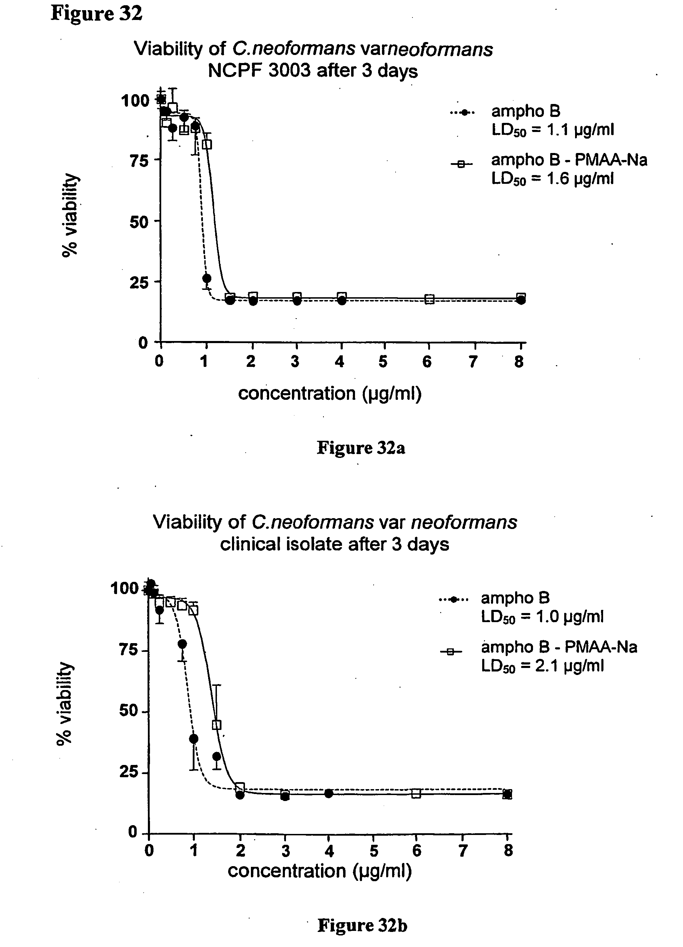

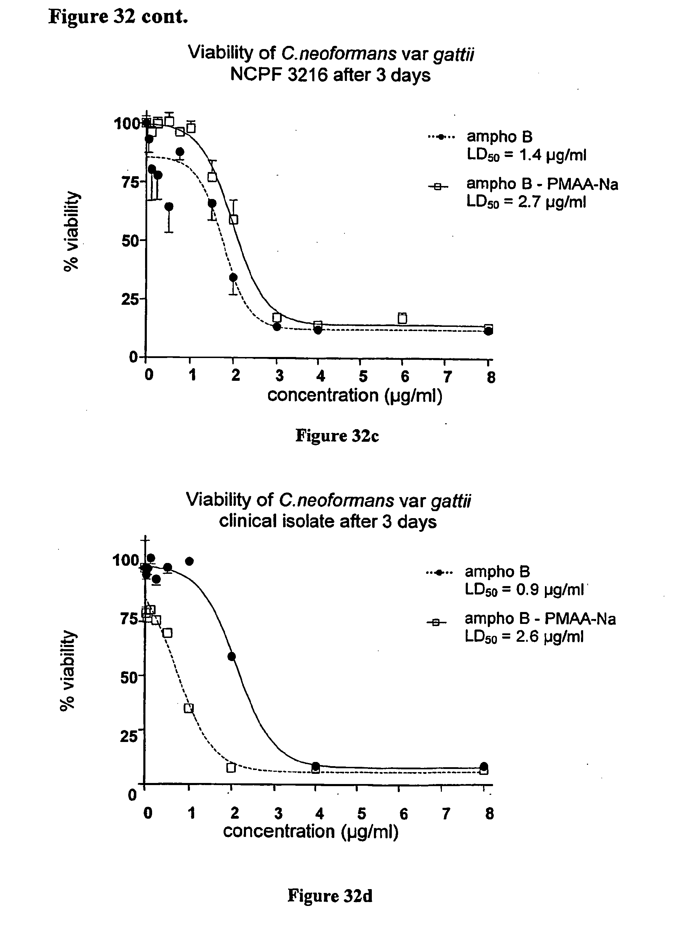

   Figure 32 shows the viability of various *Cryptococcus neoformans* strains that have infected human monocyte derived macrophages.
   The viability was measured after using various concentrations of amphotericin B - PMAA-Na complex prepared as described in Example C (solid squares) for three days, with amphoteracin B (open circles) as a reference for comparison..
   Figure 32a shows the results for *C. neoformans* var *neoformans NCPF 3003,*
   Figure 32b shows the results for *C. neoformans* var *neoformans* clinical isolate,
   Figure 32c shows the results for *C. neoformans* var *gatti* NCPF 3216, and Figure 32d shows the results for *C*. *neoformans* var *gatti* clinical isolate when those organisms have infected human monocyte derived macrophages. The LD₅₀ for amphotericin B and for amphotericin B - PMAA-Na were similar in all of the 4 experiments performed and also similar to the 4 experiments shown in Figures 31 a to 31d. In the case of *C*. *neoformans* var *neoformans,* the LD₅₀ for amphotericin B was 0.9 to 1.4 µg/ml and that for amphotericin B - PMAA-Na was similar at 1.6 to 2.7 µg/ml. For *C*. *neoformans* var *gatti* the LD₅₀ for amphotericin B was 0.06 to 1.0 µg/ml and that for amphotericin B - PMAA-Na was similar at 0.1 to 0.5 µg/ml. Therefore, the complex is as active against cryptococci as amphotericin B alone.
Figure 33:

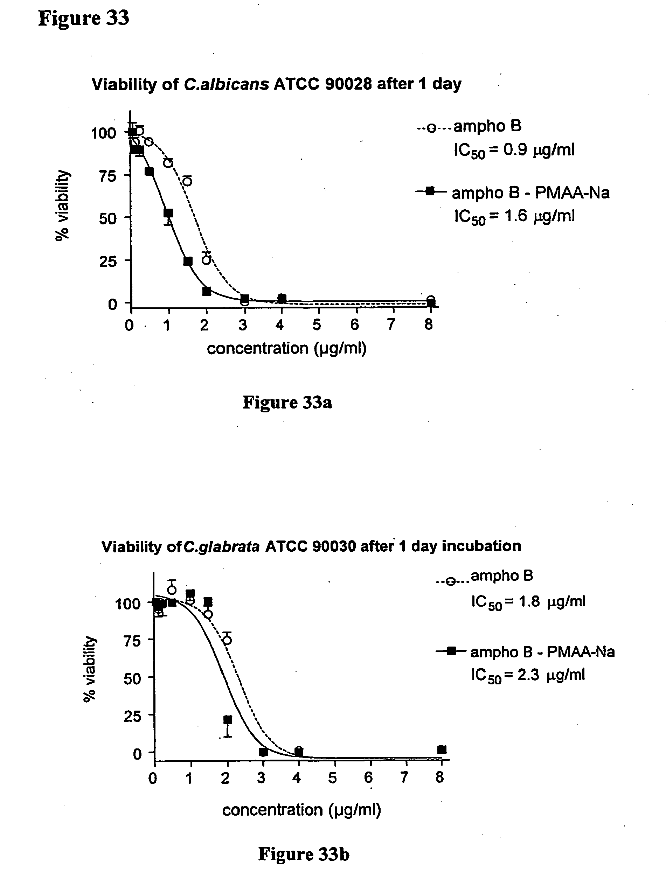

   Figure 33a shows the viability of *Candida albicans* ATCC 90028 and Figure 33b the viability of *Candida glabrata* ATCC 90030 after treatment for one day with amphoteracin B (reference) or amphotericin B - PMAA-Na complex at various concentrations.
   Viability is expresses as a percentage. Viability was determined by turbidity as measured using a spectrophotometer (490 nm). 100% viability was established using the optical density of the untreated yeast in control wells.
   The LD₅₀ for amphotericin B(0.9 to 1.8 µg/ml) and for amphotericin B - PMAA-Na complex prepared as described in Example C (1.6 to 2.3 µg/ml) are similar for *Candida albicans* (Figure 33a) and Figure 33b shows the results for *Candida glabrata..* Therefore, the complex is as active against Candida sp. as amphotericin B alone.

### Detailed description of the invention

As disclosed above, the present invention relates to various uses of a narrow molecular weight distribution polymer that includes units derived from an acrylic acid or a salt thereof in treatment of infections by pathogenic organisms, in treatment of cancer, and/or as an immune potentiating adjuvant, and also relates to complexes that comprise a narrow molecular weight distribution polymer that includes units derived from an acrylic acid or a salt thereof and (i) a substance that has pharmacological activity against a pathogenic organism, or (ii) a substance that has pharmacological activity against a cancer, or (iii) one or more agents selected from antigens and immunogens.

The present invention is based on our observation that a narrow molecular weight distribution polymer that includes units derived from an acrylic acid or a salt thereof induced the release of the beta-chemokines MIP-1α and MtP-1β and of interferon-y from primary human tissue macrophages, i.e., antigen presenting cells without also inducing the release of toxic amounts of pro-inflammatory cytokines. The polymer was not toxic to human cells at high concentrations. The polymer tested was produced according to the methods described herein. Without being bound by the following theory, we consider that the above observations indicate that a narrow molecular weight distribution polymer that includes units derived from an acrylic acid or a salt thereof has the potential to act as a Th1 immune potentiating adjuvant by stimulating cell surface receptors that include Toll-like receptors, i.e., to act as a substance that enhances the immune stimulating properties of an antigen.

We have demonstrated in practice that a narrow molecular weight distribution polymer that includes units derived from an acrylic acid or a salt thereof does indeed act as a Th1 immune potentiating adjuvant. A complex comprising an antigen, namely tuberculin purified protein derivative BP, and a methacrylic acid sodium salt homo-polymer (PMAA-Na) produced as described herein caused more T lymphocyte proliferation than did the tuberculin antigen on its own, and also increased interferon-γ secretion from T lymphocytes more than did the tuberculin antigen alone.

A narrow molecular weight distribution polymer that includes units derived from an acrylic acid or a salt thereof may therefore be used as an immune potentiating adjuvant in conjunction with antigens and/or immunogens as used in conventional vaccines, for example, antigens and immunogens that are derived directly or indirectly from organisms against which therapeutic and/or protective vaccination is desired. Such antigens and immunogens are, for example, antigens and/or immunogens obtained from natural sources, i.e., derived directly from the relevant organism, and subunit antigens and immunogens, and antigens and immunogens produced by recombinant DNA technology and/or by chemical synthesis, which antigens and immunogens are derived indirectly from the relevant organisms. The polymer may be formulated in vaccine compositions with the appropriate antigen(s) and/or immunogen(s). Carriers and excipients may be present, as may delivery system adjuvants. The polymer and the antigen and/or immunogen may be used in the form of a complex of the invention, or may be co-administered, see below.

Examples of vaccines and of antigens and immunogens for use in such vaccines include but are not limited to vaccines directed against diphtheria, tetanus, typhoid, pertussis, measles, rubella, mumps, polio, H. influenza eg type b, meningitis, hepatitis A, hepatitis B, cholera, rabies, encephalitis (of various kinds), yellow fever, and influenza, and antigens and immunogens used in and suitable for use in such vaccines. Such antigens and immunogens may be used according to the present invention.

However, not only does a narrow molecular weight distribution polymer that includes units derived from an acrylic acid or a salt thereof have an immune potentiating effect on co-administered antigens and immunogens, it also has the ability, in the absence of co-administered antigens or immunogens, to promote protective Th1 immune responses against pathogenic organisms, for example, a pathogenic organism that is predominantly but not exclusively an intracellular organism, in particular against an organism that exists and persists predominantly in cells of macrophage origin and/or in other antigen presenting cells including dendritic cells. This may be achieved by administering the polymer and a substance that has pharmacological activity against a pathogenic organism (called herein a "drug"), for example, a pathogenic organism that is predominantly but not exclusively an intracellular organism, in particular a pathogenic organism that exists and persists in cells of macrophage origin and/or in other antigen presenting cells including dendritic cells, especially a substance that kills or otherwise disrupts such an organism. The killing or disruption of the organism results in the release of antigenic material. The presence of the polymer potentiates the immune response to the antigen because the dendritic cells that are recruited into this altered microenvironment take up and process the antigens released. The dendritic cells then initiate the CD4+ T cell activation that promotes the generation of effector cytotoxic T-lymphocyte responses. Some of these responses will be therapeutically directed against any persisting organisms in the remaining chronically infected cells and also organisms in the environment of such cells. Other effector cytotoxic T cell responses will be able to provide protective vaccine based responses against future re-infection. Pharmacological treatment, therapeutic vaccination and protective vaccination are therefore achieved.

The invention therefore relates to a narrow molecular weight distribution polymer that includes units derived from an acrylic acid or a salt thereof the polymers for use with a substance that has pharmacological activity against a pathogenic organism in the treatment of an infection by the pathogenic organism and/or for inducing an immune response to the pathogenic organism. The invention also relates to a method of treating an infection by a pathogenic organism and/or inducing an immune response to the pathogenic organism in a subject in need of such treatment, which comprises administering to the subject a narrow molecular weight distribution polymer that includes units derived from an acrylic acid or a salt thereof and a substance that has pharmacological activity against the pathogenic organism.

The polymer and the pharmacologically active substance are preferably in the form of a complex of the invention, or alternatively they may be co-administered, see below.

The pathogens against which an immune response is to be induced are primarily those that replicate and/or persist intracellularly, in particular those that replicate and/or persist in tissue based macrophages and other antigen presenting cells, for example, dendritic cells. Such organisms, and diseases and disorders caused by such organisms, include the following:
a) Organisms that cause superficial mycoses, including ringworm; tinea; thrush; Malassezia infections, including pityriasis versicolor, Malassezia folliculitis, seborrhoeic dermatitis and Scytalidium infections; Otomycosis; and Keratomycosis.
b) Candida species that cause invasive and chronic fungal infections, including Candida albicans, Candida tropicalis and Candida glabrata; Aspergillus species, including Aspergillus fumigatus, Aspergillus flavus and Aspergillus niger; Cryptococcus neoformans; Mucormycosis, for example, caused by species of Absidia, Rhizopus and Rhizomucor, Fusarium species; Trichosporon species; Blastomycosis; Sporothrix species; Sporotrichum species; Histoplasmosis, for example, caused by Histoplasma capsulatum var. capsulatum; African histoplasmosis, for example, caused by Histoplasma capsulatum var. duboisii; Blastomycosis, for example, caused by Blastomyces dermatitidis; Coccidioidomycosis, for example, caused by Coccidioides immitis; Paracoccidioidomycosis, for example, caused by Paracoccidiodes brasiliensis; and infections caused by Penicillium marneffei.
c) Organisms that cause mycobacterial diseases, for example, tuberculosis and leprosy caused by members of the mycobacterial family, for example, Mycobacterium tuberculosis, atypical mycobacteria, and mycobacterium leprae.
d) Members of the schistosoma family that cause Schistosomiasis, for example, Schistosoma haematobium, Schistosoma mansoni, Schistosoma japonicum, Schistosoma intercalatum, and Schistosoma mekongi.
e) Organisms that cause typhoid and paratyphoid fevers, for example, members of the salmonella family of serotypes A, B, C and D.
f) Organisms that cause toxoplasmosis, for example, Toxoplasma gondii.
g) Organisms that cause Human African Trypanosomiasis, for example, Trypanosoma brucei gambiense or Trypanosoma brucei gambiense.
h) Organisms that cause American Trypanosomiasis, for example, Trypanosoma cruzi.
i) Organisms that cause malaria, for example, Plasmodium falciparum, Plasmodium vivax, Plasmodium ovale and Plasmodium malariae.
j) Organisms that cause HIV and HTLV infections, for example HIV-1 and HIV-2 and HTLV and HTLV-II.
k) Organisms that cause Pneumocystis carinii infections.
l) Organisms that cause leishamaniasis, for example, the visceral form, e.g., kala azar or the cutaneous form, for example, L. donovani and L. mexicana.

Pharmacologically active substances (drugs) used to treat such disease and disorders are well known in the art, see for example, Principles and Practice of infectious Diseases. by Mandell G.L, Bennett J.E., & Dolin R. Fifth Edition.
Churchill Livingstone. (2000). Manson's Tropical Diseases. by Cook & Zumla. Twenty-first Edition. Saunders. (2003), and further drugs are being developed.

Examples of substances that have pharmacological activity against pathogenic organisms include but are not limited to clotrimazole, flucytosine, fluconazole, griseofulvin, itraconazole, ketoconazole, miconazole, pyrazinamide, ciprofloxacin, rifampicin, thiacetazone, cycloserine, clofazimine, dapsone, rothionamide, metriphonate, oxamniquine, praziquantel, co-trimoxazole, pyrimethamine, sulfadoxine, spiramycin, melarsoprol, nifurtimox, amodiaquine, chloroquine, mefloquine, primaquine, proguanil, quinine, zidovudine, efavirenz, indinavir, ribavirin, vidarabine, levamisole, and acyclovir.

For use according to this aspect of the present invention it is not necessary for a treatment to be fully successful, i.e., to cure the subject or to completely eliminate the organism as the immune response to the organism that is being generated will provide an effective "second line" of defence. Any residual organisms will be eliminated by the effector cytotoxic T-lymphocyte responses generated because they will be therapeutically directed against any persisting organisms in the remaining chronically infected cells. What is necessary is that some organisms are killed, thereby releasing antigens. One of the advantages of the present invention is that even if the treatment does not completely eliminate the organism, the induction of effector cytotoxic T-lymphocyte responses will provide protective vaccine based responses against future re-infection. Pharmacological treatment, therapeutic vaccination and protective vaccination are therefore achieved.

The same considerations apply to cancer. Use of a complex of the invention that comprises a narrow molecular weight distribution polymer that includes units derived from an acrylic acid or a salt thereof and a substance that has pharmacological activity against cancer, especially a cytotoxic agent that kills or partially kills otherwise disrupts the transformed, i.e., cancerous cells. The killing or disruption of the cells results in the release of antigenic material some of which will be "seen" by macrophages and antigen presenting cells as non-self antigens. The presence of the polymer potentiates the immune response to the antigen(s) because the dendritic cells that are recruited into this altered microenvironment take up and process the antigen(s) released. The dendritic cells then initiate the CD4+ T cell activation that promotes the generation of effector cytotoxic T-lymphocyte responses. Some of these responses will be therapeutically directed against other cancer cells and will therefore provide protective vaccine based responses against the recurrence of the cancer and the growth of any remaining micrometastasis. These responses will be greatest for those cancers in which large numbers of macrophages and antigen presenting cells are present. This is especially so in lymphomas and leukaemias. Pharmacological treatment, therapeutic vaccination and protective vaccination are therefore achieved.

Substances that have pharmacological activity against cancer are well known in the art and new active agents are being developed, see for example, Cancer Principles and practice of oncology. V.T. DeVita, S Hellman & S.A. Rosenburg, Published by Lippincott Williams & Wilkins, 6tth Edition, 2001.. Examples of anticancer agents include but are not limited to aclarubicin, actinomycin D, amsacrine, azacytidine, bleomycin, carmustine, chlorambucil, cisplatin, dacarbazine, daunorubicin, hydroxyurea, melphalan, mercaptopurine, methotrexate, mitomycin C, mitozantrone, tresulfan, vinblastine, vincristine and crisantaspase.

As indicated above, it is generally preferable that the polymer, the substance that has pharmacological activity against a pathogenic organism, or a substance that has pharmacological activity against a cancer (both called "drug") are administered in the form of a complex of the invention. Such a complex is taken up by the antigen presenting cells more effectively than the drug alone, and it also ensures that the drug and the polymer are both present at the same time in the same cell so that when the drug kills the organism and the antigens are released, the polymer is ready *in situ* to generate a micro-environment that potentiates Th1 immune responses. However, the polymer and the drug may be co-administered, either in the same pharmaceutical preparation, or in separate preparations. In the latter case, one preparation may be administered before the other, but it is generally preferable to administer the two preparations substantially simultaneously.

It is also generally preferable to administer the polymer and antigens and/or immunogens in the form of a complex of the invention. However, the polymer and the antigens and/or immunogens may be co-administered, either in the same pharmaceutical preparation, or in separate preparations. In the latter case one preparation may be administered before the other, but it is generally preferable to administer the two preparations substantially simultaneously.

A complex of the invention comprises a narrow molecular weight distribution polymer that includes units derived from an acrylic acid or a salt thereof and
(i) a substance that has pharmacological activity against a pathogenic, as defined in claim 1, organism, or
(ii) a substance that has pharmacological activity against a cancer, or
(iii) one or more agents selected from antigens and immunogens.

Pathogenic organisms, substances that have pharmacological activity against a pathogenic organism, substances that have pharmacological activity against a cancer, and antigens and immunogens are, for example, as described above.

A complex of the invention, or a narrow molecular weight distribution polymer that includes units derived from an acrylic acid or a salt thereof may be in the form of a pharmaceutical preparation comprising the complex and a pharmaceutically suitable carrier. The preparation may be or be regarded as a conventional pharmaceutical preparation or as a vaccine, or both, depending on the components, in particular the nature of the substances (I), (ii) and (iii). If intended for use In inducing an immune response, a delivery system adjuvant, for example, alum, may also be present.

If a narrow molecular weight distribution polymer that includes units derived from an acrylic acid or a salt thereof is to be co-administered with a substance (i), (ii) or (iiii) as defined above instead of being administered In the form of a complex with the substance, the polymer and the substance (i), (ii) or (iii) may be formulated in the same pharmaceutical preparation or they may be formulated in separate preparations, in each case in admixture with a pharmaceutically suitable carrier. If in separate preparations, one may be administered before the other, but it may be preferable to administer the two preparations substantially simultaneously.

As stated above, the present invention encompasses both conventional pharmaceutical treatment of diseases and disorders caused by pathogenic organisms and of cancer, and the induction of immune responses to such diseases, disorders and cancer. It will be understood that an immune response induced in a subject in heed of such is preferably a therapeutic and/or prophylactic immune response. A therapeutic immune response assists in the treatment of the disease or disorder. Such a response may be a short term response. A prophylactic immune response provides longer-term protection, for example, against recurrence of the disease or disorder, or subsequent infection or re-infection, or the growth and spread of the micrometastases of cancer. Such responses are often called therapeutic and prophylactic "vaccination".

The use of a narrow molecular weight distribution polymer that includes units derived from an acrylic acid or a salt thereof and a substance that has pharmacological activity against a pathogenic organism in treating a disease caused by the pathogenic organism and, at the same time, inducing an immune response has been illustrated in the case of leishmaniasis. Infection due to Leishmaniasis can be either of the visceral form, e.g., kala azar or of the cutaneous form. Visceral leishmaniasis is a disseminated protozoal infection. For many years, conventional therapy has consisted of pentavalent antimony given once daily by intravenous or intramuscular injection for 28 days. However, since 1990, the failure of large scale antimony treatment in India has led to the introduction of amphotericin B deoxycholate (AmB) as an effective anti-leishmanial agent. Long-term cure rates of 97% are obtained by the intravenous administration of amphotericin B either daily or, more typically, every other day for a total of 15 = 20 infusions at a dose of 0.75 -1 mg/kg/day. These prolonged courses of treatment are associated with a high hospital burden and substantial costs. Consequently, this often leads to non-compliance or to the abandonment of the treatment regimen because of the associated adverse events that often occur.

Lipid based complexes of amphotericin B accumulate in tissue based macrophages: They have been shown to be very effective against a large number of yeasts and fungi that can grow in human macrophages. It has been shown that lipid formulations of amphotericin B have high level efficacy against visceral leishmaniasis and that they result in a greater than 90% cure rate when given for 5 -10 days. Sundar et al. have shown that a short course, i.e., 5 day of treatment with liposomal amphotericin B (AmBisome; Gilead Sciences) administered daily in infusions of 1.5 mg/kg/day cured 93% of patients. In another follow-up trial, a single total dose infusion of 5 mg/kg of liposomal amphotericin B cured 91% of patients. There were very few adverse events. (Sundar, S et al. Treatment of Indian visceral leishmaniasis with single or daily infusion of low dose liposomal amphotericin B: a randomised trial. Brit. Med. J. 2001; 323: 419-422. Sundar, S. et al. Single-dose liposomal amphotericin B in the treatment of visceral leishmaniasis in India: a multicenter study. Clin. Inf. Dis. 2003: 37; 800-804). Therefore, single dose liposomal amphotericin B treatment can be considered safe and effective for the treatment of visceral leishmaniasis in India. However, even at a single dose the treatment is costly, and a practical disadvantage is that the lipid-based amphotericin B formulations are not stable during long-term storage, particularly at the high temperatures of the tropics where many of the cases of visceral leishmaniasis occur.

The immune response that promotes healing and parasite clearance in leishmaniasis is dominated by an interferon gamma mediated Th-1 response. Although macrophages ingest leishmania efficiently, they are not activated by the ingestion of the organism; consequently pro-inflammatory chemokines, pro-inflammatory cytokines and interferon-γ are not released. In contrast to macrophages, dendritic cells take up leishmania parasites, mature and then promote the development of cellular immune responses. In animal models, it has been shown that if infected macrophages can be activated, killing of the parasite ensues. Therefore, two different processes, namely, antigen processing and cellular maturation/activation must be combined for an effective cellular vaccine response. As demonstrated by microbes, substances that possess both an antigenic component and a dendritic cell activation/maturation component can propel dendritic cells towards a Th1 mediated response. Lipid-based amphotericin B formulations have no immuno-modulatory or adjuvant activity.

A complex of amphotericin B and poly(methacrylic acid, sodium salt) (PMAA-Na) according to the invention was prepared and tested, as described in detail in the Examples. The complex was shown to have the following unique properties:
a) It enables the effective organ delivery of amphotericin B to liver, spleen and lymph nodes after intravenous administration. These are the organs primarily infected by Leishmaniasis in animals and in man.
b) It enables the effective intracellular delivery of amphotericin B to Leishmania infected macrophages when given intravenously.
c) The amphotericin B-poly(methacrylic acid, sodium salt) complex accumulates in the intracellular organelles within which the Leishmania amastigotes survive, multiply and persist, and this enhances the intracellular killing of the Leishmania amastigotes.
d) *In vivo* studies in an animal model of visceral leishmaniasis showed that the anti-leishmanial activities of clinical grade amphotericin B, the commercial liposomal amphotericin B preparation AmBisome, and the amphotericin B-poly(methacrylic acid, sodium salt) preparation were not significantly different from each other. The amphotericin B-poly(methacrylic acid, sodium salt) preparation was as effective as the commercial liposomal amphotericin B preparation AmBisome in the animal model of visceral leishmaniasis.
e) The poly(methacrylic acid, sodium salt) is released from the amphotericin B within tissue macrophages and it then promotes the release of β-chemokines and of interferon-γ. This generates a local Th1 adjuvant response.
f) The local induction of chemokine and cytokine expression facilitates the recruitment and activation of cells of the innate immune system to the site. This includes immature dendritic cells and CD4+ T lymphocytes. They stimulate macrophages to produce more TNF-α, IL-1β and IL-6.
g) Activation of the innate immune system induces dendritic cells to mature and migrate from tissue to regional lymph nodes where their newly released antigens are presented. This results in the induction and generation of CD4+ T cell responses and effector CD8+ T cell responses.
h) Therefore, the killing of Leishmania within antigen presenting cells and the subsequent availability of Leishmania antigens in the immediate vicinity of a Th1 promoting adjuvant converts the infected macrophage into a cellular vaccine.
i) The cellular immune response is significantly enhanced because of the close proximity of the antigen presenting cells, the release of Leishmania antigens by the killing activity of amphotericin B, and the simultaneous promotion of an appropriate and local Th1 cytokine/chemokine environment.
j) Concurrent cure of the disease and generation of a therapeutic effector cellular immune response i.e. vaccination generates long-term protective immunity against the organism with which the individual was infected.
k) Cure of the disease and therapeutic vaccination are therefore achieved simultaneously and without the need to administer an external adjuvant or an external source of Leishmania antigens.
l) Single dose treatment is effective, unlike amphotericin B, which typically requires 15-20 doses.
m) The preparation is more stable, especially at the higher ambient temperatures found in the tropics than the commercially available liposomal amphotericin B preparations.
n) The toxicity of free amphotericin that is often seen when it is administered intravenously in man is reduced to a minimum when the preparation is used.
o) By forming a complex between amphotericin B and the PMAA-Na, chemical derivatisation of the drug is avoided. Consequently, the efficacy of amphotericin B against Leishmania sp. is not changed or reduced. This is particularly relevant to the amino group in the sugar moiety of amphotericin B because this group is important in mediating the pharmacological activity of this drug. The amine on the sugar moiety has previously been used as a conjugation point because of its reactivity (Conover C. D. et al. Utility of poly(ethylene glycol) conjugation to create prodrugs of amphotericin B. Bioconjugate Chem. 2003: 14: 661-666).

As stated above, the present invention provides a pharmaceutical preparation that comprises a complex of the present invention or a narrow molecular weight distribution polymer that includes units derived from an acrylic acid or a salt thereof, as defined in claim 1 in admixture or conjunction with a pharmaceutically suitable carrier.

A pharmaceutical preparation of the invention may be in a form suitable for administration intravenously, intra-arterially, into the lymphatic circulation, into a. lymph node, orally, intraperitoneally, topically, buccally, rectally, to the surface of the skin, transdermally, subcutaneously, intramuscularly, into the joint space, intranasally, intravitreally, or pulmonarily, directly to an organ, around an organ, by injection into an organ, or by direct infusion through an organ. The present invention includes administration of a complex of polymer in accordance with the invention by any of such routes. A pharmaceutical preparation of the invention may be in the form of a depot or reservoir preparation, or an aerosol preparation. Suitable formulations for that above and other routes of administration are known in the art, see for example, Remington's Pharmaceutical Sciences by E. W. Martin. See also Wang, Y. J. and Hanson, M. A., Journal of Parenteral Science and Technology, Technical Report No. 10, Supp. 42:2S, 1988.

A complex of the invention or a narrow molecular weight distribution polymer that includes units derived from an acrylic acid or a salt thereof may be "particulate associated". Such particulates may be produced by emulsion, homogenisation and spray drying processes, which are known in the art. For a review of such formulations see for example, Hanes J, Cleland JL & Langer R, Adanced Drug Delivery Reviews 28 (1997) 97-119. Pharmaceutical compositions comprising such particulate associated complexes of the invention may be administered mucosally by a pulmonary or nasal route, by ingestion, or by a non-rnucosal parenteral route, especially subcutaneously or intramuscularly.

In a pharmaceutical preparation of the invention in liquid form, the concentration of the polymer may be, for example, from 0.1 to 2,500 µg/ml, for example, from 1 to 500 µg/ml. Other formulations may comprise the polymer in analogous amounts, for example, calculated on the basis of the liquid preparations.

The present invention is concerned with a narrow molecular weight distribution polymer that includes units derived from an acrylic acid or a salt thereof and with complexes comprising such a polymer and an antigen or immunogen, or a substance that has pharmacological activity against a pathogenic organism or a cancer. The polymer may be a homopolymer or copolymer including units derived from an acrylic acid, for example, derived from acrylic acid or methacrylic acid, or a salt thereof. Some polymers including units derived from an acrylic acid are known. As indicated above, the polydispersibility is 1.7 or less, for example 1.6 or less, for example 1.5 or less, for example 1.4 or less, for example, 1.2 or less, for example less than 1.7, for example less than 1.6, for example less than 1.5, for example less than 1.4, for example, less than 1.2. In general, the lower the polydispersibility, the better. Accordingly, a polydispersibility of less than 1.2 is generally preferred. Accordingly, a polydispersibility of less than 1.2 is generally preferred.

The polymer generally has a molecular weight such that the polymer when present in the blood remains substantially in the circulating blood during and after passage through the kidney, with little or none of the polymer undergoing filtration through the glomerular apparatus. Renal filtration (also known as glomerular filtration) of large molecules is a function *inter alia* of the size and shape of the molecule, which is dependent in part on the molecular weight. In general, for any particular polymer, there is a threshold molecular weight or a narrow range of molecular weights below which renal filtration occurs and above which little or no renal filtration occurs. The threshold molecular weight for any particular polymer may be determined by standard tests, for example, using radiolabelled polymer.

The polymer has a molecular weight of 100.000.or less, for example, less than 100,00, for example, 80,000 or less, for example, 75,000 or less, for example,65,000 or less, for example, 55,000 or less, for example, 45,000 or less, for example. The polymer generally has a molecular weight of 4,000 or more, for example, 5,000 or more, for example, 10,000 or more, for example, 20,000 or more, for example, 30,000 or more, for example, 40,000 or more. A polymer may have a molecular weight within a range that combines any of the upper molecular weight values given above with any of the lower molecular weight values given above. Examples of such ranges include but are not limited to ranges of from 80,000 to 4,000, 75,000 to 5,000, 65,000 to 10,000, 55,000 to 10,000, and 45,000 to 10,000. Further examples include the ranges from 50,000 to 4,000, for example, from 40,000 to 25,000. Molecular weights in the range of from 45,000 to 10,000 may generally be preferred.

It is generally desired that the polymer or the complex should remain in the circulating blood for an appropriate period of time. As is known to the person skilled in the art, the period of time that is appropriate will depend on a number of factors including, in the case of a complex, the nature of the substance complexed to the polymer. As an example, the polymer or complex may remain in the circulation for several hours, for example, for up to 24 hours, for example, from about 4 to 6 hours up to about 24 hours.

The polymer including units derived from an acrylic acid or a salt thereof comprises a unit (I) 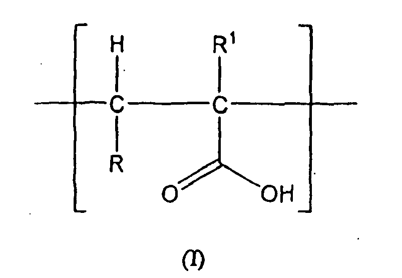 wherein R is hydrogen, methyl or carboxylic acid, and R¹ is selected from the group consisting of hydrogen and C₁-C₆alkyl groups; and salts thereof, for example, alkali metal salts, for example, sodium salts, or ammonium salts thereof.

Preferably, R is hydrogen or methyl. Preferably R¹ is, independently of R, hydrogen or methyl. In particular R is hydrogen and R¹ is methyl.

Examples of block copolymers that include units derived from an acrylic acid or a salt thereof include block copolymers comprising the unit (II) 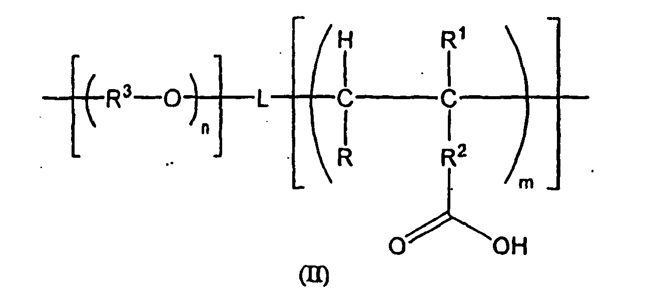 in which R and R¹ are defined as above and R² is a bond; R³ is selected from the group consisting of C₁-C₁₈alkylene, C₂-C₁₈alkenylene, C₇-C₁₈aralkylene, C₇-C₁₈alkarylene, and C₆-C₁₈arylene; L is a divalent linker joining the blocks; and m and n is each an integer 1 or greater than 1.

Most preferably R is selected from hydrogen and methyl.

Preferably R¹ is selected from the group consisting of hydrogen, methyl, ethyl, propyl, butyl, pentyl or isomers thereof. Most preferably R¹ is selected from hydrogen and methyl.

Preferably the groups R³, which may be the same or different, are selected from the group consisting of C₁-C₈ alkylene groups, preferably 1,2-alkylene, and C₆-C₁₂ arylene groups, most preferably methylene, ethylene, 1,2-propylene and 1,3-propylene. Preferably all groups R³ are the same, most preferably all are 1,2-ethylen6 or 1,2-propylene.

L preferably comprises a C₁-C₁₈ alkylene or C₆-C₁₈ arylene group which may be substituted and/or interrupted with 1 or more heteroatoms. Preferably L comprises a group selected from the group consisting of C₁-C₆ alkylene, C₆-C₁₂ arylene, C₁-C₁₂ oxyalkylene and C₁-C₆ acyl. Where L comprises an alkylene group, it can be branched, linear or cyclical, substituted or unsubstituted with one or more alkyl groups, and is preferably methylene, 1,2-ethylene, 1,2-propylene, 1,3-propylene, ^{tert}butylene, ^{sec}butylene, hexylene or octylene. Where L comprises an arylene group, it is preferably benzylene, tolylene or xylylene. Most preferably L comprises a - COR^{a} group, wherein R^{a} is selected from the group consisting of C₁-C₆ alkylene or C₆-C₁₂ arylene, preferably methylene, 1,2-ethylene, 1,2-propylene, 1,3-propylene, ^{tert}butylene and ^{sec}butylene.

A polymer may be a homopolymer incorporating unit (I) or may be a copolymer or block copolymer incorporating other polymeric, oligomeric or monomeric units, for example, a block copolymer comprising units (II) as described above. For example, further polymeric units incorporated in a homopolymer or copolymer or block copolymer may comprise acrylic polymers, alkylene polymers, urethane polymers, amide polymers, polypeptides, polysaccharides and ester polymers. Preferably, where the polymer is a heteropolymer, additional polymeric components comprise polyethylene glycol, polyaconitic acid or polyesters.

For example, a polymer having units (I) or (II) may also comprise further units that enable, for example, solubilisation of the precursor polymer and/or that enable control of the number of free acrylate acid groups (and hence salt-forming groups) in the polymer to be used according to the invention. For example, a polymer comprising units (I) may have the structure (III) or (IV) 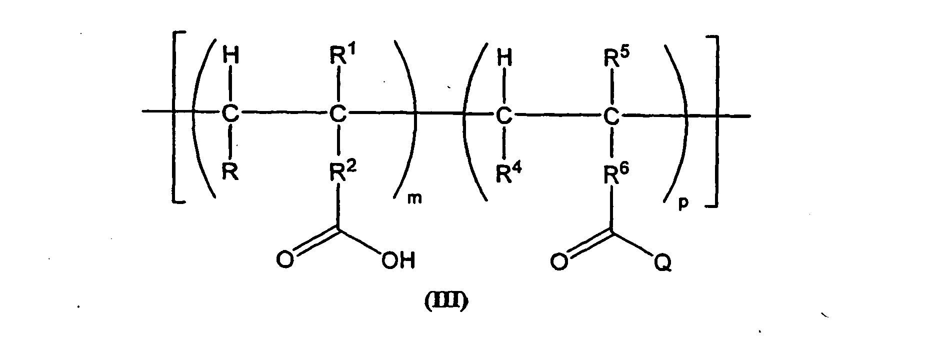 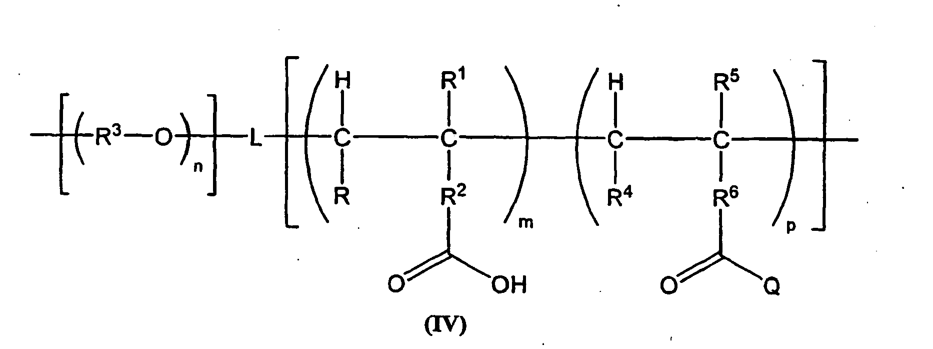 in which R, R¹, R² and R³, L, m and n are defined as above, R⁴, R⁵and R⁶ are selected, independently, from the same groups as R, R¹and R², respectively; Q denotes a group that is not cleaved or is not substantially cleaved under the conditions used to produce the polymer; and p denotes an integer 1 or greater than 1. If desired, Q may be a targeting group, i.e. a group that targets the polymer to a cell type, eg macrophages, or to an organ eg the liver.

Q may be, for example, selected from the group consisting of C₁-C₁₂ alkyl, C₂-C₁₂ alkenyl, C₇-C₁₂ aralkyl, C₇-C₁₂alkaryl, C₁-C₁₂alkoxy, C₁-C₁₂hydroxyalkyl, C₁-C₁₂alkylamino, C₁-C₁₂alkanoyl, C₁-C₁₂aminoalkyl or any one of C₁-C₁₂ alkyl, C₂-C₁₂ alkenyl, C₇-C₁₂ aralkyl, C₇-C₁₂alkaryl, C₁-C₁₂alkocxy, C₁-C₁₂hydroxyalkyl, C₁-C₁₂alkylamino, C₁-C₁₂alkanoyl substituted with an amine, hydroxyl or thiol group. Preferably Q comprises an amine group, for example, a C₁-C₁₂hydroxyalkylamino group, for example, a 2-hydroxypropylamino or a hydroxyethylamino moiety.

The group Q may be a solubilising group for the polymer in aqueous solutions. For example, the polymer may be a water soluble polyacrylamide homo-or copolymer, preferably a polymethacrylamide or polyethacyrlaide homo- or copolymer. Furthermore, when the groups Q are not cleaved (or the majority of groups Q are not cleaved) under conditions under which the polymer is produced, the presence of the groups Q enables control of the number of free acrylate acid groups (and hence salt-forming groups) in the polymer. For example, the proportion of the Q-containing units relative to the number of leaving group X-containing units in the precursor polymer may be selected. The relative proportion of the Q-containing units relative to the number of leaving group X-containing units will determine the number of acrylate acid or salt groups in the polymer product. As set out below, the polyanionic nature of the polymer may influence its biological activity. The ability to manipulate the ionic nature of the polymer is therefore useful in producing a polymer having desired properties.

A polymer for use according to the present invention, for example, for forming a complex of the invention, may be, for example, a polymethacrylic acid having a polydispersibility of 1.7 or less, for example 1.6 or less, for example 1.5 or less, for example 1.4 or less, for example 1.2 or less, for example less than 1.7, for example less than 1.6, for example less than 1.5, for example less than 1.4, for example, less than 1.2. In general, the lower the polydispersibility, the better. Accordingly, a polydispersibility of less than 1.2 is generally preferred. The molecular weight of the polymer is generally as described in the Definitions section above. Examples of such polymethacrylic acids and salts thereof and of their production are given in the Examples herein. Such polymethacrylic acids and salts thereof may be particularly useful in the practice of the present invention.

We have found that the release of β-chemokines and interferon-γ from primary tissue macrophages, i.e., antigen-presenting cells induced by the polymethacrylic acid sodium alt (PMAA-Na) complexes produced as described herein was not seen with commercially available samples of methacrylic acid sodium salt homo-polymers having a similar narrow molecular weight distribution. The observed differences in the release of chemokines and cytokines from tissue macrophages suggest that there may be structural differences between PMAA-Na prepared as described herein and commercially available PMAA-Na. Since the *M*ₙ of the PMAA-Na prepared as described herein was comparable to one of the commercial samples (i.e., *M*ₙ ~ 22 kg/mol), an immunological effect that is due to the influence of molecular weight seems unlikely. PMAA-Na is a polyanion. It has been suggested that the biological effects of polyanions could be dependent upon structural features such as molecular weight, charge density and tacticity (Ottenbrite, R., et al., Biological activity of poly(carboxylic acid) polymers., in Polymeric Drugs, L. Donaruma and O. Vogl, Editors. 1978, Academic Press: New York. p. 263-304.). While not being bound by the hypotheses above and below relating to the biological activity of the polymers used according to the present invention, we consider that the biological data indicates that it may be the preparation method that gave the structural detail that resulted in the PMAA-Na having the unique biological properties described.

The polymer used in the Examples herein is polymethacrylic acid, sodium salt, which was produced by hydrolysis of poly(N-methacryloxysuccinimide) (PMOSu) using sodium hydroxide. Such a method, and similar methods, in particular involving hydrolysis of the precursor polymer, may be useful for the production of that and other polymers for use according to the present, invention.

For example, a polymer for use according to the invention may be produced by a method involving hydrolysis of a precursor polymer using a basic agent, for example, an alkali metal or alkaline earth metal base, for example, a sodium, potassium, caesium, calcium, magnesium, or lithium base. Such a base may be, for example, a hydroxide, carbonate or hydrogen carbonate, for example, sodium hydroxide. The precursor polymer may be PMOSu, or it may be another suitable precursor polymer, for example, a precursor polymer as described below The hydrolysis is carried out in a suitable solvent. As the precursor polymers are generally hydrophobic molecules, the reaction is generally carried out in an organic solvent, for example, DMF, DMSO, DMPU or dimethylaclamide. The hydrolysis may be carried out under mild conditions, for example, ambient temperature and pressure and gentle heating, for example, 40 to 60°C, for example for 2 to 16 hours, or stronger conditions may be used.

Polymers produced by such a method may have biological properties analogous to some or all of the biological properties demonstrated by the PMAA-Na produced as described herein.

The POMSu used as the precursor polymer in the production of PMAA-Na as described herein was produced as described in WO 01/18080 and in Example A4 herein by homogeneous polymerisation of methacryloxysuccinimide using an atom transfer radical polymerisation method, in particular a copper mediated method. Such a method for the production may be useful for the production of that and other precursor polymers for use according to the present invention, but the invention is not limited to such methods, nor to precursor polymers produced by such methods nor to polymers produced from such precursor polymers.

A polymer including units derived from an acrylic acid or a salt thereof for use according to the invention may be produced by hydrolysis of a corresponding precursor polymer that has, in place of the hydrogen atom of the acrylate carboxylic acid in the units derived from an acrylic acid, a group that can be cleaved by hydrolysis to give the acid, for example, cleaved by mild hydrolysis.
A group that can be cleaved by hydrolysis is, for example, an appropriate leaving group, for example, an electron withdrawing group, for example, an acylating group, which is preferably a carboxylate activating, generally selected from the group consisting of N-succinimidyl, pentachlorophenyl, pentafluorophenyl, para-nitrophenyl, dinitorphenyl, N-phthalimido, N-bornyl, cyanomethyl, pyridyl, trichlorotriazine, 5-chloroquinolino, and imidazolyl goups, preferably an N'-succinimidyl or imidazolyl group, and especially an N-succinimidyl group. Hydrolysis may be carried out using a basic agent, for example, as described above, for example, using sodium hydroxide, and may be carried out under mild conditions, for example, ambient temperature and pressure and gentle heating, for example, 40 to 60°C, for example for 2 to 16 hours.

A polymer comprising units (I) or (II) as described above may be produced from a polymer (a "precursor polymer") described in WO 01/18080 or from a similar polymer, or from a polymer described in WO 03/059973 or a similar polymer. Such precursor polymers include polymers comprising the unit (1a) and polymers comprising the unit (IIa) 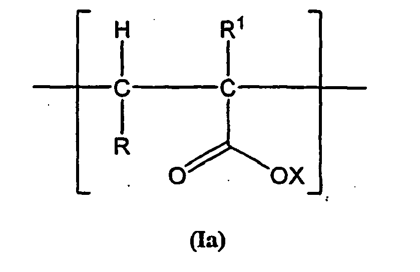 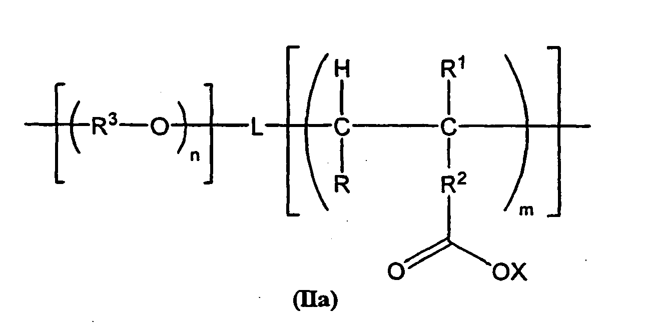 in which R, R¹, R², R³, L, m and n are as defined above, and X denotes a leaving group, for example, an acylating group, which is preferably a carboxylate activating, generally selected from the group consisting of N-succinimidyl, pentachlorophenyl, pentafluorophenyl, para-nitrophenyl, dinitorphenyl, N'-phthalimido, N-bornyl, cyanomethyl, pyridyl, trichlorotriazine, 5-chloroquinolino, and imidazolyl goups, X preferably denotes an N-succinimidyl or imidazolyl group.

A polymer comprising a unit that comprises the group Q ie a polymer comprising the unit (III) or a polymer comprising the unit (IV) may be produced from a corresponding precursor polymer (IIIa ) or (IVa) 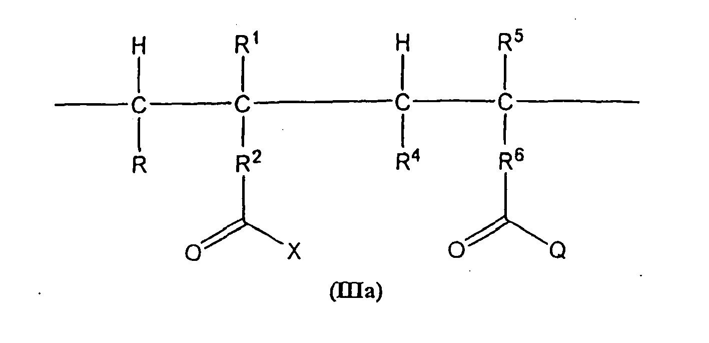 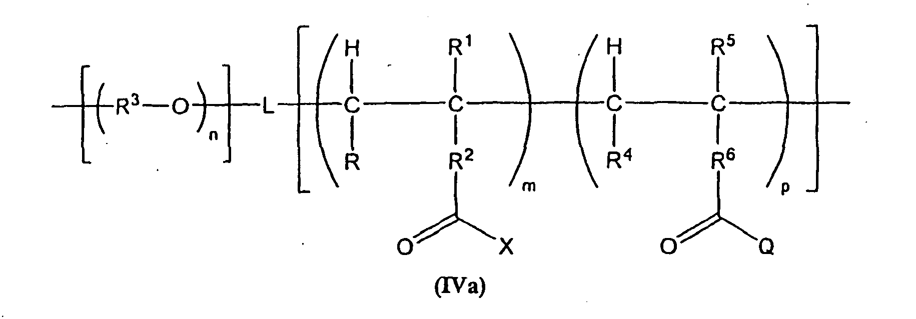 in which R, R¹, R² and R³, R⁴; R⁵ and R⁶, L, Q, X, m, n and p are defined as above. m, n and p may each represent an integer of up to 500, and preferably the total of m, n and p is not greater than about 500.

A polymer precursor having the desired polydispersity may be produced by atom transfer radical polymerisation method, for example, a copper mediated atom transfer radical polymerisation, or other methods including free radical polymerisation, may be used. Such methods are described in WO 01/18080 and in WO 03/059973. Examples are given below.

Where the polymerization is carried out by atom transfer radical polymerization, a suitable radical initiator is utilised. Such initiators commonly comprise alkylhalides, preferably alkylbromides. In particular, the initiator is 2-bromo-2-methyl-(2-hydroxyethyl)propanoate. The polymerization is also carried out in the presence of a polymerization mediator comprising a Cu(I) complex. Such complexes are usually Cu(I)Br complexes, complexed by a chelating ligand. Typical mediators are Cu(I)Br (Bipy)₂. Cu(I)Br(Bipy), Cu(I)Br (Pentamethyl diethylene), Cu(I)Br[methyl]₆ tris(2-aminoethyl)amine] and Cu(I)Br(N,N'-,N",N"'-pentamethyldiethylenetriamine).

The reaction should take place in the presence of a suitable solvent. Such solvents are generally aprotic solvents, for example, tetrahydrofuran, acetonitrile, dimethylformamide, dimethylsulphoxide and sulpholane and mixtures thereof. Alternatively, water may be used. Particularly preferred solvents are dimethylsulphoxide and dimethylformamide and mixtures thereof.

As regards the biological properties of a complex of the invention, we have found that different methods of producing a complex of the invention may influence the properties of the complex. It appears to be advantageous to form a complex of the invention by hydrolysing the polymer precursor, especially under mild conditions, to form the polymer in the presence of the antigen or immunogen or the substance that has pharmacological activity against a pathogenic organism or a cancer rather than to form a complex between a preformed polymer and the antigen or immunogen or the substance that has pharmacological activity against a pathogenic organism or a cancer.

The hydrolysis of the polymer precursor in the presence of the antigen or immunogen or the substance that has pharmacological activity against a pathogenic organism or a cancer should generally be carried out under conditions such that the antigen, immunogen or substance is not substantially affected adversely, for example, the hydrolysis should generally be carried out under mild conditions. For example, the conditions should be such that the antigen, immunogen or substance is not substantially decomposed, degraded or otherwise caused to lose relevant activity.

For example, the hydrolysis of the polymer precursor in the presence of the antigen or immunogen or the substance that has pharmacological activity against a pathogenic organism or a cancer may be carried out using a basic agent, for example, an alkali metal or alkaline earth metal base, for example, a sodium, potassium, caesium, calcium, magnesium, or lithium base. Such a base may be, for example, a hydroxide, carbonate or hydrogen carbonate, for example, sodium hydroxide. The hydrolysis is carried out in a suitable solvent, for example, an organic solvent, for example, DMF, DMSO, DMPU or dimethylactamide. The hydrolysis may be carried out under mild conditions, for example, under ambient temperature and pressure and gentle heating, for example, 40 to 60°C. The reaction may be carried out, for example for 2 to 16 hours.

A method of producing a complex of the invention by producing a polymer for use according to the invention, for example, by hydrolysing a polymer precursor, in the presence of the antigen or immunogen or the substance that has pharmacological activity against a pathogenic organism or a cancer, for example, as described above, is part of the present invention, as is a complex obtainable by such a method.

It is considered that, in a complex of the invention, the association between the polymer and the substance that has pharmacological activity against a pathogenic organism or the antigen or the cancer is predominantly non-covalent (i.e., by ionic or Van der Waal's bonding), but that some molecules of the pharmacologically active substance or the antigen may be covalently linked to the polymer. The extent of covalent bonding may be dependent on the nature of the pharmacologically active substance or antigen and on the nature of the polymer.

As set out above the term "complex" is used herein to denote an association between the polymer and the substance that has pharmacological activity against a pathogenic organism or the antigen or the cancer is predominantly non-covalent, but that may include some covalent linkages.

The complexes of the invention are polyelectrolytes having acidic groups. The extent of deprotonation may vary according to the environment of the complex. A complex of the invention may be in the form of a salt. A salt may be, for example, with a monovalent, bivalent, trivalent or quadrivalent counter ion. A monovalent counter ion may be, for example, an alkali metal ion, for example, a sodium or potassium ion, or an ammonium ion. A bivalent counter ion may be, for example, an alkaline earth metal ion, for example, a calcium or magnesium ion. Other counter ions include ions of transition metals, for example, iron, tin etc. More than one type of counter ion may be present and associated with the polymer. Furthermore, the number and proportion of salt-forming groups may be controlled, for example, by the use of appropriate polymer precursors, for example, as described above. It will be appreciated that, as the polymer has acidic groups and hence a negative charge, the substance or agent that is to form a complex with the polymer should generally be capable of sustaining a positive charge to enable direct formation of the complex by means of non-covalent interactions including electrostatic forces due to opposing charges. For example, the substance or agent used for complex formation may having basic, cationic or zwitterionic properties or may be adapted to have such properties. Alternatively, it is possible to vary the composition of the polymer, in particular by means of appropriate selection of the group Q so that a desired substance or agent can form a complex, for example, via association with the group Q.

In one embodiment of the invention, the polymer used in a complex of the invention or in conjunction with an antigen or immunogen, or a substance that has pharmacological activity against a pathogenic organism or a cancer is a polymethacrylic acid (PMAA) or a salt thereof, for example, a sodium salt, having a polydispersity of less than 1.4, preferably less than 1.2. The molecular weight of the polymer is generally as described in the Definitions section above. It may be advantageous to produce the polymer as described or substantially as described in the Examples herein, for example, to hydrolyse a precursor polymer, for example, a precursor polymer having an N-succinimide leaving group, using sodium hydroxide. It may be advantageous to form a complex with an antigen or immunogen, or with a substance that has pharmacological activity against a pathogenic organism or a cancer *in situ* during the production of the polymer.

The present invention relates in particular to a complex of the invention that comprises a complex of the invention that comprises a substance that has pharmacological activity against a pathogenic organism, for example, against leishmaniasis, for example, amphoteracin B, to a pharmaceutical preparation comprising such a complex, for example, a pharmaceutical preparation as described above, and to the various uses of such a complex in treating a disease or disorder caused by the pathogen and/or inducing an immune response against the pathogen as described above. The polymer component of the complex is a narrow molecular weight distribution polymer that includes units derived from an acrylic acid or a salt thereof, as defined in claim 1, for example a polymethacrylic acid polymer or salt thereof as described above.

The present invention particularly provides a complex that comprises amphotericin B and a polymethacrylic acid polymer or salt thereof as described above, especially a PMAA polymer or salt thereof produced as described in the Examples herein, and provides a pharmaceutical preparation comprising such a complex, for example, a pharmaceutical preparation as described above. The invention also provides the various uses of such a complex in treating leishmaniasis and/or inducing an immune response against a pathogen that causes leishmaniasis, including achieving therapeutic and/or prophylactic vaccination against leishmanisis, as described above.

In an alternative method for treating leishmaniasis and/or inducing an immune response against a pathogen that causes leishmaniasis, including achieving therapeutic and/or prophylactic vaccination against leishmanisis, a polymer of the invention and a substance that has pharmacological activity against a pathogenic organism, for example, against leishmaniasis, for example, amphotericin B, may be co-administered in separate preparations, as described in general terms above. One preparation may be administered before the other, but it is generally preferable to administer the two preparations substantially simulataneously.

The following non-limiting Examples illustrate the invention.

### Examples:

### Example A1: Chemical synthesis: Preparation of methacrylic acid sodium salt homo-polymer:

Poly(methacrylic acid, sodium salt) (PMAA-Na) **2** was prepared by the hydrolysis of poly(N-methacryloxysuccinimide) (PMOSu) **1** with sodium hydroxide (Scheme 1).

The PMOSu 1 had previously been synthesised by the polymerisation of methacryloxysuccinimide **3** (Scheme 2) using an Atom Transfer Radical Polymerisation (ATRP) procedure (Brocchini S. J., and Godwin A., "Uniform molecular weight precursors°, International Patent Publication Numbers WO 01/18080A1 & EP 99307152.1 (March 15, 2001)).

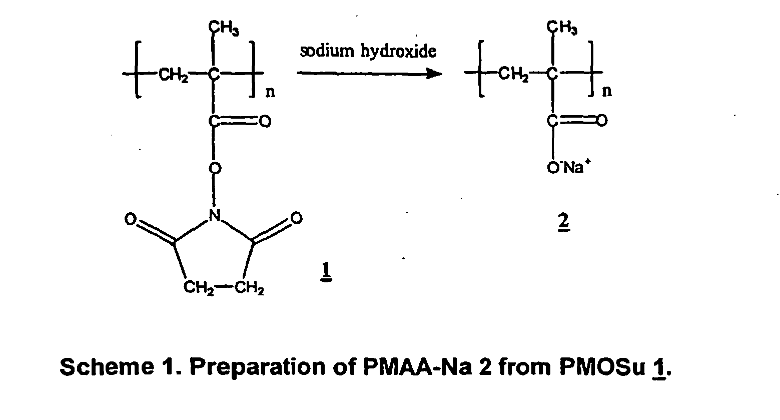

### Example A2: Synthesis of PMOSu 1.

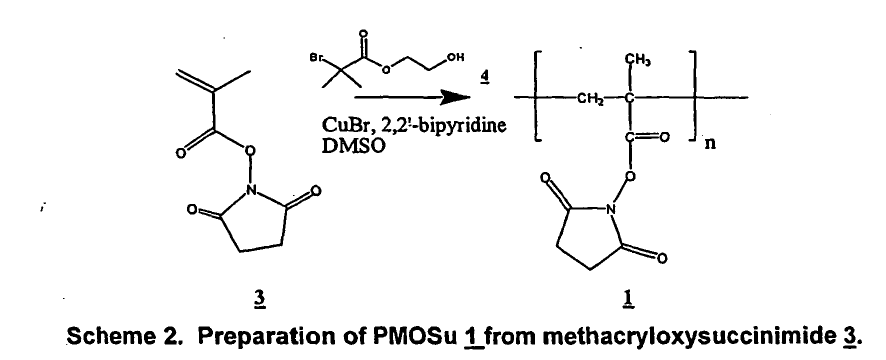

*Synthesis of methacryloxysuccinimide* ***3*.** To N-hydroxysuccinimide (6.6 g, 57 mmol) in dichloromethane (12 ml) was added dropwise a dichloromethane (12 ml) solution of methacryloyl chloride (6.0 g, 57 mmol) simultaneously with a dichloromethane solution (12 ml) of triethylamine (5.8 g, 57 mmol) maintaining the temperature below 5 °C. After complete addition the reaction mixture was further stirred for 1 h and then washed with aqueous sodium hydrogen carbonate (0.1 M) and water (3 times). The organic phase was then isolated and dried with magnesium sulfate. The solvent was removed to leave the product as a white solid which was recrystalised from ethyl acetate:hexane. Mass 8 g, m.p.= 102°C.
(¹H, 500MHz, DMSO-d₆): 2.00 (3H, s, CH3), 2.84 (4H, s, (CH₂)₂), 6.09 (1H, s, =CH₂), 6.34 (1 H, s, =CH₂).

*Homogeneous polymerisation:* This reaction is shown in Scheme 2. In a typical copper mediated polymerisation using DMSO as solvent at the preferred weight concentration of 56% in monomer **3**, copper(I)bromide (31.3 mg, 0.2 mmol), 2-2'-bipyridine (Bpy) (68.3 mg, 0.4 mmol) and monomer **3** (2.00 g, 10.9 mmol) were added to a round bottomed flask which was then sealed with a septum. Into the flask was then injected DMSO (1.3 g). The resulting brown mixture was gently heated until a solution had formed and then purged with argon for approximately 5 min. An argon purged solution of 2-bromo-2-methyl-(2-hydroxyethyl)propaneate **4** (46.1 mg, 0.2 mmol) in DMSO (0.2 g) was then injected into the mixture and the flask was heated to 100 °C in an oil bath. The reaction mixture became viscous after a few minutes and was removed from the heat after 10-15 minutes and rapidly cooled. The polymeric product was isolated by addition of 7-8 ml of DMSO to dissolve the crude product mixture which was slowly added to a stirred solution of acetone (100 ml) to precipitate PMOSu **1** as a white solid. The acetone solution turned a green colour during the precipitation of polymer 1 due to the dissolution of copper species and the ligand. Atomic absorption analysis indicated the copper content in polymer **1** when at a concentration of 28.0 mg/ml in DMF to be 0.153 ppm. Precipitation of polymer **1** from the DMSO reaction solution into acetone may offer a viable alternative to alumina chromatography that has been typically used in copper mediated polymerisations to remove copper from the product polymer. The isolated yield of polymer **1** was 1.78 g (89%). The number average molecular weight was 22,700 g/mol and polydispersity index was 1.20. Apparent molecular weights and molecular weight distributions for PMOSu **1** were determined using Waters Styragel HR4 and HR3 (7.3 x 300 mm) columns coupled to a Gibson 133 refractive index detector, poly(methyl methacrylate) PMMA calibration standards and DMF with 0.1% LiCl eluent.

Polymerisations were conducted with different ratios of monomer **3** and initiator **4** to give narrow MWD PMOSu **1** with different molecular weights. These experiments are listed in Table 1 and have been conducted on reaction scales ranging from 2-6 g in methacryloxysuccinimide **3**. These homogeneous polymerisation conditions in DMSO gave the polymer **1** in a matter of minutes (e.g. experiment 6 in Table 1 was quenched after 2 minutes to give a significant yield of narrow MWD polymer **1**).

Polymerisations were conducted at temperatures ranging from 80-130°C to maintain solution homogeneity at methacryloxysuccinimide **3** to solvent weight ratios spanning 33-91 %. The preferred solvent was DMSO, but similar results were obtained with DMF. The weight ratio of monomer **3** to polar solvent (DMSO or DMF) was critical for the outcome of the polymerization. In DMSO at weight ratios less than 56% monomer **3** (e.g. 50 and 41%) resulted in lower yields of polymer (52 and 40% respectively). At weight concentrations higher than 60% monomer **3** in DMSO, the polymerisation solution solidified. Likewise in DMF, the weight concentration of monomer was critical for the outcome of the polymerisation reaction, however the maximal yield in DMF was less than in DMSO. A 50 % yield of polymer **1** was isolated at monomer 3:DMF weight ratio of 61 %. No polymer was isolated when the reaction was conducted at a monomer **3** weight ratio of 33 %. At higher monomer weight concentrations (above 75 %), the reaction mixture solidified.

**TABLE 1**

| Experiment | 1:2:CuBr:Bpy^{a} | T °C | Yield, % | *M̅ₙ* | *M̅_{w}*/*M̅ₙ* |
|---|---|---|---|---|---|
| 1 | 10:1:1:2 | 100 | 85 | 12500 | 1.17 |
| 2 | 20:1:1:2 | 80 | 92 | 16800 | 1.15 |
| 3 | 50:1:1:2 | 100 | 89 | 22700 | 1.20 |
| 4 | 100:1:1:2 | 100 | 96 | 29000 | 1.14 |
| 5 | 150:1:1:2 | 110 | 80 | 40700 | 1.13 |
| 6^{b} | 100:1:1:2 | 100 | 49 | 23330 | 1.15 |

| | | | | | |
|---|---|---|---|---|---|
| (a) Ratio of initial monomer and initiator concentrations. (b) Reaction stopped after 2.5 minutes by dilution with, DMSO and rapid cooling. | | | | | |

*(B) Precipitation polymerisation:* Copper mediated polymerisations of monomer **3** in solvents such as THF, ethyl acetate, toluene and acetone also gave narrow MWD polymer 1. Yields ranged from 10-95 % depending on the polymer molecular weight. Higher molecular weight polymer **1** up to 25,000 g/mol was obtained in mixed solvent systems such as THF and ethylene carbonate. At molecular weights above 10,000 g/mol the yields were sometimes less than that observed when the polymerisation was conducted in DMSO or DMF. Exemplary copper mediated polymerisations using 0.5 g in monomer **1** were conducted in THF over a 16 h time period at 70 °C and are listed in Table 2. The copper chelating legend used in these THF reactions was N, N, N', N", N"-pentamethyldiethylenetriamine (PMDETA). Additional precipitation reactions are listed in Table 3 with 2-bromo-2-methyl propionic acid (BMA) used as initiator.

**TABLE 2**

| Experiment | 1:2:CuBr:PMDETA^{a} | *M̅ₙ* | *M̅_{w}*/*M̅ₙ* |
|---|---|---|---|
| 1 | 100:1:1:1.2 | 14,800 | 1.10 |
| 2 | 200:1:1:1.2 | 1,800 | 1.12 |
| 3 | 100:1:0.3:1.2 | 13,100 | 1.09 |

**TABLE 3**

| [MOSu]ₒ:[BMA]ₒ: | Solvent | Yield | Mₙ | M_{w}/Mₙ |
|---|---|---|---|---|
| [CuBr]ₒ:[Ligand]ₒ | (wt%) | (%) | | |
| 50:1:1:2(bipy) | EC (45) | 39 | 34,600 | 1.43 |
| 100:1:1:1.2(PMDETA) | THF/EC 5:1 (74) | 67^{a} | 25,300 | 1.39 |
| 100:1:1:1 (bipy) | THF/EC 6:1 (78) | 75^{a} | 19,500 | 1.29 |
| 100:1:1:1(bipy) | THF/EC 20:1 (84) | 82^{a} | 12,200 | 1.12 |
| 50:1:0.5:1 (bipy) | THF/EC 20:1 (84) | 74^{a} | 12,000 | 1.09 |
| 100:1:1:1 (bpy) | THFJEC 27:1 (87.5) ^{b} | 22 | 10,000 | 1.08 |
| 50:1:0.5:1(bpy) | THF/EC 27:1 (87.5)^{b} | 24 | 12,500 | 1.10 |

| | | | | |
|---|---|---|---|---|
| ^{a} The product was directly filtered and washed. ^{b} Freshly distilled THF | | | | |

### Example A3: Hydrolysis of PMOSu 1 to give PMAA-Na 2 (Scheme 1).

PMOSu **1** (1.00 g, Mₙ = 24,800 g/mol, M_{w}/Mₙ =1.20, DMF eluent, PMMA standards) was dissolved in DMF (5.0 ml) and then 2 M aqueous sodium hydroxide (6.0 ml) was added drop-wise under stirring causing some precipitation of the polymer. The reaction vessel quickly became warm and a homogeneous solution soon followed. The solution was then heated at 70 °C for 24 h after which time further water was added (approximately 50 ml). The diluted solution was then dialysed against water using regenerated cellulose membrane (MWCO 2000, SpectraPor). Freeze-drying of the dialysed solution gave a white solid product, PMAA-Na **2** (0.3 g). GPC (PBS eluent, PMAA-Na standards) Mₙ = 22,000 g/mol and M_{w}/Mₙ = 1.28; FT-IR (ATR) 1675 cm⁻¹, 1547 cm⁻¹, 1194 cm⁻¹.

The molecular weight value for PMAA-Na **2** can be used to determine the degree of polymerization (DP) to know the number of repeat units for any polymer derived from **1**. In this example the repeat unit molecular weight of PMAA Na **2** is 108, the DP for this sample was approximately 203 (i.e. 22,000 g/mol ÷ 108 g/mol). This means the DP for PMOSu **1** is 203, and since the molecular weight of the repeat unit of PMOSu **1** is 183 g/mol, then the absolute number average molecular weight of PMOSu **1** in this example was 37,149 g/mol (i.e. 183g/mol □ 203). The value of 203 for the DP of narrow MWD PMOSu **1** can be used in an analogous fashion to determine the absolute molecular weight of polymers derived from **1**.

### Example A4: Hydrolysis of PMOSu 1.

PMOSu **1** (200 mg, Mₙ= 32,200 g/mol, M_{w}/Mₙ =1.24, DMF eluent, PMMA standards), was dissolved in DMSO (2 ml) and under stirring, 1 M aqueous sodium hydroxide (2.2 ml) was added dropwise. Typically, some precipitation occurred. Additional water (23 ml) was added immediately following the addition of sodium hydroxide and the mixture left to stir at room temperature for 1 h. The resulting reaction solution was then diluted to 44 ml and dialysed against 1 L water for 24 h using Visking dialysis membrane (MWCO 7000, Medicell International). During dialysis, the water was changed 6 times. The dialysed solution was filtered through a 0.2 µm filter and then freeze-dried to afford 0.2 g of PMAA-Na product 2. GPC (Triple detection, NaNO₃ 0.2 M/ 10% CH₃CN) Mₙ = 35,700 g/mol and M_{w}/Mₙ = 1. 18).

Each preparation of PMAA-Na **2** that was prepared was characterised using ¹H Nuclear Magnetic Resonance and Fourier-Transform Infra-Red Spectroscopy and was found to be consistent with the desired product.

### Example A5: Synthesis of PMOSu 1 by free radical polymerization with AIBN followed by hydrolysis to give PMAA-Na 2.

An argon purged solution of methacryloxy succinimide **3** (3 g) and AIBN (0.135 g) in acetone (30.0 ml) was heated at 50°C in a closed vessel for 24 h. The white precipitate formed was isolated by filtration, dissolved in DMSO (6.0 ml) and reprecipitated in rapidly stirring acetone. After isolating by filtration and drying in-vacuo, some of the dried PMOSu **1** (0.48 g) in DMSO (4.8 ml) was mixed with 1 N sodium hydroxide (5.2 ml) and water (56 ml). The resulting solution was allowed to stir for 1 h at room temperature whereupon it was diluted to 105 mL with fresh water and dialysed against 5 L of water for 24 h using a Visking dialysis membrane (MWCO 7000, Medicell International). The 5 L of water was changed 6 times. The dialysed solution was filtered though a 0.2 µm filter and then freeze-dried to afford PMAA-Na **2** (0.56 g) as a solid product; Mw 26,100 Da, Mn 15,200 g/mol, Mw/Mn 1.7 (SEC 0.2 M aqueous NaNO₃/ 10% CH₃CN, PMAA-Na standards)

### Example A6: Synthesis of PMOSu 1 by free radical polymerization with 4,4'-azobis(cyanovaleric acid) followed by hydrolysis to give PMAA-Na 2.

A pressure tube was charged with methacryloxysuccinimide 3 (0.5-1.0 g), and 4,4'-azobis(cyanovaleric acid) (15-30 wt%) and solvent (for example acetone, freshly distilled THF, or mixtures of these solvents including toluene and ethylene carbonate) and the resulting solution was sealed and purged with argon for 15 min. The sealed flask was then heated at 70-120 °C for 0.25-2.5 h. See Table 4 for the conditions of example reactions. PMOSu **1** was isolated as a white precipitate by filtration and dried in vacuo and GPC determined in DMF using PMMA standards.

**TABLE 4**

| initiator wt% | Solvent (wt% monomer 3) | Time (h) | T/°C | Mₙ (g·mol⁻¹) | M_{w}/Mₙ | Yield (%) |
|---|---|---|---|---|---|---|
| 15 | Acetone (85) | 3.5 | 70 | 14,100 | 1.65 | 80 |
| 15 | THF (86) | 0.5 | 70 | 15,700 | 1.54 | 48 |
| 30 | THF (81.6) | 2.5 | 70 | 14,500 | 1.51 | 74 |
| 30 | THF (81.6) | 2 | 100 | 9,200 | 1.40 | 46.5 |
| 30 | Toluene (81.2) | 1 | 100 | 11,500 | 1.70 | 75 |

### Example B: Synthesis block copolymer PEG-PMOSu 6 and its hydrolysis to give block copolymer PEG-PMAA-Na 7.

The polymerisation and hydrolysis reaction is shown in Scheme 3 and examples have previously been described (Brocchini S. J and Godwin A. "Block Copolymers." International Patent Publication number WO 03/059973 and Pedone et al, An information rich biomedical polymer library, J. Mat. Chem., 2003, 13, 2825-2837).

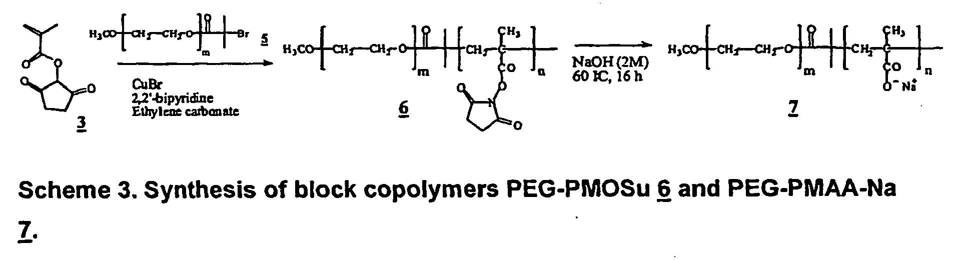

### (A) Preparation of macroinitiators 5.

The PEG macroinitiators 5 were prepared by the procedure of Jankova et al (Macromolecules (1998), 31, 538-541). Triethylamine (12.5×10⁻³ mol, 1.265 g, 1.75 ml) in 15 ml dry CH₂Cl₂ was added to a 250 ml three-neck round-bottom flask equipped with a condenser, dropping funnel, gas inlet and a magnetic stirrer. After cooling to 0°C 2,2-bromoisobutyryl bromide (12.5×10⁻³ mol, 2,874 g" 1.55 ml) in 10 ml CH₂Cl₂ was added and the mixture purged with nitrogen. Then monomethoxy capped PEG (Mn = 2,000 g/mol) (5×10⁻³ mol, 10 g) in 50 ml CH₂Cl₂ was added dropwise during 1 h under nitrogen. The PEG had been previously dried by azeotropic distillation in toluene and the residual toluene removed in vacuum.The temperature of the reaction mixture was allowed to rise to room temperature and the reaction continued for 18h. The solution was filtered, half of the solvent evaporated under vacuum and the product was precipitated in cold ether. The precipitate was recrystallised in absolute ethanol (stored overnight in the fridge). The macroinitiator 5 was filtered, washed with cold ether and dried under vacuum. The crude product was purified by dissolving 4 g in 80 ml water. The solution pH was raised to pH 8 in order to hydrolyse the excess of i-BuBr. Then the solution was extracted with CH₂Cl₂ (70 ml). A stable emulsion was obtained and several hours were needed for complete phase separation. The solvent was removed in vacuum. The product was dissolved in hot EtOH and put in a fridge to crystallise. Then it was filtered and washed with ether and dried under vacuum. The purified product **5** was white in colour. The degree of substitution calculated by the H MNR spectra. This procedure was also used to prepare PEG macroinitiators **5** derived from PEG 5000 and PEG 10000.

### (B) Representative preparation of block PEG-PMOSu 6.

A mixture of monomer **3** (as synthesised in WO 01/18080), ethylene carboate and bipyridine was placed in a tube sealed with a septum and it was purged with argon for 5 minute and the CuBr was added. The misture was gently heated to form a solution (deep brown in colour) and purged with argon for another 30 minutes. Then a solution of the PEG macroinitiator **5** in the amount relative to the monomer specified in Table 5 in ethylene carbonate (gently heated to liquidfy both the ethylene carbon and **5**) was purged with argon for 10 minutes and added to the monomer solution by syringe washed with argon. The mixture was placed in an oil bath and stirred. The reaction was stopped by exposure to air, cooling and diluting with DMF. Then the solution was passed through a column filled with alumina and the polymer precipitated in methanol. The PEG-PMOSu **6** precipitate was filtered, washed with ether and dried in vacuum. The PEG-PMOSu **6** was obtained as a white powder. Table 5 show polymerisation conditions, yield and molecular weight characteristics of polymerisation conducted with microinitiator **5** derived from PEG of molecular weight 2000 g/mol.

**TABLE 5**

| | 3 mmol (g) | 5 (mmol) (g) | CuBr (mmol) (g) | Spy (mmol) (g) | 3:5:Cu Br bpy | EC¹ (g) | T (°C) | Time (h) | Yield (%) | Mn² | PD (Mw/Mn) |
|---|---|---|---|---|---|---|---|---|---|---|---|
| 1 | 5 | 0.1 | 0.1 | 0.2 | 50:1:1:2 | 1.3 | 80 | 1.5, | 56 | 17,000 | 1.39 |
| | 0.917 | 0.215 | 0.0157 | 0.0342 | | | | | | | |
| 2 | 5 | 0.1 | 0.1 | 0.2 | 50:1:1:2 | 1.0 | 80 | 1.0 | 40 | 17,600 | 1.30 |
| | 0.917 | 0.215 | 0.0157 | 0.0342 | | | | | | | |
| 3 | 5 | 0.1 | 0.1 | 0.2 | 50:1:1:2 | 1.3 | 80 | 5.0 | 87 | 20,100 | 1.53 |
| | 0.917 | 0.215 | 0.0157 | 0.0342 | | | | | | | |
| 4 | 5 | 0.05 | 0.05 | 0.1 | 100:1:1:2 | 0.65 | 80 | 5.0 | 100 | 31,300 | 1.32 |
| | 0.917 | 0.1075 | 0.0078 | 0.0171 | | | | | | | |
| 5 | 2.5 | 0,05 | 0.05 | 0.1 | 50:1:1:2 | 0.65 | 60 | 5.0 | 20 | 13,230 | 1.35 |
| | 0.458 | 0.1075 | 0.008 | 0.0171 | | | | | | | |
| 6 | 5 | 0.1 | 0.1 | 0.2 | 50:1:1:2 | 1.3 | 80 | 5.5 | -32 | 19,700 | 1.29 |
| | 0.917 | 0.215 | 0.0157 | 0.0342 | | | | | | | |
| 7 | 2.5 | 0.05 | 0.05 | 0.1 | 50:1:1:2 | 0.65 | 100 | 5.0 | 64 | 28,000 | 1.38 |
| | 0.458 | 0.1075 | 0.005 | 0.0171 | | | | | | | |
| 8 | 2.5 | 0,5 | 0.05 | 0.1 | 50:1:1:2 | 0.65 | 80 | 5.0 | 56 | 25,700 | 1.29 |
| | 0.458 | 0.1075 | 0.008 | 0.0171 | | | | | | | |
| 9 | 2.5 | 0,05 | 0.05 | 0.1 | 50:1:1:2 | 0.65 | 80 | 5.0 | 47 | 22,800 | 1.27 |
| | 0.458 | 0.1075 | 0.008 | 0.0171 | | | | | | | |
| 10 | 2.5 | 0,05 | 0.025 | 0.1 | 50:1:1:2 | 0.5 | 80 | 5.0 | 74³ | 30,200 | 1.28 |
| | 0.458 | 0.1075 | 0.008 | 0.017 | | | | | | | |
| 11 | 2.5 | 0,05 | 0.025 | 0.15 | 50:1:0.5:3 | 0.5 | 80 | 3.0 | 42.4³ | 18,100 | 1.25 |
| | 0.458 | 0.1075 | 0.004 | 0.017 | | | | | | | |
| 12 | 5 | 0,05 | 0.05 | 0.1 | 100:1:1:2 | 0.5 | 110 | 4:35 | 100 | 18,600 | 1.33 |
| | 0.917 | 0.1075 | 0.008 | 0.0171 | | | | | | | |

| | | | | | | | | | | | |
|---|---|---|---|---|---|---|---|---|---|---|---|
| ¹ EC = ethylene carbonate ² Gel permeation chromatography used DMF eluent with PMMA standards ³ The reaction mixture was purged with argon for 1 hour. | | | | | | | | | | | |

### (C) Detailed example of the synthesis of PEG-PMMA-Na 7 derived from poly(ethylene glycol) of molecular weight 2000 g/mol.

Copper(I) bromide (31.2 mg, 0.2 mmol), bpy (68.4 mg, 0.4 mmol) and N-methacryloxysuccinimide **3** (3.67 g, 20 mmol) and ethylene carbonate (2 g) were added to a round-bottomed flask that was then sealed with a septum. The resulting brown mixture was gently heated until a solution had formed and then purged with argon for approximately 15 min. An argon-purged solution of PEG₂₀₀₀-macroinitiator **5** (430 mg, 0.2 mmol) in ethylene carbonate (0.6 g) was then injected into the mixture and the flask was heated to 80°C in an oil bath for 1 h. The viscous reaction mixture was then removed from the heat and rapidly cooled. The resulting crude polymeric product was dissolved in DMF (8 ml) and the resulting solution was slowly added to stirred methanol (500 ml) to precipitate PEG-PMOSu **6** as a white solid (3.8 g, 92%); Mₙ = 37,160 g.mol⁻¹ M_{w}/Mₙ = 1.32 SEC (DMF 0.1% LiCl). ¹H NMR (DMSO-d₆) δ 1.38 (br, 3H, CH₃), 2.42 (br m, 2H, CH₂C), 2.78 (br, 4H, CH₂CH₂), 3.50 (s, 4H, OCH₂CH₂O).

Hydrolysis of block PEG-PMOSu **6** was conducted. Hydrolysis of this to PEG-PMAA-Na salt was conducted by slowly adding 2M NaOH (0.6 ml) dropwise to a stirred solution of block polymer precursor (0.1 g, 0.54 mmol) in DMF (0.5 ml) in a 10 ml round bottom flask. The reaction mixture was heated to 60°C for 16 hand more water (5.0 ml) was added. The dilute solution was then dialysed using Visking tubing (MWCO 12,000-14,000) and the dialysate was freeze dried to give a white solid product PEG₂₀₀₀-PMAA-Na salt 7 (95% yield) Mₙ = 34,110 g.mol⁻¹ M_{w}/Mₙ = 1.34 (Triple detection-SEC; NaNO₃ 0.2 M/ 10% CH₃CN). ¹H-NMR (D₂O) δ 0.85-0.96 (br s, 3H, CH₃) 1.60, 1.88 (br, m, 2H, CH₂) and 3.58 (s, 4H, OCH₂CH₂O) confirming the hindered ester bond to the PEG₂₀₀₀-block was not hydrolysed.

### (D) Detailed example of the synthesis of PEG-PMMA-Na 7 derived from poly(ethylene glycol) of molecular weight 5000 g/mol.

Copper(I) bromide (10.4 mg, 0.072 mmol), 2,2'-bipyridine (bpy, 22.6 mg, 0.145 mmol), and N-methacryloxysuccinimide (2.0 g, 10.8 mmol), and PEG₅₀₀₀-macroinitiator 5 (362 mg, 0.072 mmol) and ethylene carbonate (2.362 g) were added to a Schlenk flask which was then sealed with a septum. The resulting brown mixture was degassed by freeze thaw method. The mixture was heated to 110°C in an oil bath for 2 h. The viscous reaction mixture was then removed from the heat and rapidly cooled. The resulting crude polymeric product was then dissolved in DMF (8 ml). The resulting solution was slowly added to stirred methanol/diethylether (2:1 v/v, 300 ml) to precipitate PEG-PMOSu 6 as a white solid (1.8 g, 76%). Mₙ = 32,860 g.mol⁻¹ M_{w}/Mₙ= 1.36 (SEC (DMF 0.1% LiCl). ¹H NMR (DMSO-d₆) δ 1.38 (br, 3H, CH₃), 2.42 (br m, 2H, CH₂C), 2.78 (br, 4H, CH₂CH₂), 3.50 (s, 4H, OCH₂CH₂O).

Hydrolysis of PEG-PMOSu **6** was conducted by slowly adding 2M NaOH (0.6 ml) dropwise to a stirred solution of the block polymer precursor 6 (0.1 g, 0.54 mmol) in DMF (0.5 ml) in a 10 ml round bottom flask. The reaction mixture was heated to 60 °C for 16 h and more water (5.0 ml) was added. The dilute solution was then dialysed using Visking tubing (MWCO 12,000-14,000) and the dialysate was freeze dried to give PEG-PMAA-Na salt **7** as white solid product PEG₅₀₀₀-PMAA-Na salt (75% yield); Mₙ = 29,360 g·mol⁻¹ M_{w}/Mₙ= 1.28 (Triple detection-SEC; NaNO₃ 0.2 M/10% CH₃CN). ¹H-NMR (D₂O) δ 0.87-0.97 (br s, 3H, CH₃) 1.63, 1.89 (br, m, 2H, CH₂) and 3.58 (s, 4H, OCH₂CH₂O) confirming the hindered ester bond to the PEG₅₀₀₀-block was not hydrolysed.

### EXAMPLE C) Preparation of amphotericin B - PMAA-Na preparation:

### Example C1:

The polymer, PMOSu (40 mg), was dissolved in DMSO (0.4 ml) and the solution added to a vial containing amphotericin B (the "drug") (50 mg). Under stirring, 1 M aqueous sodium hydroxide (0.44 ml) was added dropwise to the resulting drug mixture. Typically, some precipitate was observed: Additional water (4.7 ml) was added immediately following the addition of sodium hydroxide and the resulting mixture left to stir at room temperature for 1 h. The reaction solution was then diluted to 8.8 ml and dialysed against water (1L) for 24 h using Visking dialysis membrane (MWCO 7000, Medicell International). During dialysis the water was changed 6 times. The dialysed solution was filtered through a 0.2 µm filter and then freeze-dried to afford 0.9 g of a yellow solid product. FT-IR (ATR) 1676 cm⁻¹, 1568 cm⁻¹, 1404 cm⁻¹, 1070 cm⁻¹, 1012 cm⁻¹.

The presence of drug and polymer in the preparations was confirmed by ¹H NMR and FT-IR spectroscopy. The weight percent (wt%) of amphotericin B in the preparations was estimated using UV spectrometry at 419.5 nm against a calibration curve made using amphotericin B obtained from Bristol-Myers Squibb Pharmaceuticals Ltd. The wt% in example C1 was estimated to be 44 to 48%.

Prior to their biological evaluation, aqueous solutions of methacrylic acid sodium salt homo-polymers and co-polymers were treated to remove endotoxin using activated carbon or polymixin B columns. Endotoxin levels were determined using the limulus amebocyte lysate (LAL) assay and the compounds used in the experiments described contained <0.06 endotoxin units/ml. This is the European Community standard for water for injection.

### D) Cellular toxicity of methacrylic acid sodium salt homo-polymer and co-polymers on primary cells:

All experiments were performed on primary human cells.

### Example D1: Human red blood cells:

A 2% v/v solution of human red blood cells was prepared in RPMI 1640. A stock solution of PMAA-Na ("drug") was prepared in RPMI 1640. A 1% solution of Triton X-100 was used as a positive reference for 100% cell lysis. Dextran and poly-L-lysine were used as negative and positive controls respectively. An equal volume of the sample and red blood cells were aliquoted into a 96 well microtitre plate and incubated at 37°C. After 1 h and after 24 h, each sample was centrifuged (2,000g, 10 min) and the supernatant added to a 96 well microtitre plate. The absorbance was measured at 490 nm using a spectrophotometer. The degree of lysis was expressed as a percentage of the 100% lysis caused by Triton X-100. No significant toxicity was seen with the PMAA-Na using human red blood cells from 3 donors (A, B and C) up to a concentration of 2,000 µg/ml after 1 h or after 24 hours as shown in Figure 1.

### Example D2: Human whole blood:

A 2% v/v solution of human whole blood was prepared from donor D in RPMI 1640. A stock solution of PMAA-Na ("drug") was prepared in RPMI 1640. A 1% solution of Triton X-100 was used as a positive reference for 100% cell lysis. Dextran and poly-L-lysine were used as negative and positive controls respectively. An equal volume of the sample and whole blood were aliquoted into a 96 well microtitre plate and incubated at 37°C. After 1 h, 6 h and after 24 h, each sample was centrifuged (500g, 10 min) and the supernatant added to a 96 well microtitre plate. The absorbance was measured at 490 nm using a spectrophotometer. The degree of lysis was expressed as a percentage of the 100% lysis caused by Triton X-100. No significant toxicity was seen with the PMAA-Na using human whole blood up to a concentration of 500 µg/ml after 1 h or after 6 h or after 24 hours as shown in Figure 2.

### Example D3

### a) Monocyte derived macrophages:

Monocytes were separated from fresh blood from single donors and cultured in RPMI 1640, 20 mM L-glutamine, penicillin (200 IU/ml), streptomycin (200 µg/ml) and 10 % human serum and plated at a density of 1 x 10⁶ cells/ml. The monocytes were allowed to differentiate to monocyte derived macrophages (MDMs) by a further 3 days of adherence. Media containing PMAA-Na was then added to the cells over the concentration range of 0 - 2,000 µg/ml. The cells were incubated for 71 h prior to the addition of thiazolyl blue (MTT, Sigma 5 mg/ml). The viability of the cell culture was expressed as a percentage of the viability of cells grown in the absence of any compound. Dextran and poly(L-lysine) were used as negative and positive controls respectively. PMAA-Na was not toxic to MDMs at the highest concentration of 2,000 µg/ml tested using the MTT assay and the Trypan blue assay as shown in Figure 3a.

### b) Peritoneal tissue macrophages:

Human peritoneal cells were cultured in RPMI 1640 medium, 20 mM L-glutamine, 10% mixed donor human serum, 200 IU/ml penicillin and 200 µg/ml streptomycin and their density adjusted to 1 x 10⁶ cells/ml. Media containing PMAA-Na was then added to the cells over the concentration range of 0 - 2,000 µg/ml. The cells were incubated for 71 h prior to the addition of MTT. The viability of the cells was expressed as a percentage of the viability of cells grown in the absence of any compound. Dextran and poly(L-lysine) were used as negative and positive controls respectively. PMAA-Na was not toxic to peritoneal macrophages up to 500 µg/ml. Between 500 µg/m and 2,000 µg/ml, a modest amount of toxicity was seen using the MTT assay and the Trypan blue assays as shown in Figure 3b.

### E) Anticoagulant activity:

***Example E1:*** The anticoagulant activity was determined as a measure of the prothrombin time (PT), kaolin partial thromboplastin time (APTT), thrombin time (TT), fibrinogen and anti-Xa activity. The compounds were solubilised in phosphate-buffered saline solution and were mixed 50 : 50 with plasma / veronal buffer (1 : 1) and tested. The anti-Xa activity was expressed as units of heparin/ml (HEP (Xa) (V/ml))*.* PMAA-Na prolonged the APTT and the TT but did not affect the PT. The anti-Xa assay was negative. Therefore, the anticoagulant activity of these molecules was not due to "heparin like" activity, see Table 6.

**Table 6**

| Anticoagulant activity of PMAA-Na: | | | | | | | |
|---|---|---|---|---|---|---|---|
| | Dissolved in | Conc (µg/m)) | PT (sec) | APTT (sec) | TT (sec) | Fibrinogen (g/L) | Anti-Xa (V/ml) |
| Control | Plasma/veronal buffer | | 16.7 | 40 | 14 | 1.31 | 0 |
| PMAA-Na | Plasma/veronal buffer | 500 | 14.9 | 144 | 67 | 0.47 | 0 |

Peritoneal cells from patients on continuous ambulatory peritoneal dialysis (CAPD) were cultured with each compound and culture supematants harvested after 36 h of incubation. Pro-inflammatory chemokines and pro-inflammatory cytokines were measured by EIA (R & D Systems).

### Example F1:

Human peritoneal cells from 3 donors (A, B and C) were cultured with PMAA-Na (500 µg/ml) and culture supernatants harvested after 36 h were analysed for MIP-1β. All reagents and the PMAA-Na contained <0.06 endotoxin units/ml (EU/ml) as determined using the *Limulus* amebocyte lysate assay (Pyrotell, Associates of Cape Cod, US). This is the European Community standard value for water for injection. There was a significant release of MIP-1β in the presence of PMAA-Na as shown in Figure 4.

### Example F2:

Human peritoneal cells from 2 donors (A and B) were cultured with PMAA-Na (500 µg/ml and 2,000 µg/ml) and culture supernatants harvested after 36 h were analysed for TNF-α. All reagents and the PMAA-Na contained <0.06 endotoxin unit/ml. There was a significant release of TNF-α in the presence of PMAA-Na at both concentrations as shown in Figure 5.

### Example F3:

Human peritoneal cells were cultured with PMAA-Na (500 µg/ml) and culture supernatants harvested after 36 h were analysed for the pro-inflammatory chemokines MIP-1α, MIP-1β and IL-8, and for the pro-inflammatory cytokines TNF-α, I L-1β and IL-6. All reagents and the PMAA-Na contained <0.06 endotoxin unit/ml. The results with cells from up to 3 different human donors (A, B and C) are shown in Figure 6. The release of these chemokines and cytokines from tissue antigen presenting cells was at a level that would promote a pharmacological Th1 response in man but not high enough to cause significant adverse side-effects in man.

### Example F4:

Blood-derived monocyte derived macrophages were cultured with PMAA-Na at concentrations of up to 2,000 µg/ml. All reagents and the PMAA-Na contained <0.06 endotoxin unit/ml. No release of MI P-1β was seen. The results with cells from 3 different human donors (A, B and C) are shown in Figure 7. There is therefore a differential immuno-modulatory effect of PMAA-Na on cells of blood monocyte origin compared to cells of macrophage origin and other antigen presenting cells (e.g., dendritic cells) from tissue body based compartments.

### G) Lack of Th1 adjuvant activity of commercially available PMAA-Na:

### Example G1:

Human peritoneal macrophages were cultured with commercially available PMAA-Na (Polymer Standards Service, Germany) of a range of MWt's (*M*ₙ = 1,300, 22,100 and 129,000 g/mol) and narrow molecular weight distribution. All reagents and the PMAA-Na contained <0.06 endotoxin unit/ml. One of the commercially available PMAA-Na possessed a MWt comparable to our synthetic preparation of PMAA-Na (*M*ₙ = 22,000 g/mol). The culture supernatants were harvested after 36 h. There was no release of MIP-1β. Figure 8 shows the individual results from 2 human donors (A and B).

### Example G2:

Human peritoneal macrophages were cultured with commercially available PMAA-Na (Polymer Standards Service, Germany) of a range of MWt's (*M*ₙ = 1,300, 22,100 and 129,000 g/mol) and narrow molecular weight distribution. One of the commercially available PMAA-Na possessed a MWt comparable to our synthetic preparation of PMAA-Na (*M*ₙ = 22,000 g/mol). All reagents and the PMAA-Na contained <0.06 endotoxin unit/ml. The culture supernatants were harvested after 36 h. There was no release of TNF-α. Figure 9 shows the individual results from 2 human donors (A and B).

### H) Cellular toxicity of the amphotericin B - PMAA-Na preparation on primary human cells:

### Example H1: Red blood cells:

The method was as described for Example D1. The comparison was made between clinical grade amphotericin B and the amphotericin B - PMAA-Na preparation (both called "drug" in the Figure). Stock solutions of the compounds for testing were prepared in MGM. Human red cell lysis was determined after 1 hour (Figure 10), 6 hour (Figure 11) and 24 hour (Figure 12) incubation in 3 different donors (A, B and C). No toxicity was seen with the amphotericin B - PMAA-Na preparation after a 1 hour incubation. After a 6 hour incubation, the toxicity of the amphotericin B - PMAA-Na preparation was considerably less than that of clinical grade amphotericin B. When the incubation time was increased to 24 h, the toxicity of the clinical grade amphotericin B increased to 100% but there was no further increase in the toxicity of the amphotericin B - PMAA-Na preparation.

### Example H2: Red blood cells:

The method was as described for Example H1. The degree of red cell lysis by the amphotericin B - PMAA-Na preparation ("drug") was determined after a 1 hour and 24 hour incubation for concentrations up to 1,000 µg/ml. The results are shown in Figure 13.

### Example H3: Peripheral blood mononuclear cells:

Peripheral blood mononuclear cells (PBMCs) cells were suspended in RPMI medium, 10% mixed donor human serum, 200 IU/ml penicillin and 200 µg/ml streptomycin at 1 x 10⁵ cells and cultured in 96-well tissue culture plates at 37°C with 5% CO₂. Media containing the amphotericin B - PMAA-Na preparation ("drug") was added to the cells over the concentration range of 0 - 70 µg/ml in a final volume of 100 µl/well. The cells were incubated for 1 day or 2 days or 6 days prior to the addition of MTT (5 mg/ml). The MTT solution was removed after 1 h and DMSO (100 µl) added to dissolve the MTT crystals. The optical density was measured at 550 nm using a plate reader (Molecular Devices, Wokingham, UK). The viability of the cell culture was expressed as a percentage of the viability of the cells grown in the absence of any compound. Dextran and poly(L-lysine) were used as negative and positive controls respectively. The compound was not toxic to peripheral blood mononuclear cells at the highest concentration of 70 µg/ml tested using the MTT assay and the Trypan blue assay after 1 day of culture, 2 days of culture or after 6 days of culture. Figure 14 shows the individual results from up to 3 representative human donors. The results from each donor are shown as line graphs in Figure 14.

### Example H4: Monocyte derived macrophages:

Monocyte derived macrophages (MDM) cells were separated from fresh blood from single donors by density gradient centrifugation and cultured in macrophage growth medium (MGM) containing RPMI 1640, 20 mM L-glutamine, penicillin (250 IU/ml), streptomycin (250 µg/ml) and 10 % human serum. They were plated at a density of 1 x 10⁶ cells/ml and allowed to differentiate to MDMs for 3 days. Media containing the compounds ("drugs") was then added to the cells up to a concentration of 125 µg/ml. The cells were incubated for 2 days or 3 days prior to the addition of MTT. Cell viability was expressed as a percentage of the viability of cells grown in the absence of any compound. Dextran and poly(L-lysine) were used as negative and positive controls respectively. The amphotericin B - PMAA-Na preparation was less toxic than clinical grade amphotericin B at all of the concentrations up to 125 µg/ml tested using the MTT assay and the Trypan blue assay after both 2 days and after 3 days of culture as shown in Figure 15.

### J) Determination of the anti-leishmania activity of the amphotericin B - PMAA- Na preparation against Leishmania promastigotes:

### Example J1:

*Leishmania mexicana* promastigotes were used for these experiments. They were maintained in Schneider's Drosophila growth medium (Invitrogen) supplemented with 15% fetal calf serum (heat inactivated at 56°C for 1 hour) and gentamicin (1 mg/100 ml). The parasite concentration was adjusted to 2 x 10⁶ parasites/ml. Doubling dilutions of the test compound were prepared at twice the desired final concentration in growth media. An equal volume of the drug and the parasite suspension were then mixed in a 96 well plate and incubated for 24 h at 26°C. At this time, MTT (5 mg/ml) was added and the plate incubated for a further 24 h at 26°C. The plate was then centrifuged (2000g, 5 min), the supernatant discarded and the pellet resuspended in 100 µl DMSO. Absorbance was measured at 570 nm using a spectrophotometer. The results were expressed as the percentage viability of the promastigotes with 100% viability being determined using the OD of the untreated, replicating promastigotes in the control wells.

The 50% lethal dose (LD₅₀) of clinical grade amphotericin B for *Leishmania mexicana* promastigotes was 0.14 µg/ml (Figure 16 inset). The 90% lethal dose (LD₉₀) of clinical grade amphotericin B for Leishmania mexicana promastigotes was 1.49 µg/ml (Figure 16 inset). The results from 3 experiments with the amphotericin B - PMAA-Na preparation are shown in Figure 16. The 50% lethal dose (LD₅₀) of the amphotericin B - PMAA-Na preparation for Leishmania mexicana promastigotes was 0.10 - 0.19 µg/ml (Figure 16). The 90% lethal dose (LD₉₀) of amphotericin B - PMAA-Na preparation for Leishmania mexicana promastigotes was 1.02 - 1.49 µg/ml (Figure 16). The activity of the amphotericin B - PMAA-Na preparation against Leishmania mexicana promastigotes was similar to that of clinical grade amphotericin B on a weight per weight basis.

### Example J2:

*Leishmania donovania* promastigotes were used for these experiments. They were maintained in Schneider's Growth Media 199 supplemented with 15% fetal calf serum (heat inactivated at 56°C for 1 hour) and gentamicin (1 mg/100 ml). The parasite concentration was adjusted to 2 x 10⁶ parasites/ml. Doubling dilutions of the test compound were prepared at twice the desired final concentration in growth media. An equal volume of the drug and the parasite suspension were then mixed in a 96 well plate and incubated for 24 h at 26°C. At this time, MTT (5 mg/ml) was added and the plate incubated for a further 24 h at 26°C. The plate was then centrifuged (2000g, 5 min), the supernatant discarded and the pellet resuspended in 100µl DMSO. Absorbance was measured at 570 nm using a spectrophotometer. The results were expressed as the percentage viability of the promastigotes with 100% viability being determined using the OD of the untreated, replicating promastigotes in the control wells.

The 50% lethal dose (LD₅₀) of clinical grade amphotericin B for *Leishmania donovani* promastigotes was 0.08 µg/ml (Figure 17 inset). The 90% lethal dose (LD₉₀) of clinical grade amphotericin, B for Leishmania donovania promastigotes was 1.91 µg/ml (Figure 17 inset). The results from 3 experiments with the amphotericin B - PMAA-Na preparation are shown in Figure 17. The 50% lethal dose (LD₅₀) of amphotericin B - PMAA-Na preparation for Leishmania donovani promastigotes was 0.10-0.17 µg/ml (Figure 17). The 90% lethal dose (LD₉₀) of amphotericin B - PMAA-Na preparation for Leishmania donovani promastigotes was 0.98 - 1.28 µg/ml (Figure 17). The activity of the amphotericin B - PMAA-Na preparation against Leishmania donovani promastigotes was therefore similar to that of clinical grade amphotericin B on a weight-per-weight basis.

### K) Determination of the anti-leishmania activity of the amphotericin B - PMAA-Na preparation against Leishmania amastigotes:

### Example K1:

*Leishmania mexicana* amastigotes were used to infect human monocyte derived macrophages that were maintained in RPMI 1640 (Invitrogen) supplemented with 10% human serum (heat inactivated at 56°C for 1 hour) and 200 IU/ml penicillin and 200 µg/ml streptomycin. The cells were plated at 10⁶ cells/ml into Lab-Tek - Chamber Slides (Nunc) and incubated at 37°C/5% CO₂ for 3 days. The media was then aspirated from the chamber slides and an equal volume of fresh media added back in which the amastigote concentration had been adjusted to give a parasite:cell infection ratio of 5:1. The cells were then incubated at 32°C for 20 h to enable infection of the macrophages to become established. Media was then aspirated from the chamber slides and replaced with doubling dilutions of the test compound. The cells were then incubated at 32°C for 72 h. After washing with PBS, the chambers were detached, the slides allowed to dry and then fixed in methanol. Each slide was then stained with Giemsa and examined microscopically. A total of 250 cells were counted to determine the number of infected and uninfected cells.

The infection index was calculated as the average number of parasites/cell multiplied by the percentage of infected cells. A 50% inhibition of intracellular *Leishmania mexicana* amastigote growth was achieved with 0.18 - 0.32 µg/ml of the amphotericin B - PMAA-Na preparation (Figure 18) compared to 0.14 µg/ml of clinical grade amphotericin B (Figure 19a) or 0.45 µg/ml of Ambisome (Figure 19b). A 90% inhibition of intracellular *Leishmania mexicana* amastigote growth was achieved using 1.18 - 1.55 µg/ml of the amphotericin B - PMAA-Na preparation compared to 0.95 µg/ml of clinical grade amphotericin B or 3.89 µg/ml of Ambisome.

This graphs in Figures 19 and 20 compare the slopes of relative activity of clinical grade amphotericin B and Ambisome (liposomal amphotericin B, Gilead Sciences, Great Abingdon, Cambridge, UK) and amphotericin B - PMAA-Na preparation against intracellular *Leishmania mexicana* amastigotes. They demonstrate that the killing curve for amphotericin B - PMAA-Na preparation is steeper than the killing curve for Ambisome.

### Example K2:

*Leishmania donovani* amastigotes were used to infect human monocyte derived macrophages that were maintained in RPMI 1640 (Invitrogen) supplemented with 10% human serum (heat inactivated at 56°C for 1 hour) and 200 IU/ml penicillin and 200 µg/ml streptomycin. The cells were plated at 10⁶ cells/ml into Lab-Tek Chamber Slides (Nunc) and incubated at 37°C/5% CO₂ for 3 days. The media was then aspirated from the chamber slides and an equal volume of fresh media added back in which the amastigote concentration had been adjusted to give a parasite:cell infection ratio of 5:1. The cells were then incubated at 32°C for 20 h to enable infection of the macrophages to become established. Media was then aspirated from the chamber slides and replaced with doubling dilutions of the test compound. The cells were then incubated at 32°C for 72 h. After washing with PBS, the chambers were detached, the slides allowed to dry and then fixed in methanol. Each slide was then stained with Giemsa and examined microscopically. A total of 250 cells were counted to determine the number of infected and uninfected cells.

The infection index was calculated as the average number of parasites/cell multiplied by the percentage of infected cells. A 50% inhibition of intracellular *Leishmania donovani* amastigote growth was achieved with 0.30 - 0.71 µg/ml of the amphotericin B - PMAA-Na preparation (Figure 21) compared to 0.54 µg/ml of clinical grade amphotericin B (Figure 22a) or 1.96 µg/ml of Ambisome (Figure 22b). A 90% inhibition of intracellular *Leishmania donovani* amastigote growth was achieved using 2.18 - 3.18 µg/ml of the amphotericin B - PMAA-Na preparation compared to 2.31 µg/ml of clinical grade amphotericin B or >8 µg/ml of Ambisome.

### L) The interferon-y releasing, Th1 adjuvant activity of the amphotericin B - PMAA-Na preparation:

### Example L1:

Human peritoneal cells that included macrophages and dendritic cells were cultured in macrophage growth medium with each compound. All reagents and compounds contained <0.06 endotoxin unit/ml. The culture supernatants were harvested 24 h later. Interferon-γ was measured by EIA. The release of the Th1 promoting cytokine, interferon-γ, from peritoneal macrophages was significantly higher in the presence of the amphotericin B - PMAA-Na preparation (100 µg/ml) compared to cells only control, clinical grade amphotericin B, commercial PMAA-Na, or the PMAA-Na as shown in Figure 23. The level of interferon-y release with the endotoxin free amphotericin B - PMAA-Na preparation would be sufficient to promote a pharmacological Th1 response in man but would not be high enough to cause significant adverse side-effects in man.

### Example L2:

The purpose of the experiments described in this section was to establish whether a preparation of an antigen and PMAA-Na could also elicit a Th1 helper response.

The tuberculin-PMAA-Na preparation was made as follows. The homo-polymer, PMAA-Na (30 mg), prepared as described in **Examples A1 to A4** was added to tuberculin purified protein derivative BP (10 ml, 100,000 units/ml, Evans Vaccines) and the resulting solution was stirred for 1.5 h at room temperature. The solution was then dialysed against 1 litre of water using Visking dialysis membrane (MWCO 7000, Medicell International) over 24 h, changing the water six times. The dialysed solution was filtered through a 0.2 µm filter and freeze-dried to afford an off-white solid product (20 mg). FT-IR (ATR) 1648 cm⁻¹, 1545 cm⁻¹, 1450 cm⁻¹, 1398 cm⁻¹, 1259 cm⁻¹. PBMCs were then isolated from whole blood using Ficoll-Paque and resuspended in RPMI 1640 supplemented with 10% human serum, 200 µg/ml penicillin and 200 IU/ml streptomycin. All reagents and compounds contained <0.06 endotoxin unit/ml as determined using the *Limulus* amebocyte lysate assay (Pyrotell, Associates of Cape Cod, US). The cells (10⁵ cells/well) and each compound were mixed in duplicate and incubated at 37°C / 5% CO₂ for 4 days. [³H]-Thymidine (specific activity 20-30 Ci/mmol; Amersham Biosciences, UK) was added at 1 µCi/well for a further 18 hours. The cells were then harvested and proliferation determined using a liquid scintillation counter. The results were expressed as the mean counts/minute (cpm) ± sem. Figure 24a shows that there was significantly increased proliferation of T lymphocytes when they were cultured with 1 µg/ml of the tuberculin-PMAA-Na, preparation. There was even more proliferation of T lymphocytes when they were cultured with 10 µg/ml of tuberculin-PMAA-Na preparation (P = 0.001 ).

Figure 24b shows the results for donor B. There was significantly more T lymphocyte proliferation with 10 µg/ml of the tuberculin-PMAA-Na preparation than with 10 µg/ml of tuberculin antigen (P = 0.002). This shows that the tuberculin-PMAA-Na preparation causes significantly more T lymphocyte proliferation than the tuberculin antigen on its own.

### Example L3: ELISpot assay for Interferon-γ secreting cells:

Human PBMCs were isolated and adjusted to 2 x 10⁵ cells/well in RPMI 1640 supplemented with 10% human serum, 200 µg/ml penicillin and 200 IU/ml streptomycin. All reagents and the compounds contained <0.06 endotoxin unit/ml. The PBMCs and the compounds were mixed in the wells of an ELISpot PVDF-backed microplate coated with the human interferon-γ monoclonal antibody (R&D Systems, UK). Unstimulated cells were used as the negative control and recombinant human interferon-γ was used in the positive control. The plate was incubated for 24 hours at 37°C / 5% CO₂. The manufacturer's instructions were then followed to develop the microplate and the number of positive spots for interferon-γ counted. Each spot represented a single interferon-γ secreting cell. Figure 25 shows that the tuberculin PPD - PMAA-Na preparation was significantly more effective than tuberculin antigen alone in stimulating interferon-γ release from primary human T lymphocytes. This increase in interferon-y secretion from T lymphocytes was seen with 50 µg/ml of the tuberculin PPD - PMAA-Na preparation and with 100 µg/ml of the tuberculin PPD - PMAA-Na preparation.

### M) Determination of the anti-leishmania activity of the amphotericin B - PMAA-Na preparation against Leishmania donovani in a mouse model of visceral leishmaniasis:

### Example M1:

*Leishmania donovani* strain (MHOM/ET/67/L82) amastigotes were collected from the spleens of heavily infected donor Syrian Hamsters-*Mesocritefus auratus.* An inoculum containing 7.5 -10 x 10⁷ amastigotes/mL was prepared. Female BALB/c mice (20 g) that were specified pathogen free were infected intravenously with 200 µL (equivalent to 1.5 - 2 x 10⁷ amastigotes) on day 0. The end point of infection was evaluated by microscopic reading on day 14 to check for the level of infection in one mouse, and on day 21 post-infection of Giemsa stained liver impressions to determine the total parasite burden.

The following compounds were administered intravenously according to the following regimens:-
a) untreated control after infection.
b) 0.5 mg/kg of blank liposome on days 14, 16 and 18 after infection.
c) 8 mg/kg of PMAA-Na on days 14, 16 and 18 after infection.
d) 1 mg/kg of clinical grade amphotericin B on days 14, 16 and 18 after infection.
e) 0.5 mg/kg of AmBisome on days 14, 16 and 18 after infection.
f) 0.5 mg/kg of amphotericin B - PMAA-Na preparation on days 14, 16 & 18 after infection.
g) 1 mg/kg of amphotericin B - PMAA-Na preparation on days 14, 16 and 18 after infection.
h) 2 mg/kg of amphotericin B - PMAA-Na preparation on days 14, 16 and 18 after infection.

There were 5 animals in each group. The carrier solution for the intravenous - injections of PMAA-Na and for amphotericin B - PMAA-Na preparation was 5% dextrose. All drug preparations were filtered using a 0.2 µL syringe filter. The intravenous volume of injection was 200 µL per injection.

Groups of mice were weighed before and after treatment as a measure of toxicity and the percentage weight change noted. On day 21 after infection (which was 3 days after the completion of treatment), the parasite burden was determined microscopically from methanol fixed liver impression slides stained with 10% Giemsa. The number of amastigotes/500 liver cells were counted microscopically and the results expressed as a percentage of the untreated control.

Figure 26 shows that the blank liposomes and the PMAA-Na had no anti-leishmanial activity. There was significant killing activity (P<0.0001) by clinical grade amphotericin B, Ambisome, amphotericin B - PMAA-Na preparation at 1 mg/kg, and amphotericin B - PMAA-Na preparation at 2 mg/kg of *Leishmania donovani* amastigotes compared to the controls. The anti-leishmanial activities of the clinical grade amphotericin B, Ambisome, and the amphotericin B - PMAA-Na (2 mg/kg) were not significantly different from each other (P<0.05). These results therefore show that the amphotericin B - PMAA-Na preparation was as effective as Ambisome in this animal model of visceral leishmaniasis.

### Example N: PMAA-Na Synthesis

### Generation of PMAA-Afa constructs of varying molecular weight -

During the synthesis of the PMAA-NA precursor, PMOSu, the polymerisation conditions were altered as described in Example A to create several polymers with molecular weights that ranged from 19 kD to 37 kD. The molecular weight and the polydispersity index were established using gel permeation chromatography (GPC).

The toxicity of PMAA-Na constructs of different molecular weights for primary human cells (red blood cell lysis and peripheral blood mononuclear cells (PBMCs)) was established using an MTT assay. The PMAA-Na constructs did not cause red blood cell lysis at a concentration of 2 mg/ml after a 1 h, 5 h and a 24 h incubation, see Figure 27. In addition, they were not toxic to peripheral blood mononuclear cells at 2 mg/ml after incubation for 1 day and for 2 days, see Figure 28. These results were not affected by the molecular weight of the PMAA-Na constructs made.

### Example O: Stability of amphotericin B - PMAA-Na complex during storage:

Lyophilised amphotericin B - PMAA-Na was stored at 4°C under argon for 4 months. It was also solubilised in 5% dextrose at a concentration of 1 mg/ml and stored at 4°C for 7 months under argon. At the end of this time, the stability of the amphotericin B - PMAA-Na complex was determined by incubating it with human red blood cells. Previous experiments have shown that the amphotericin B - PMAA-Na complex is much less toxic than amphotericin B. This assay was therefore repeated after storage of the complex for several months.

The experiment was carried out as previously described. Freshly prepared, clinical grade amphotericin B was used as a reference for the comparison. Incubations were undertaken for 1 h and 6 h. Figure 29 shows that the amphotericin B - PMAA-Na complex was not toxic to red blood cells after storage. The complex was therefore stable when it was stored as a lyophilised powder for 4 months, and also when it was stored in 5% dextrose at 4°C for 7 months.

The anti-leishmanial activity of the complex after storage was also determined as described in Examples J1 and K1 above. Figure 30 shows that the anti-leishmanial activity of amphotericin B - PMAA-Na against the free *Leishmania* promastigotes and against the intracellular amastigotes. The activity of amphotericin B - PMAA-Na was not affected by storage as a powder for 4 months at 4°C. The activity of amphotericin B - PMAA-Na was not affected by storage for 7 months at 4°C in 5% dextrose.

### EXAMPLE P: Cryptococcus neoformans

*Cryptococcus neoformans* mucoidal strains var *neoformans* 3003 and var *gattii* 3216 from the National Collection of Pathogenic Fungi were tested. A non-mucoidal clinical isolate of var *neoformans* and a non-mucoidal clinical isolate of var *gattii* were also tested. The organisms were maintained on Sabouraud dextrose agar plates supplemented with chloramphenicol (Oxoid, UK) at 32°C in a humidified chamber with 5% CO₂.

Strains were subcultured on Sabouraud dextrose agar for 48 h before being suspended in sterile water and their concentration adjusted to 4 x10⁴ colony forming units (CFU) per ml. Doubling dilutions of the sample were made at twice the desired final concentration in x2 yeast nitrogen base (YNB, Anachem). Equal volumes of sample and yeast suspension were then added to a 96 well flat bottom plate and incubated at 32°C. After 3 days incubation, the plate was shaken thoroughly to disperse the yeast evenly across wells and turbidity measured using a spectrophotometer (490 nm). 100% viability was defined using the optical density of the untreated yeast in control wells.

For the infection of primary human macrophages, *Cryptococcus* strains were subcultured in YNB supplemented with 10% human serum for 48 h in the presence of 5% CO₂ to allow them to form a capsule. Peritoneal human macrophages or monocyte derived macrophages (~750,000) were plated onto chamber slides (Lab-Tek, USA) and allowed to adhere for 24 h. Yeast cells were spun down, washed, and resuspended in RPMI supplemented with 10% human serum and 2% glucose; the latter facilitates the growth of the yeast. This suspension was added at 15 times the concentration of the macrophages and the infection allowed to proceed for -21 h at 37°C in a humidified chamber with 5% CO₂. Media was then aspirated and the macrophages washed with PBS. Doubling dilutions of the test sample were then added in RPMI supplemented with 10% human serum, and incubated at 37°C in a humidified chamber with 5% CO₂ for 3 days. Media was aspirated and the macrophages washed extensively with PBS to remove any extracellular cryptococci. Chambers were removed and the slides allowed to dry before fixing with methanol and heat, and then Gram stained. The number of infected and uninfected macrophages, and the number of gram positive yeast CFU were counted. The results were expressed as the infection index, which was calculated as the average number of CFU/percentage of infected cells.

Figure 31 (i) and (ii) show the results for *Cryptococcus neoformans* var *neoformans,* and Figure 31 (iii) and (iv) show the results for *Cryptococcus neoformans* var *gatti.* The LD₅₀ for amphotericin B (0.9 -1.4 µg/ml) and for amphotericin B - PMAA-Na (1.6 - 2.7 µg/ml) are similar. Figure 32 (i) and (ii) show the results for *Cryptococcus neoformans* var *neoformans,* and Figure 32 (iii) and (iv) show the results for *Cryptococcus neoformans* var *gatti* when these organisms have infected human monocyte derived macrophages. The LD₅₀ for amphotericin B and for amphotericin B - PMAA-Na were similar in all of the 8 experiments performed. In the case of *Cryptococcus neoformans* var *neoformans,* the LD₅₀ for amphotericin B was 0.9-1.4 µg/ml and that for amphotericin B - PMAA-Na was similar at 1.6 - 2.7 µg/ml. For *Cryptococcus neoformans* var *gatti* the LD₅₀ for amphotericin B was 0.06 -1.0 µg/ml and that for amphotericin B-PMAA-Na was similar at 0.1 - 0.5 µg/ml. Therefore, the complex is as active against cryptococci as amphotericin B alone.

### EXAMPLE Q: Candida sp.

*Candida albicans* (ATCC 90028) and *Candida glabrata* (ATCC 90030) were used. In each case the strain was maintained on Sabouraud dextrose agar plates supplemented with chloramphenicol (Oxoid, UK) at 32°C in a humidified chamber with 5% CO₂. It was subcultured on Sabouraud dextrose agar for 48 h before its concentration was adjusted to 2x10⁵ CFU/ml. Doubling dilutions of the sample were made at twice the desired final concentration in x2 yeast nitrogen base (YNB, Anachem). Equal volumes of sample and yeast suspension were added to a 96 well flat bottom plate and incubated at 32°C. After 1 day, the plate was shaken thoroughly to disperse the yeast growth evenly across the wells. Turbidity was measured using a spectrophotometer (490 nm). 100% viability was established using the optical density of the untreated yeast in control wells.

Figure 33 (i) shows the results for *Candida albicans* and Figure 33 (ii) shows the results for *Candida glabrata.* The LD₅₀ for amphotericin B (0.9 -1.8 µg/ml) and for amphotericin B - PMAA-Na (1.6 - 2.3 µg/ml) are similar. Therefore, the complex is as active against Candida sp. as amphotericin B alone.

## Claims

1. A complex that comprises a polymer that includes units derived from an acrylic acid or a salt thereof, said polymer having a polydispersity of 1.7 or less; a molecular weight of 100,000 or less; and which includes units of the general formula (I): 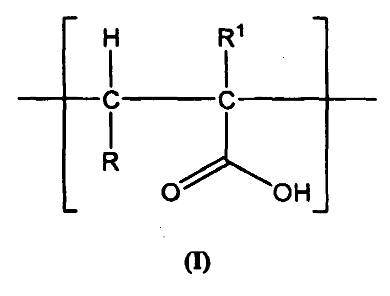 in which R is hydrogen, methyl or carboxylic acid and R¹ is hydrogen or C₁₋₆alkyl; or a salt thereof; and
(i) a substance that has pharmacological activity against a pathogenic organism, or
(ii) a substance that has pharmacological activity against a cancer, or
(iii) one or more agents selected from antigens and immunogens.

2. A complex as claimed in claim 1, wherein the pathogenic organism is predominantly but not exclusively an intracellular organism.

3. A complex as claimed in claim 2, wherein the pathogenic organism is an intracellular organism that exists and/or persists in cells of macrophage origin and/or in other antigen presenting cells.

4. A complex as claimed in claim 1, wherein the pathogenic organism is selected from the following organisms :
a) Organisms that cause superficial mycoses; tinea; thrush; Malassezia infections; Otomycosis; and Keratomycosis.
b) Candida species that cause invasive and chronic fungal infections; Aspergillus species; Cryptococcus neoformans; Mucormycosis; Fusarium species; Trichosporon species; Blastomycosis; Sporothrix species; Sporotrichum species; Histoplasmosis; African histoplasmosis; Blastomycosis; Coccidioidomycosis; Paracoccidioidomycosis; and Infections caused by Penicillium marneffei.
c) Organisms that cause mycobacterial diseases.
d) Members of the schistosoma family that cause Schistosomiasis.
e) Organisms that cause typhoid and paratyphoid fevers.
f) Organisms that cause toxoplasmosis.
g) Organisms that cause Human African Trypanosomiasis.
h) Organisms that cause American Trypanosomiasis.
i) Organisms that cause malaria.
j) Organisms that cause HIV and HTLV infections.
k) Organisms that cause Pneumocystis carinii infections.

5. A complex as claimed in claim 1, wherein the pathogenic organism causes leishmaniasis.

6. A complex as claimed in any one of claims 1 to claim 5, wherein the pharmacologically active substance is amphotericin B.

7. A complex as claimed in claim 1, wherein an antigen or immunogen is derived directly or indirectly from an organism that causes tuberculosis, tetanus, anthrax, cholera, diptheria, measles, mumps, rubella, Hepatitis A, Hepatitis B, influenza, herpes zoster, poliomyelitis, rabies, smallpox, yellow fever, varicella, herpes zoster, herpes simplex, influenza or leishmaniasis.

8. A complex as claimed in claim 1, wherein an antigen or immunogen is derived directly or indirectly from an organism as defined in any one of claims 2 to 5.

9. A complex as claimed in any one of the preceding claims, wherein the polymer has a molecular weight of 4,000 or more.

10. A complex as claimed in any one of the preceding claims, wherein the polymer is a poly(methacrylic acid) or a salt thereof.

11. A pharmaceutical preparation which comprises a complex as claimed in any one of the preceding claims in admixture or conjunction with a pharmaceutically suitable carrier.

12. A pharmaceutical preparation as claimed in claim 11, which comprises a delivery system adjuvant.

13. A complex as claimed in any one of claims 1 to 10 or a pharmaceutical preparation as claimed in either claim 11 or claim 12, for use as a medicament.

14. A narrow molecular weight distribution polymer that includes units derived from an acrylic acid or a salt thereof, said polymer having a polydispersity of 1.7 or less; a molecular weight of 100,000 or less; and which includes units of the general formula (I): 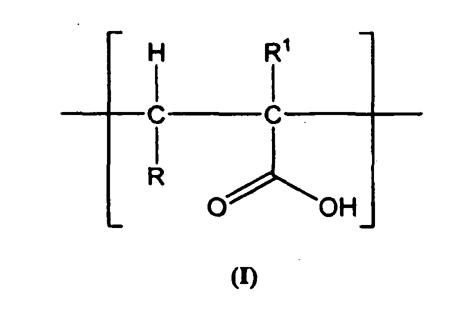 in which R is hydrogen or methyl and R¹ is, independently of R, hydrogen or methyl; or a salt thereof; for use with (i) a substance that has pharmacological activity against a pathogenic organism, for the treatment of an infection by the pathogenic organism and/or for inducing an immune response to the pathogenic organism; (ii) a substance that has pharmacological activity against a cancer for the treatment of the cancer; or (iii) an antigen or immunogen, for inducing an immune response to the antigen or immunogen.

15. A complex, preparation or polymer as claimed in either claim 13 or claim 14, for use in treatment of an infection by a pathogenic organism and/or for inducing an immune response to the pathogenic organism, which complex comprises a narrow molecular weight distribution polymer that includes units derived from an acrylic acid or a salt thereof, said polymer having a polydispersity of 1.7 or less; a molecular weight of 100,000 or less; and which includes units of the general formula (1): 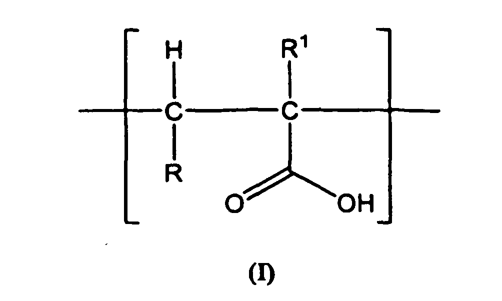 in which R is hydrogen or methyl and R¹ is, independently of R, hydrogen or methyl; or a salt thereof; and a substance that has pharmacological activity against the pathogenic organism.

16. A complex, preparation or polymer as claimed in either claim 13 or claim 14, for treatment of leishmaniasis and/or for inducing an immune response to an organism that causes leishmaniasis in any of its clinical forms.

17. A complex, preparation or polymer as claimed in either claim 13 or claim 14, for use in treatment of a cancer or for inducing an immune response to an antigen or immunogen.

18. A narrow molecular weight distribution polymer that includes units derived from an acrylic acid or a salt thereof, said polymer having a polydispersity of 1.7 or less; a molecular weight of 100,000 or less; and which includes units of the general formula (I): 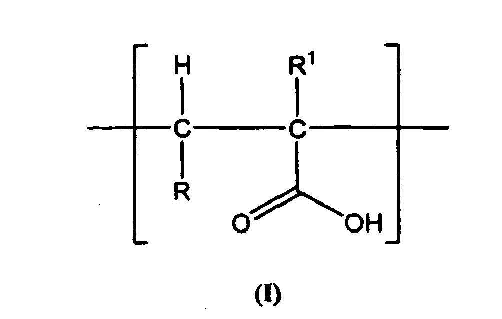 in which R is hydrogen or methyl and R¹ is, independently of R, hydrogen or methyl; or a salt thereof; for use as an immune potentiating adjuvant in the manufacture of a vaccine.

## Patentansprüche

1. Komplex, umfassend ein Polymer, das Einheiten enthält, die von einer Acrylsäure oder einem Salz derselben stammen, wobei das Polymer eine Polydispersität von 1,7 oder weniger und ein Molekulargewicht von 100.000 oder weniger aufweist, und wobei das Polymer Einheiten der allgemeinen Formel (I) enthält: 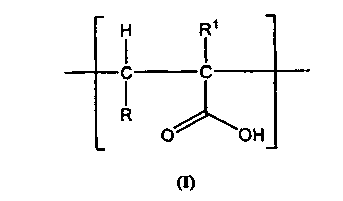 in welcher der Rest R Wasserstoff, eine Methyl- oder Carbonsäure-Gruppe darstellt, und der Rest R¹ Wasserstoff oder eine C₁₋₆-Alkyl-Gruppe darstellt; oder ein Salz davon; und
(i) eine Substanz, die eine pharmakologische Aktivität gegen einen pathogenen Organismus aufweist, oder
(ii) eine Substanz, die eine pharmakologische Aktivität gegen eine Krebserkrankung aufweist, oder
(iii) ein oder mehrere Mittel, die aus Antigenen und Immunogenen ausgewählt werden.

2. Komplex nach Anspruch 1, wobei der pathogene Organismus vorwiegend, jedoch nicht ausschließlich ein intrazellulärer Organismus ist.

3. Komplex nach Anspruch 2, wobei der pathogene Organismus ein intrazellulärer Organismus ist, welcher in Zellen, die von einem Makrophagen stammen, und/oder in anderen antigen-präsentierenden Zellen existiert und/oder persistiert.

4. Komplex nach Anspruch 1, wobei der pathogene Organismus aus den folgenden Organismen ausgewählt wird:
(a) Organismen, die Mykosen an Oberflächen verursachen; Tinea; Soor; Malassezia-Infektionen; Otomykose und Keratomykose;
(b) Candida-Spezies, die invasive und chronische Pilzinfektionen verursachen; Aspergillus-Spezies; Cryptococcus neoformans; Mukormykose; Fusarium-Spezies; Trichosporon-Spezies; Blastomykose; Sporothrix-Spezies; Sporotrichum-Spezies; Histoplasmose; Afrikanische Histoplasmose; Blastomykose; Kokzidioidomykose; Parakokzidioidomykose; und Infektionen, die durch Penicillium marneffei verursacht werden;
(c) Organismen, die mykobakterielle Krankheiten verursachen;
(d) Mitglieder der Schistosoma-Familie, die Schistosomiasis verursachen;
(e) Organismen, die typhoides und paratyphoides Fieber verursachen;
(f) Organismen, die Toxoplasmose verursachen;
(g) Organismen, die humane afrikanische Trypanosomiasis verursachen;
(h) Organismen, die amerikanische Trypanosomiasis verursachen;
(i) Organismen, die Malaria verursachen;
(j) Organismen, die HIV- und HTLV-Infektionen verursachen;
(k) Organismen, die Pneumocystis carinii-Infektionen verursachen.

5. Komplex nach Anspruch 1, wobei der pathogene Organismus Leishmaniose verursacht.

6. Komplex nach einem der Ansprüche 1 bis 5, wobei die pharmakologisch wirksame Substanz Amphotericin B darstellt.

7. Komplex nach Anspruch 1, wobei ein Antigen oder Immunogen unmittelbar oder mittelbar aus einem Organismus stammt, der Tuberkulose, Tetanus, Anthrax, Cholera, Diphtherie, Masern, Mumps, Röteln, Hepatitis A, Hepatitis B, Influenza, Herpes zoster, Poliomyelitis, Tollwut, Pocken, Gelbfieber, Windpocken, Herpes simplex oder Leishmaniose verursacht.

8. Komplex nach Anspruch 1, wobei ein Antigen oder Immunogen unmittelbar oder mittelbar aus einem Organismus stammt, wie er in einem der Ansprüche 2 bis 5 definiert wurde.

9. Komplex nach einem der vorstehenden Ansprüche, wobei das Polymer ein Molekulargewicht von 4.000 oder mehr aufweist.

10. Komplex nach einem der vorstehenden Ansprüche, wobei das Polymer eine Poly(methacrylsäure) oder ein Salz derselben darstellt.

11. Pharmazeutische Zubereitung, welche einen Komplex umfasst, wie er in einem der vorstehenden Ansprüche definiert wurde, in Mischung oder Verbindung mit einem pharmazeutisch geeigneten Träger.

12. Pharmazeutische Zubereitung nach Anspruch 11, welche einen Transportsystem-Hilfsstoff umfasst.

13. Komplex nach einem der Ansprüche 1 bis 10 oder pharmazeutische Zubereitung nach Anspruch 11 oder Anspruch 12 für die Verwendung als ein Medikament.

14. Polymer mit enger Molekulargewichts-Verteilung, das Einheiten enthält, die von einer Acrylsäure oder einem Salz derselben stammen, wobei das Polymer eine Polydispersität von 1,7 oder weniger und ein Molekulargewicht von 100.000 oder weniger aufweist, und wobei das Polymer Einheiten der allgemeinen Formel (I) enthält: 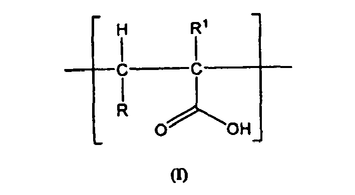 in welcher der Rest R Wasserstoff oder eine Methylgruppe darstellt, und der Rest R¹ unabhängig vom Rest R Wasserstoff oder eine Methylgruppe darstellt, oder ein Salz davon; für die Verwendung
(i) mit einer Substanz, die eine pharmakologische Wirkung gegenüber einem pathogenen Organismus aufweist, für die Behandlung einer Infektion durch den pathogenen Organismus und/oder für die Auslösung einer Immunantwort gegenüber dem pathogenen Organismus;
(ii) mit einer Substanz, die eine pharmakologische Wirkung gegenüber einer Krebserkrankung aufweist, für die Behandlung der Krebserkrankung; oder
(iii) mit einem Antigen oder Immunogen für die Auslösung einer Immunantwort gegenüber dem Antigen oder Immunogen.

15. Komplex, Zubereitung oder Polymer nach Anspruch 13 oder Anspruch 14 für die Verwendung zur Behandlung einer Infektion durch einen pathogenen Organismus und/oder für die Auslösung einer Immunantwort gegenüber dem pathogenen Organismus, welcher Komplex ein Polymer mit einer engen Molekulargewichts-Verteilung umfasst, das Einheiten enthält, die von einer Acrylsäure oder einem Salz derselben stammen, wobei das Polymer eine Polydispersität von 1,7 oder weniger und ein Molekulargewicht von 100.000 oder weniger aufweist, und wobei das Polymer Einheiten der allgemeinen Formel (I) enthält: 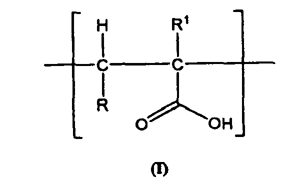 in welcher der Rest R Wasserstoff oder eine Methylgruppe darstellt und der Rest R¹ unabhängig vom Rest R Wasserstoff oder eine Methylgruppe darstellt; oder ein Salz davon; und eine Substanz, die eine pharmakologische Aktivität gegenüber dem pathogenen Organismus aufweist.

16. Komplex, Zubereitung oder Polymer nach Anspruch 13 oder Anspruch 14 zur Behandlung der Leishmaniose und/oder für die Auslösung einer Immunantwort gegenüber einem Organismus, der Leishmaniose in einer beliebigen klinischen Form verursacht.

17. Komplex, Zubereitung oder Polymer nach Anspruch 13 oder Anspruch 14 für die Verwendung zur Behandlung einer Krebserkrankung oder für die Auslösung einer Immunantwort gegenüber einem Antigen oder Immunogen.

18. Polymer mit einer engen Molekulargewichts-Verteilung, das Einheiten enthält, die von einer Acrylsäure oder einem Salz derselben stammen, wobei das Polymer eine Polydispersität von 1,7 oder weniger und ein Molekulargewicht von 100.000 oder weniger aufweist; und wobei das Polymer Einheiten der folgenden allgemeinen Formel (I) enthält: 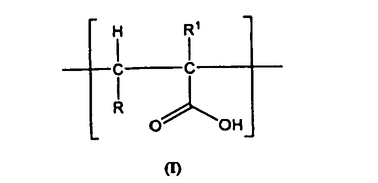 in welcher der Rest R Wasserstoff oder eine Methylgruppe darstellt und der Rest R¹ unabhängig vom Rest R Wasserstoff oder eine Methylgruppe darstellt; oder ein Salz davon; für die Verwendung als ein immun-potenzierender Hilfsstoff bei der Herstellung eines Impfstoffs.

## Revendications

1. Complexe comprenant un polymère qui comprend des motifs dérivés d'un acide acrylique ou d'un sel de celui-ci, ledit polymère ayant une polydispersité de 1,7 ou moins ; un poids moléculaire de 100000 ou moins ; et qui comprend des motifs de la formule générale (I) : 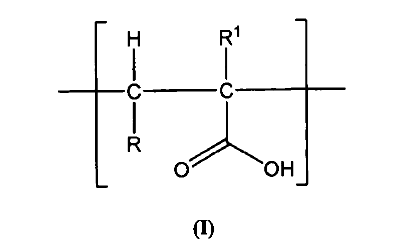 où R est un atome d'hydrogène, un groupe méthyle ou un acide carboxylique et R¹ est un atome d'hydrogène ou un groupe alkyle en C₁-C₆ ou un sel de celui-ci ; et
(i) une substance qui possède une activité pharmacologique vis-à-vis d'un organisme pathogène, ou
(ii) une substance qui possède une activité pharmacologique vis-à-vis d'un cancer, ou
(ii) un ou plusieurs agents choisis parmi les antigènes et les immunogènes.

2. Complexe selon la revendication 1, dans lequel l'organisme pathogène est principalement mais pas exclusivement un organisme intracellulaire.

3. Complexe selon la revendication 2, dans lequel l'organisme pathogène est un organisme intracellulaire qui existe et/ou persiste dans les cellules d'origine macrophage et/ou dans d'autres cellules présentant l'antigène.

4. Complexe selon la revendication 1, dans lequel l'organisme pathogène est choisi parmi les organismes suivants:
a) les organismes qui provoquent des mycoses superficielles ; la teigne ; le muguet ; les infections à Malassezia ; l' otomycose ; et la kératomycose ;
b) les espèces de candida qui provoquent des infections fongiques invasives et chroniques ; les espèces d'Aspergillus Cryptococcus neoformans ; la mucormycose ; les espèces de Fusarium ; les espèces de Trichosporon ; la blastomycose ; les espèces de Sporothrix; les espèces de Sporotrichum ; l'histoplasmose ; l'histoplasmose africaine; la blastomycose; la coccidioïdomycose ; la paracoccidioïdomycose ; et les infections provoquées par Penicillium marneffei ;
c) les organismes provoquant des maladies myco-bactériennes ;
d) les membres de la famille des schistosomes provoquant la schistosomiase ;
e) les organismes provoquant les fièvres typhoïde et paratyphoïde ;
f) les organismes provoquant la toxoplasmose ;
g) les organismes provoquant la trypanosomiase africaine humaine ;
h) les organismes provoquant la trypanosomiase américaine ;
i) les organismes provoquant la malaria;
j) les organismes provoquant les infections par le VIH et le HTLV.
k) les organismes provoquant les infections à Pneumocystis carinii.

5. Complexe selon la revendication 1, dans lequel l'organisme pathogène provoque la leishmaniose.

6. Complexe selon l'une quelconque des revendications 1 à 5, dans lequel la substance pharmacologiquement active est l'amphotéricine B.

7. Complexe selon la revendication 1, dans lequel un antigène ou un immunogène est directement ou indirectement dérivé d'un organisme provoquant la tuberculose, le tétanos, le charbon, le choléra, la diphtérie, la rougeole, les oreillons, la rubéole, l'hépatite A, l'hépatite B, la grippe, le zona, la poliomyélite, la rage, la variole, la fièvre jaune, la varicelle, le zona, l'herpès, la grippe ou la leishmaniose.

8. Complexe selon la revendication 1, dans lequel un antigène ou un immunogène est directement ou indirectement dérivé d'un organisme défini selon l'une quelconque des revendications 2 à 5.

9. Complexe selon l'une quelconque des revendications précédentes, dans lequel le polymère a un poids moléculaire de 4000 ou plus.

10. Complexe selon l'une quelconque des revendications précédentes, dans lequel le polymère est un poly (acide méthacrylique) ou un sel de celui-ci.

11. Préparation pharmaceutique qui comprend un complexe selon l'une quelconque des revendications précédentes en mélange ou conjointement avec un support pharmaceutiquement acceptable.

12. Préparation pharmaceutique selon la revendication 11, qui comprend un adjuvant du mode d'administration.

13. Complexe selon l'une quelconque des revendications 1 à 10 ou préparation pharmaceutique selon la revendication 11 ou 12, pour une utilisation en tant que médicament.

14. Polymère de distribution de poids moléculaire étroite qui comprend des motifs dérivés d'un acide acrylique ou d'un sel de celui-ci, ledit polymère ayant une polydispersité de 1,7 ou moins ; un poids moléculaire de 100000 ou moins ; et qui comprend des motifs de la formule générale (I) : 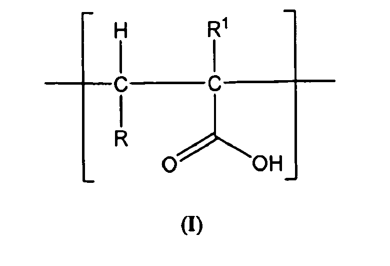 où R est un atome d'hydrogène ou un groupe méthyle et R¹ est, indépendamment de R, un atome d'hydrogène ou un groupe méthyle ; ou un sel de celui-ci ; pour une utilisation avec (i) une substance qui possède une activité pharmacologique vis-à-vis d'un organisme pathogène, pour le traitement d'une infection par l'organisme pathogène et/ou pour l'induction d'une réponse immune vis-à-vis de l'organisme pathogène ; (ii) une substance qui possède une activité pharmacologique vis-à-vis d'un cancer, pour le traitement du cancer ; ou (iii) un antigène ou un immunogène, pour induire une réponse immune vis-à-vis de l'antigène ou de l'immunogène.

15. Complexe, préparation ou polymère selon la revendication 13 ou 14, pour une utilisation dans le traitement d'une infection par un organisme pathogène et/ou pour l'induction d'une réponse immune vis-à-vis de l'organisme pathogène, lequel complexe comprend un polymère ayant une distribution de poids moléculaire étroite qui comprend des motifs dérivés d'un acide acrylique ou d'un sel de celui-ci, ledit polymère ayant une polydispersité de 1,7 ou moins ; un poids moléculaire de 100000 ou moins ; et qui comprend des motifs de la formule générale (I) : 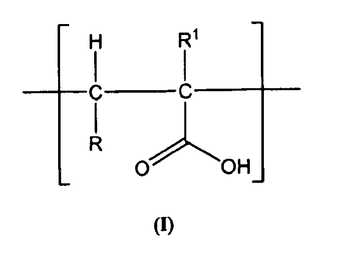 où R est un atome d'hydrogène ou un groupe méthyle et R¹ est, indépendamment de R, un atome d'hydrogène ou un groupe méthyle ; ou un sel de celui-ci ; et une substance qui possède une activité pharmacologique vis-à-vis de l'organisme pathogène.

16. Complexe, préparation ou polymère selon la revendication 13 ou 14, pour le traitement de la leishmaniose et/ou pour l'induction d'une réponse immune vis-à-vis d'un organisme provoquant la leishmaniose sous n'importe quelle forme clinique.

17. Complexe, préparation ou polymère selon la revendication 13 ou 14, pour une utilisation dans le traitement d'un cancer ou pour l'induction d'une réponse immune vis-à-vis d'un antigène ou d'un immunogène.

18. Polymère de distribution de poids moléculaire étroite qui comprend des motifs dérivés d'un acide acrylique ou d'un sel de celui-ci, ledit polymère ayant une polydispersité de 1,7 ou moins ; un poids moléculaire de 100000 ou moins ; et qui comprend des motifs de la formule générale (1) : 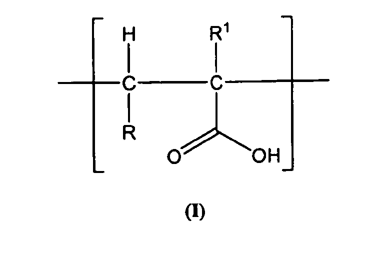 où R est un atome d'hydrogène ou un groupe méthyle et R¹ est, indépendamment de R, un atome d'hydrogène ou un groupe méthyle ; ou un sel de celui-ci ; pour une utilisation en tant qu'adjuvant de potentialisation immunitaire dans la fabrication d'un vaccin.
